(19) Europäisches Patentamt
European Patent Office
Office européen des brevets

(11) EP 3 274 447 B1

(12) **EUROPEAN PATENT SPECIFICATION**

(45) Date of publication and mention of the grant of the patent:
**16.10.2019 Bulletin 2019/42**

(21) Application number: **16721253.9**

(22) Date of filing: **28.03.2016**

(51) Int Cl.:
***C12N 7/00*** *(2006.01)*

(86) International application number:
**PCT/IN2016/000074**

(87) International publication number:
**WO 2016/157208 (06.10.2016 Gazette 2016/40)**

(54) **RECOMBINANT MUMPS VIRUS JERYL LYNN 2 BASED VACCINE**

AUF REKOMBINANTEM MUMPS-VIRUS JERYL LYNN 2 BASIERTER IMPFSTOFF

VACCIN BASÉ SUR LE VIRUS RECOMBINANT DES OREILLONS JERYL LYNN 2

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **27.03.2015 IN 1055MU2015**

(43) Date of publication of application:
**31.01.2018 Bulletin 2018/05**

(73) Proprietor: **Cadila Healthcare Limited**
**Ahmedabad, Gujarat 380015 (IN)**

(72) Inventors:
- **GLUECK, Reinhard**
**Ahmedabad**
**Gujarat 380015 (IN)**
- **GIANNINO, Viviana**
**Ahmedabad**
**Gujarat 380015 (IN)**
- **GUPTA, Gaurav**
**Ahmedabad**
**Gujarat 380015 (IN)**

(74) Representative: **Gill Jennings & Every LLP**
**The Broadgate Tower**
**20 Primrose Street**
**London EC2A 2ES (GB)**

(56) References cited:
**WO-A1-2011/012999 WO-A2-01/09309**

- **CHAMBERS P ET AL: "Molecular differences between two Jeryl Lynn mumps virus vaccine component strains, JL5 and JL2", JOURNAL OF GENERAL VIROLOGY., vol. 90, no. 12, 5 August 2009 (2009-08-05), pages 2973-2981, XP055294794, GB ISSN: 0022-1317, DOI: 10.1099/vir.0.013946-0**
- **AMEXIS G ET AL: "Sequence Diversity of Jeryl Lynn Strain of Mumps Virus: Quantitative Mutant Analysis for Vaccine Quality Control", VIROLOGY, vol. 300, no. 2, 1 September 2002 (2002-09-01), pages 171-179, XP055294796, AMSTERDAM, NL ISSN: 0042-6822, DOI: 10.1006/viro.2002.1499**
- **DATABASE UniProt [Online] 22 September 2009 (2009-09-22), "RecName: Full=Nucleocapsid {ECO:0000256|RuleBase:RU361245}; AltName: Full=Nucleocapsid protein {ECO:0000256|RuleBase:RU361245};", XP055336463, retrieved from EBI accession no. UNIPROT:C7C5R8 Database accession no. C7C5R8**
- **DATABASE UniProt [Online] 22 September 2009 (2009-09-22), "SubName: Full=P protein {ECO:0000313|EMBL:CBA10120.1};", XP55336471, retrieved from EBI accession no. UNIPROT:C7C5S1 Database accession no. C7C5S1**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 274 447 B1

## Description

## FIELD OF THE INVENTION

The present invention provides a novel vaccine against mumps composed of highly immunogenic rMuV$^{JL2}$ (recombinant mumps virus Jeryl Lynn2 based). The invention accordingly provides an isolated genome comprising the mumps virus of the invention, along with vectors, host cells and compositions comprising the virus. The vaccine according to the present invention is safe, cost effective, highly efficacious and stable and consistent in terms of productivity.

## BACKGROUND OF THE INVENTION

Mumps is an acute viral illness characterized by fever and swelling of the parotid gland(s) that affects typically young children and may lead to complications such as orchitis, oophoritis, pancreatitis, and meningo-encephalitis. However, approximately half of infected individuals develop classical disease. Mumps virus (MuV) is a member of paramyxoviridae family, subfamily Paramyxovirinae and genus Rubulavirus, which only infects humans [Hviid et al., 2008]. MuV has a single-stranded, negative sense RNA genome consisting of 15,384 nucleotides. The genome encodes two surface glycoproteins; fusion (F) and haemagglutinin-neuraminidase (HN), four core proteins; nucleoprotein (NP), phospho (P), matrix (M) and large protein (L), and the membrane associated small hydrophobic (SH) protein.

Historically, mumps was considered as a disease of childhood but over the past 2 decades, it has affected older children and adults in the countries where mumps immunization has been in routine use. Despite high vaccination coverage, re-emergence of mumps has been reported in a number of countries including the USA, UK, Canada, the Republic of Moldova and The Netherlands [Bernard et al., 2008; Brockhoff et al., 2010; CDC, 2010; Whelan et al., 2010; Yung et al., 2010]. In India, scanty data are available regarding the epidemiology of mumps, its incidence and antibody prevalence. A few mumps outbreaks have been reported from the States of Kerala and Maharashtra [Geeta and Kumar, 2004; John, 2004; Ghatage and Kakade, 2007]. Mumps vaccine is given to children together with measles and rubella vaccines at or after 12 months of age. Two strains of live attenuated mumps virus have been used in vaccines in the U.K., namely the Urabe strain, derived from a wild-type Japanese isolate by passage in egg amnion and the Jeryl Lynn strain, derived by tissue culture passage of wild-type American isolate. Vaccines other than those based on live attenuated virus strains are not available for mumps.

The Jeryl Lynn (JL) mumps virus (MuV) vaccine contains two different component strains, MuVJL5 and MuVJL2 that differ considerably in their nucleotide sequences (Amexis et al., 2002).

The MuVJL vaccine has been reported to be derived from a single clinical isolate (Afzal et al., 1993), which was converted into a live-attenuated vaccine by passage of virus in non-human host cells. The complete nucleotide sequences of MuVJL5 and MuVJL2 have been reported (Clarke et al., 2000; Amexis et al., 2002) and show 414 nucleotide changes, resulting in 87 amino acid changes, between MuVJL5 and MuVJL2. This level of variation is at the same level as differences between genotypes of MuV. There are biological differences between MuVJL5 and MuVJL2. For example, MuVJL2 grows better in embryonated eggs than MuVJL5, (Amexis et al., 2002). Both MuVJL5 and MuVJL2 are non-neurovirulent in the rat neurovirulence test (Rubin et al., 1999, 2000, 2003).

It is known that MuV growth is strain- as well as host cell dependent [Afzal et al, 1990]. It is also reported that a strong intrinsic feature of MuV strains to interact specifically with Vero cell culture results in very different forms of cytopathic effect (CPE). During viral infection of cell, virus replicative cycle is accompanied by a number of biochemical and morphological changes within the cell which usually culminate with cell death. These morphological changes are referred to as the virus cytopathic effect (CPE). CPE may take several forms, e.g. syncytia formation, cell rounding, disorientation, swelling or shrinking, detachment from the surface, total cell lysis, etc. The form of CPE depends both on the virus and on the cells in which it is grown. [Vaccine 28 (2010) 1887-1892]

It is mentioned in Amexis et al., 2002 that the MAPREC (a molecular method to determine ratio sub strains of Jeryl Lynn in various Jeryl Lynn preparations) data confirmed the results from the cloning experiments, that by the first passage of Merck MVL on Vero cells, the percentage of JL2 decreased from 20 to 2% and none could be detected in subsequent passages, while, passaging of Jeryl Lynn strain in embryonated chicken eggs (ECE) resulted in the rapid accumulation of JL2 (over 90%) and loss of JL1, suggesting that JL2 has a strong selection advantage in ECE.

It is further concluded by the Author that the rapid disappearance of JL2 during virus passaging in Vero and CEF cells, as detected by cloning and MAPREC, suggests that JL2 replicates poorly in Vero and CEF cell cultures or may require the presence of JL1 as a helper.

From the above disclosure, it is well understood that rescue model development of JL2 strain by approaching Vero cell or CEF as a host cell is difficult for further development of vaccine. Several factors such as cytopathic effect, host cell, virus strain, combination of virus strain and host cell affect in case of development of immunogenic composition against Jeryl Lynn mumps virus.

In the present invention, Inventors have developed JL2 strain based immunogenic composition which can be further formulated along with the suitable stabilizer for vaccination purpose. Such an immunogenic composition according to present invention is having higher immunogenicity, free from extraneous proteins and other agents and finally a good candidate for the development of vaccine against mumps. In addition, such vaccine is more stable than the conventional vaccine probably due to higher genetic homogenicity of the strain.

Surprisingly, Inventors of the present invention are able to develop said stable immunogenic composition by using Vero cells as a host cell. Though, Vero cell is known as not a good candidate for JL2 strain development, surprisingly Inventors of the present invention have developed novel immunogenic composition against mumps virus using mammalian cells such as Vero cells or MRC-5 cells or HEK 293T cells.

## SUMMARY OF THE INVENTION

The present invention provides a stable Jeryl Lynn 2 (herein after as JL2) strain that can be used to produce a vaccine against mumps.

In a first aspect, the invention provides an isolated full-length genome of mumps virus attenuated JL2 strain which comprises the polynucleotide sequence having SEQ ID NO.8.

In a second aspect, the invention provides a vector comprising the polynucleotide sequence of the first aspect (full length genome of isolated JL2 strain as described).

In a third aspect, the present invention provides a host cell comprising the vector of the second aspect. Here, host cells can be suitable mammalian cell which are stable and consistent in terms of quality and productivity. Such mammalian cells can be Vero cell, MRC-5 cell, HEK 293T cell, for example.

In a fourth aspect, the present invention provides a mumps virus vaccine comprising full-length genome of mumps virus attenuated JL2 strain according the first aspect.

In a fifth aspect, the present invention provides an immunogenic composition containing full-length genome of mumps virus attenuated JL2 strain according to the first aspect.

In a sixth aspect, the present invention provides a rescue composition for recombinant mumps virus JL2 strain, the rescue composition comprising a transcription vector comprising the polynucleotide sequence according to the first aspect; an expression vector comprising isolated gene(s) encoding trans-acting protein(s) wherein trans-acting protein is selected from NP protein, P protein, L protein and combination thereof; and RNA polymerase for appropriate transcription of polynucleotide sequence according to the first aspect.

Thus, the present invention provides novel immunogenic compositions protecting against mumps virus. The immunogenic composition according to the present invention contains genetically homogenous mumps viruses derived from the Jeryl Lynn strain.

The present invention provides a stable vaccine comprising said immunogenic composition protecting against mumps virus.

Such vaccine can be combined with measles, rubella and/ or varicella vaccine components to make combined vaccine.

The present invention provides a stable immunogenic composition derived from Jeryl Lynn 2 strain that can protect against mumps virus. Such an immunogenic composition is derived by using reverse genetics methodology.

Such an immunogenic composition is made by using various mammalian cells as a host to develop a stable vaccine against mumps virus.

Further, the disclosure provides methods for the development of said highly immunogenic and stable composition.

## DETAILED DESCRIPTION OF THE FIGURES

Figure 1 depicts chemically synthesized fragments of the genome of mumps virus Jeryl Lynn strain resolved on agarose gel. There are six fragments which covers full-length of genome of mumps virus attenuated JL2 strain. Here, combination of 1+2, 3+4 and 5+6 fragments are shown. It shows that fragments are appropriately obtained after the restriction digestion.

Figure 2 depicts restriction map of $pMuV^{JL2}$.

Figure 3 depicts syncytia formation in Vero cells after P0 passage.

Figure 4 depicts a) MRC5 cells without infection (control) b) MRC5 cells infected with $rMuV^{JL2}FL$.

Figure 5 depicts Vero cells fixed by TCA staining under UV light colored by crystal violet where A and B shows rMuV$^{JL2}$ syncytia.

## SUMMARY OF SEQUENCES

Sequence 1 is the nucleotide sequence of the first fragment obtained by the restriction digestion using restrictions sites *Not*I and *Rsr*II. Fragment 1 obtained from the digestion has position of 1-3576 nucleotide position of the full length DNA genome. (SEQ ID NO. 1)

Sequence 2 is the nucleotide sequence of the first fragment obtained by the restriction digestion using restrictions sites *Rsr*II and *Avr*II. Fragment 2 obtained from the digestion has position of 3576 - 6258 nucleotide position of the full length DNA genome. (SEQ ID NO. 2)

Sequence 3 is the nucleotide sequence of the first fragment obtained by the restriction digestion using restrictions sites *Avr*II and *Xho*I. Fragment 3 obtained from the digestion has position of 6258 - 8438 nucleotide position of the full length DNA genome. (SEQ ID NO. 3)

Sequence 4 is the nucleotide sequence of the first fragment obtained by the restriction digestion using restrictions sites *Xho*I and *Kpn*I. Fragment 4 obtained from the digestion has position of 8438 - 10498 nucleotide position of the full length DNA genome. (SEQ ID NO. 4)

Sequence 5 is the nucleotide sequence of the first fragment obtained by the restriction digestion using restrictions sites *Kpn*I and *Xma*I. Fragment 5 obtained from the digestion has position of 10498 - 13950 nucleotide position of the full length DNA genome. (SEQ ID NO. 5)

Sequence 6 is the nucleotide sequence of the first fragment obtained by the restriction digestion using restrictions sites *Xma*I and *Nar*I. Fragment 6 obtained from the digestion has position of 13950 - 15440 nucleotide position of the full length DNA genome. (SEQ ID NO. 6)

Sequence 7 is the complete nucleotide sequence of vector pMuV$^{JL2}$. (SEQ ID NO. 7)

Sequence 8 is the complete nucleotide sequence of rescued recombinant mumps virus rMuV$^{JL2}$FL. (SEQ ID NO. 8)

Sequence 9 is the nucleotide sequence encoding nucleoprotein (NP) of the mumps virus. (SEQ ID NO. 9)

Sequence 10 is the nucleotide sequence encoding phosphoprotein (P) of the mumps virus. (SEQ ID NO. 10)

Sequence 11 is the nucleotide sequence encoding large (L) protein of the mumps virus. (SEQ ID NO. 11)

Sequence 12 is the amino acid sequence of nucleoprotein (NP) of the mumps virus. (SEQ ID NO. 12)

Sequence 13 is the amino acid sequence of phospho protein (P) of the mumps virus. (SEQ ID NO. 13)

Sequence 14 is the amino acid sequence of large protein (L) of the mumps virus. (SEQ ID NO. 14)

Sequence 15 is the nucleotide sequence of pSC6-T7 encoding T7 RNA polymerase. (SEQ ID NO. 15)

## DETAILED DESCRIPTION OF THE INVENTION

In one of the embodiments, the present invention provides stable JL2 strain based immunogenic composition that can be used to produce a vaccine against mumps. The isolated JL2 strain is genetically homogenous and highly immunogenic. The isolated JL2 strain disclosed here is safe and efficient in terms of antibody production against mumps virus.

In a further embodiment, the present invention provides full length genome of JL2 strain developed from reverse transcribed m-RNA of JL2 strain. Full length JL2 genome was synthesized chemically in six different fragments to cover the total genome length (15,384 bp) of mumps virus. Such chemically synthesized genome fragments can be amplified using suitable plasmid vectors (intermediate vectors). Full length genome can be synthesized in less than or more than six fragments illustrated here using restriction digestion method.

The present disclosure provides method of cloning of chemically synthesized fragments of JL2 genome in suitable vector. Trans-acting viral proteins NP (nucleoprotein), P (phosphoprotein) and L (large protein) encoding genes of mumps virus and gene encoding T7 RNA polymerase are cloned into helper plasmids.

Trans-acting viral proteins can be defined as viral proteins essentially required for viral encapsidation, transcription and translation.

In a preferred embodiment, trans-acting viral proteins comprise nucleotide sequences as set forth in SEQ ID NO. 9 to 11.

In a more preferred embodiment, trans-acting viral proteins comprise amino acid sequences as set forth in SEQ ID NO. 12 to 14.

In a further embodiment, T7 RNA Polymerase containing vector comprises nucleotide sequence as set forth in SEQ ID NO. 15.

In yet another embodiment, according to the present invention vectors can be selected from pBluescript, pBluescript II, pBluescript II SK (+), pUC19, pCA, pSC6 and others like. These vectors are commercially available and known to the person skilled in the art.

In an embodiment, a vector containing full length genome of isolated JL2 strain is described. The present disclosure provides method to construct plasmid comprising the full length genome of JL2 ($pMuV^{JL2}$) by step wise cloning of chemically synthesized fragments of JL2 genome using pBluescript II SK (+) as final transcription vector.

The transcription vector comprises an operably linked transcriptional unit comprising an assembly of a genetic element or elements having a regulatory role in the expression, for example, a promoter, a structural gene or coding sequence which is transcribed into mumps RNA, and appropriate transcription initiation and termination sequences. All cloning procedures of the present invention are basically as described by Sambrook and Russel (2001). The present disclosure provides a process for rescue of JL2 strain in which JL2 strain propagate in suitable host cell(s). Here, host cells can be suitable mammalian cells or non-mammalian cells which are stable and consistent in terms of quality and productivity. It is already known that passaging of Jeryl Lynn strain in embryonated chicken eggs (ECE) resulted in the rapid accumulation of JL2 (over 90%) and loss of JL1, suggesting that JL2 has a strong selection advantage in ECE. But, ECE cells are primary cells which are not consistent and reproducible. So, it becomes difficult to maintain desired titers of JL2 stain using such primary cells.

The present invention provides for the production of an immunogenic composition exclusively by using mammalian cell. Such mammalian cell can be selected from Vero cell, MRC-5 cell, HEK 293T cell and other similar secondary or tertiary cell lines and non-mammalian cells such as *E.coli* and others like can be used. An immunogenic composition which is stable and sufficient immunogenic at a production level is not available in the art. Therefore, the immunogenic composition using such mentioned host which can be used for vaccine preparation against mumps virus is not available in the market. The present invention is expected to satisfy the current demand.

In a preferred embodiment, the present disclosure provides transfection of suitable mammalian host cells using plasmid containing full length mumps JL2 genome ($pMuV^{JL2}$) and helper plasmids containing genes encoding trans-acting viral proteins NP, P, L and plasmid containing T7 polymerase gene. Transfected cells are incubated to allow syncytia formation.

In a more preferred embodiment, helper plasmids encoding Nucleoprotein (NP) denoted as $p_N^{JL2}$, phosphor protein (P) denoted as $p_P^{JL2}$, large protein (L) denoted as $p_L^{JL2}$ and plasmid encoding T7 Polymerase denoted as pSC6-T7 (SEQ ID NO. 15) are used.

Plasmids expressing trans-acting viral proteins and T7 polymerase that are used to co-transfect the host cells along with $pMuV^{JL2}$ can be defined as helper plasmids. These are essential for viral encapsidation, transcription and translation.

Syncytia can be defined as multi-nucleated enlarge cells resulting from fusion of infected cell with neighboring cells mediated by expression of viral proteins.

These syncytia are used to infect a different mammalian host cells for viral propagation (passage P0). Viral progenies are rescued after P0 passage ($rMuV^{JL2}FL$). Passage can be defined as sub culturing of rescued virus on host cells. Following the first passage, fresh mammalian cells are infected with passage P0 for subsequent passages (PI and P2). After the final passage, rescued viruses are stored as viral stock (master seed).

In another embodiment, the present invention provides rescue composition comprising following elements:

a. Transcription vector comprising full-length genome of JL2 strain.
b. An expression vector comprises isolated gene(s) encoding trans-acting protein(s) wherein trans-acting protein is selected from NP protein, P protein, L protein and combination thereof.
c. RNA polymerase for appropriate transcription of full-length genome of JL2 strain.

Here, according to the present invention, RNA polymerase is preferably T7 RNA polymerase.

In yet another embodiment, rescued viruses are characterized by sequencing methods known in the art, more preferably primer walking sequencing method.

In an embodiment, the present invention provides novel, stable and highly immunogenic composition comprising isolated JL2 strain that can be used for the development of vaccine against mumps virus.

Such an immunogenic composition comprises genetically homogenous JL2 strain. The stable immunogenic composition according to the present invention is free from MuVJL5 component strain which is a part of conventional vaccine against mumps virus. Thus, the present invention provides stable pure and highly immunogenic composition which can protect against mump virus antigen which is conventionally protected using combination of JL2 and JL5 strains.

In a preferred embodiment, the host cell is selected from Vero cells and MRC-5 cells.

As discussed earlier, conventional JL2 strain is known to grow well in primary chicken embryo derived cells. The drawbacks of using embryonated egg cells lies in the risk of residual egg derived proteins and other unknown extraneous agents including other viruses. Such residual egg derived proteins may be cause of inducing undesired allergic reactions in the vaccinated individuals. Moreover, presence of unknown other viruses may induce an additional risk for the vaccine. Inventors of the present invention have developed a stable, homogenous and pure isolated JL2 strain and composition comprising it using Vero cells and MRC-5 cells that can be used for the development of stable vaccine against mumps virus.

In a further embodiment, the present invention provides a stable vaccine comprising genetically homogenous and heat stable JL2 strain.

In a further embodiment, the present vaccine provides combination vaccine containing measles, rubella, varicella and other live virus vaccine strains along with the Jeryl Lynn vaccine of the present invention.

The present disclosure provides method to develop said immunogenic composition using conventional reverse genetics methodology. It includes cloning method to develop full-length genome of JL2 strain of mumps virus. Such a full-length genome of JL2 strain is reverse transcribed from the original m-RNA of the isolated Jeryl Lynn strain. Stable syncytia formation will take place as a resultant cytopathic effect from the reverse transcribed JL2 strain of the present invention. Such syncytium obtained from isolated JL2 strain of the present invention is genetically homogenous and highly immunogenic. Such immunogenic vaccine strain can be grown in production cell culture using different mammalian cell lines. Inventors found cell substrates such as Vero cells, MRC-5 cells or HEK 293T cells as compared to CEF primary cells which are used in classical Jeryl Lynn vaccine, are better in cell propagation in multiple passages with lower cost of production.

The immunogenic composition obtained using mentioned mammalian cell lines is consistent in terms of quality and susceptibility to produce desired titers of JL2 stain while, it is not reproducible in case of CEF primary cells.

Moreover, the immunogenic composition according to the present invention helps to avoid risk of contamination of new adventitious agents from cell substrates by using suitable stable secondary cell line which is not in case of any primary cells. It means the immunogenic composition as described herein is commercially viable.

The method to develop said immunogenic composition according to the present disclosure comprising cloning strategy which includes use of a suitable vector to transfect suitable host cell.

The present disclosure provides a method for producing a recombinant mumps virus comprising following step:

a. Chemical synthesis of genome sequence fragments of mumps virus attenuated JL2;
b. Cloning of each obtained gene fragment into an intermediate vector for its amplification;
c. Construction of clone of full-length DNA genome of the mumps virus attenuated JL2 strain to obtain pMuVJL2 vector through ligation each gene fragment into single vector;
d. Co-transfection of host cell with pMuVJL2 and an expression vector comprising isolated gene encoding trans-acting protein wherein trans-acting protein is selected from NP protein, P protein and L protein to negative strand of mumps virus rescue;
e. Optionally, passaging of rescued mumps virus into host cell for its expansion.

In a furthermore embodiment, the present invention provides a pharmaceutical composition comprising an immunogenic composition with suitable pharmaceutically acceptable carrier or excipient.

In one of the embodiment, the present invention provides a pharmaceutical composition comprising an immunogenic composition with suitable buffer, stabilizer, tonicity agent, surfactant and cryoprotectant.

In one of the embodiments, buffer can be selected from Phosphate-buffer, histidine-buffer, citrate-buffer, succinate-buffer, acetate-buffer, arginine buffer, phosphate buffered saline, tromethamine buffer and others like, preferably phosphate buffer.

In one of the embodiments, stabilizers can be selected from amino acid(s), sugar(s), polyol(s) and their combination.

In one of the embodiments, amino acids can be selected from arginine, glycine, lysine, histidine, glutamic acid, aspartic acid, isoleucine, leucine, alanine, phenylalanine, tyrosine, tryptophane, methionine, serine, proline, cysteine / cystine and suitable combination thereof.

In the present invention, sugars can be selected from glucose, fructose, galactose, mannose, sorbose, ribose, deoxyribose, sucrose, trehalose, lactose, maltose, raffinose and suitable mixtures thereof, preferably trehalose or sorbitol or mannitol.

Further, polyol can be selected from mannitol, sorbitol, dextran, glycerol, arabitol, propylene glycol, polyethylene glycol and suitable combinations thereof, preferably sorbitol.

In one of the embodiments, suitable tonicity agents can be selected from sugar(s), salt(s) and their combination for said pharmaceutical composition.

In one of the embodiments, suitable salts can be selected from tonicity agent such as NaCl or KCl.or others like.

In the present invention, surfactant can be selected from polyoxyethylensorbitan fatty acid esters (Tween), polyoxyethylene alkyl ethers, alkylphenylpolyoxyethylene ethers, polyoxyethylene-polyoxypropylene copolymer and sodium dodecyl sulphate (SDS) and combination thereof.

Further, cryoprotactant can be selected from sugars and others like.

In further embodiment, the present invention provides an immunogenic composition containing recombinant JL2 (rMuVJL2FL) with suitable adjuvant.

An adjuvant can be defined as a substance that is added to a vaccine to increase the body's immune response to the vaccine.

Adjuvants according to the present invention can be selected from salts of aluminium, Mono Phosphoryl Lipid (MPL) analogues such as GLA (glucopyranosyl lipid adjuvant), monatide, cytokine inducers, and squalene based adjuvants, lipophilic adjuvants and others like.

In a preferred embodiment, the present invention provides vaccines containing immunogenic composition of the present invention against Jeryl Lynn. These vaccines can be administered in conventional routes and dosages.

## Analytical methods used in present invention

1. RT-PCR: Reverse transcription polymerase chain reaction (RT-PCR) is the method used for synthesis and amplification of complementary DNA by reverse transcribing the RNA. RT-PCR uses reverse transcriptase enzyme and a primer to anneal and extend a desired RNA sequence. If the RNA is present, the reverse transcriptase and primer will anneal to the RNA sequence and transcribe a complimentary strand of DNA (cDNA). This strand is then replicated with primers and Taq Polymerase, and the standard PCR protocol is followed. This protocol copies the single stranded DNA millions of times in a small amount of time to produce a significant amount of DNA. The PCR products (the cDNA strands) are then separated with agarose gel electrophoresis.

2. Agarose gel electrophoresis: Agarose gel electrophoresis is a method of gel electrophoresis used in biochemistry, molecular biology, genetics, and clinical chemistry to separate a mixed population of DNA or proteins in a matrix of agarose.

3. Primer walking sequencing method: Primer walking is a sequencing method of choice for sequencing large DNA fragments. Fragments which are too long to be sequenced in a single sequence read using the chain termination method, this method works by dividing the long sequence into several consecutive short ones. The term "primer walking" is used where the main aim is to sequence the genome.

4. Multiple sequence analysis: Multiple sequence analysis is a method used for analysis of biological sequences to determine the region of similarity and difference between the sequences which are aligned for analysis.

## EXAMPLES

The following non-limiting examples describe rescue of JL2 from genomic and structural plasmids co-transfection to suitable mammalian cell. Further, isolation of pure and genetically homogenous JL2 strain by using limiting dilution method is described herein below.

### Example 1: Construction of the clone of full-length DNA genome of the mumps virus attenuated JL2 strain

Generation of Full length cDNA of mumps JL2

Six genome sequence fragments were chemically synthesized (GeneArt-Thermo Fisher Scientific) to cover the total genome length (15,384 bp) of the mumps virus. Subsequently, each genome fragment and vector was cut by specific restriction endonuclease (New England Bio Labs) and cloned in an intermediate vectors. Each fragment was verified by resolving them on agarose gel as shown in Figure 1. Fragments obtained after restriction digestion have been described below:

Fragment 1: pos. 1 - 3576 *Not*I-*Rsr*II (SEQ ID NO.1);
Fragment 2: pos. 3576 - 6258 *Rsr*II-*Avr*II (SEQ ID NO. 2);
Fragment 3: pos. 6258 - 8438 *Avr*II-*Xho*I (SEQ ID NO.3);
Fragment 4: pos. 8438 - 10498 *Xho*I-*Kpn*I (SEQ ID NO. 4);
Fragment 5: pos. 10498 - 13950 *Kpn*I-*Xma*I (SEQ ID NO. 5);
Fragment 6: pos. 13950 - 15440 *Xma*I-*Nar*I (SEQ ID NO. 6)

Restriction sites mentioned in the present example are well known to the skilled person. A person skilled in the art can use alternative restriction sites as per his requirement.

Cloning of obtained fragments into transcription vector and ligation of fragments

Chemically synthesized and restriction endonuclease treated fragments were incorporated into the plasmid by stepwise cloning procedures, using a modified pBluescript II SK (+) as final vector. Progressive ligation of the fragments to each other through compatible cohesive ends led to the definitive $pMuV^{JL2}$ plasmid. Vector map of $pMuV^{JL2}$ plasmid has been depicted in Figure 2. The plasmid $pMuV^{JL2}$ has nucleotide sequence as set forth in SEQ ID NO. 7. Helper plasmids containing genes for structural proteins of mumps virus and plasmid containing T7 RNA polymerase gene were generated by cloning in suitable vector. Here, pSC6 vector has been used for cloning of genes encoding trans-acting proteins and T7 RNA polymerase. Cloning as described in the present application has been carried out as described in Sambrook and Russel (2001).

**Example 2: Rescue of attenuated mumps Jeryl-Lynn 2 virus**

Commercially available HEK 293T cells were transfected by $pMuV^{JL2}$ together with helper plasmids $p_N^{JL2}$, $p_P^{JL2}$, $p_L^{JL2}$ and pSC6-T7. HEK 293T cells were seeded into a 35mm well to reach ~ 50-70% confluence when being transfected. Confluence is the term commonly used as an estimate of the number of adherent cells in a culture dish or a flask, referring to the proportion of the surface which is covered by cells monolayer. 4 hour before transfection, the medium was replaced with 3 ml DMEM containing 10% FCS. All recombinant plasmids were prepared according to the QIAGEN plasmid preparation kit. The kit for the $Ca^{2+}$ phosphate coprecipitation of DNA was from Invitrogen.

Cells were co-transfected with the plasmids in the following final concentration:

| | |
|---|---|
| $p_N^{JL2}$ | 0.5 μg, |
| $p_P^{JL2}$ | 0.1 μg, |
| $p_L^{JL2}$ | 0.5 μg, |
| pSC6-T7 | 1.0 μg, |
| $pMuV^{JL2}$ | 5.0 μg |

Mentioned plasmids diluted in H2O were added in an Eppendorf tube containing 2M $CaCl_2$, the mix was added to another Eppendorf tube containing HEPES buffer under shaking conditions, and was incubated 30 min at room temperature (RT). The co-precipitates were added drop wise to the culture and the transfection was carried out at 37°C and 5% CO2 for about 18 hour. Subsequently, the transfection medium was replaced with 3.0 ml of DMEM without FCS. Transient expression of the T7 polymerase permits the efficient protein expression of T7-promoted genes, finally led to negative strand of mumps virus rescue. Transfected cells were monitored for about 15 days till syncytia formation. Syncytia formation from single plaque has been shown in Figure 3. Single syncytia was picked up to infect WHO-approved Vero cells to establish passage 0 (P0) of the virus.

The rescued recombinant attenuated virus is named as **$rMuV^{JL2}FL$**. The nucleotide sequence of $rMuV^{JL2}FL$ is as set forth in SEQ ID NO. 8.

Analytical sequencing of rescued mumps virus

After about 15 days post transfection, as soon as first syncytia appeared, single clone was aspirated under the microscope and subsequently used for characterization. Characterization was carried out by sequencing of the rescued rMuV$^{JL2}$FL virus genome. Viral RNA from rMuV$^{JL2}$FL infected Vero cells has been prepared following Qiagen RNeasy Mini kit. Starting from 2.0 μl of extracted viral RNA, reverse transcription and amplification have been sequentially carried out, following Qiagen OneStep RT-PCR kit instructions.

34 couples of primers, covering the full length genome sequence (15,384 bp) have been designed in order to amplify overlapping PCR fragments. Purified PCRs have been sequenced following a primer walking sequencing procedure.

Value Read sequences have been assembled covering the entire rMuV$^{JL2}$FL viral genome. 6 point mutations have been detected in the L gene of the rescued rMuV$^{JL2}$FL with respect to the sequence of the original plasmid pMuV$^{JL2}$, and confirmed by multiple sequence analysis.

Table-1: Point mutations and amino acid change in the L gene of the rescued rMuV$^{JL2}$ FL

| Nucleotide position | Position of mutation in codon | pMuV$^{JL2\ (Synthetic)}$ | rMuV$^{JL2}$FL $^{(Rescued)}$ | Amino acid change |
|---|---|---|---|---|
| 10441 | 3 | T | C | None |
| 11467 | 3 | T | C | None |
| 11603 | 1 | T | C | Silent |
| 11809 | 3 | T | C | None |
| 12884 | 1 | A | G | Ser to Gly |
| 12981 | 2 | A | G | Gln to Arg |

**Example 3: Expansion of rescued mumps JL2 virus**

P0 passage of the rescued rMuV$^{JL2}$FL virus was propagated in WHO-approved Vero cells. Vero cells, maintained as monolayers in DMEM supplemented with 5% FBS and when confluence reached at 70-80%, Vero cells have been infected by P0 passage of the rescued rMuV$^{JL2}$FL virus using 0.5-1.0 mL of the rescued virus in a 5.0 mL total volume of OPTIMEM. Cells were incubated for 1 hour at 37°C and 5% CO2. After incubation, the inoculum was replaced by DMEM + 5% FBS. Infected cells have been split after 2 days, and 90-100% syncytia formation was observed after 5-dpi. Cell-free (CF) and cell-associated (CA) fractions have been collected to obtain lab-scale viral stocks, reaching titers between 1 x $10^4$ pfu/mL in the collected cell-free fractions (CF) and 1 x $10^6$ pfu/mL in the collected cell-associated fractions (CA).

**Example 4: Preparation of master cell bank of rescued mumps JL2 virus and production of single virus harvest**

Rescued mumps virus from P0 passage was further passaged till passage 2 (P2) in Vero cells to obtain sufficient master seed.

Titers obtained after each passage in Vero cells is given below:

P0 passage: titers between 1 x $10^3$ pfu/mL (CF, Cell-free fraction) - 1 x $10^4$ pfu/mL (CA, Cell-associated fraction).
P1 passage: titers between 1 x $10^4$ pfu/mL (CF, Cell-free fraction) - 5 x $10^4$ pfu/mL (CA, Cell-associated fraction).
P2 passage: titers between 1 x $10^4$ pfu/mL (CF, Cell-free fraction) - 1 x $10^6$ pfu/mL (CA, Cell-associated fraction).

Such master seed is stored at a temperature between -150°C to -196°C. This master seed will be used to produce working seed for the preparation of commercial vaccine for human use. Working seed obtained from the master seed is used for production of single virus harvest in Vero cells. Working seed obtained from the master seed can be used for production of single virus harvest in MRC5 cells. Production of single virus harvest in MRC5 cell is also illustrated herein below.

Production of single virus harvest in MRC5 cells

**Propagation of cells**

The media was removed and cells were washed with MEM EBS media and TrypLE solution was added (Trypsin replacement) at 1ml/ T75 cm2 and 10ml/RB 850 cm2 and was incubated at 35±2°C. Cells were re-suspended after detachment in fresh medium and were seeded into new Culture flasks and roller bottles. The fully confluent T75 flask was split at a ratio of 1:4 to 1:16in T 75, T 175 and RB 850 roller bottles respectively in subsequent passages depending upon the batch size. Cells were split once a week and after attaining sufficient confluence. Subsequently, cells were used for virus inoculation.

**Single virus harvest production**

Single virus harvest - Mumps was produced by infecting MRC-5 cells with working virus seed and incubating at 33 ± 2°C for 60-120 minutes for virus adsorption. After adsorption, each infected roller bottle was replenished with 250-300mL of Creek's Minimal Medium (CMM) and incubated at 33±2°C at 0.5 rpm for 40 to 70 hrs.in roller apparatus. After completion of the incubation, all the roller bottles were observed for clarity and detachment of monolayer. Monolayer was washed with plain MEM media and replenished with 100-200 mL plain MEM media. The bottles were again incubated at 33 ± 2 °C at 0.5 rpm for 5 to 13 days in roller apparatus. At the end of incubation all the roller bottles were observed for the clarity and presence of cytopathic effects and harvest was collected from all roller bottles in a single container. Human serum albumin was used as stabilizing agent for collected Virus harvest. The same was then distributed in individual pre sterilized PETG bottles as single harvest. The Bulk thus obtained was clarified and diluted to required concentration to produce Mumps vaccine. MRC5 cells without infection (control) and MRC5 cells infected with $rMuV^{JL2}FL$ are shown as (a) and (b) respectively in Figure 4.

The Produces Mumps Vaccine (JL) was tested for potency and found to be in range of $10^{4.12}$/ml to $10^{4.57}$/ml $CCID_{50}$ (Cell Culture Infectious Dose which infect 50% of the cultured cells) tested by cell culture method.

**Example 5: Identification and quantification of mumps JL2 strain by plaque assay and in situ immunofluorescence**

<u>Virus titration by plaque assay</u>

Serial ten-fold dilutions of virus preparations were carried out using OPTIMEM to a final volume of 0.5 ml. Each dilution was added on 35 mm Vero cell cultures. After 1 h of virus adsorption, the inoculum was removed and the infected cells were overlaid with 2.0 ml of DMEM containing 5% FCS and 1% low melting point agarose (LMP agarose). After 5 days of incubation at 37°C and 5% CO2, cultures were fixed with 1ml of 10% TCA for 1 h, then UV cross-linked for 30 min. After removal of the agarose overlay, cell monolayers were stained with crystal violet dissolved in 4% ethanol, washed with water and the plaques were counted under the inverted microscope. Microscopic image is given as Figure 5.

<u>In situ immunofluorescence</u>

The various ten-fold dilutions of master seed were prepared from the master seed virus in MEM (minimum Essential Medium) medium containing 2% FBS (Fetal Bovine Serum). Subsequently, Vero cell suspension was prepared at a concentration range between 0.15-0.20 million/mL. This cell suspension was poured in 96-well plate over above virus dilutions and kept it at 37 °C with 5% CO2. The plate was incubated under same condition for ten days. After ten days, the cell supernatant from the plate was discarded and washed with PBS (Phosphate Buffer Saline). The cells in plate were fixed with acetone methanol fixation buffer. Anti-Mumps IgG antibodies conjugated with FITC (fluorescein isothiocyanate) was added in working dilution of 1:50. Virus titer was determined by counting the stained cells under fluorescence microscope using Spearman Karber formula.

The Spearman-Kärber formula:

$$\log 10\ (\text{end-point dilution}) = x0 - \frac{d}{2} + d \sum \frac{ri}{ni}$$

x0 = (log10 of the lowest dilution with all wells positive) d = log10 of the dilution step, one in this case ni = number of replicates, six in this case ri = number of positive wells

**Example 6: Formulation of Immunogenic composition**

Required quantity of Single harvest Of Mumps Vaccine was calculated to get the target titer of 1000 $CCID_{50}$. Similarly required quantity of raw material (Stabilizer) was calculated to formulate the vaccine of desired batch size according to the composition of finished product. Stabilizer 176 (Combination mixture of Gelatin Sorbitol & other amino acids) & M199 (diluent) as per calculated quantity was transferred in to a sterile container and to that calculated amount of Single harvest Of Mumps Vaccine was added and mixed.

The final formulation is as described below:

| Material Name | Single dose Qty. / dose | Used as |
|---|---|---|
| Single harvest Mumps | ≥ 1000 $CCID_{50}$ | Antigen |
| Stabilizer 176 | 0.25 ml | Stabilizer |
| Medium - M199 | Q.S. | Diluent |

The final formulation was filled 0.5ml/vials are half stoppered and lyophilized with a standard cycle where quick freezing to -60° C, primary drying was done at -40°C and secondary drying at 37 °C . The round intact cake was found which reconstitutes in less than 1 minutes and moisture test comes under 1.6% - 2 %.

The Produced Mumps Vaccine (JL) Vaccine was been tested for potency and found to be in range of $10^3$/dose to $10^{3.57}$/ml $CCID_{50}$ tested by cell culture method.

The immunogenic composition of the present invention is tested for determination of immunogenicity of rescued mumps virus by standard immune assay methods. Examples of such immune assay method are ELISA (Enzyme Linked Immunosorbent Assay) and other standard methods used for determination of mumps virus neutralizing antibody. Such assay can be used to determine immunogenicity of rescued virus in non-primates or primates. Such studies can be useful to determine optimum dose and dose regime for the said vaccine. The immunogenic composition of the present invention having rescued mumps virus is found to be suitable for further vaccine preparation. The process for producing rescued mumps virus according to the present invention does not involve use of any element from any other viral or non-viral pathogenic entity. Thus, the rescued mumps virus is stable and homogenous and therefore devoid of pathogenic particles other than mumps virus. Therefore rescued mumps virus according to the present invention is suitable candidate for vaccine development.

## Claims

1. An isolated full-length genome of mumps virus attenuated JL2 strain which comprises the polynucleotide sequence having SEQ ID NO.8.

2. A vector comprising the polynucleotide sequence as claimed in claim 1, and, optionally, wherein the vector is selected from pUC19, pBluescript, pBluescript II, pBluescript II SK (+), pCA or pSC6.

3. A host cell comprising vector as claimed in claim 2, and, optionally, wherein the host cell is selected from a mammalian cell or a non-mammalian cell.

4. The host cell as claimed in claim 3 wherein the mammalian cell is selected from HEK 293T cell, Vero cell or MRC-5 cell, and the non-mammalian cell is selected from bacterial cell or yeast cell.

5. A mumps virus vaccine comprising the full-length genome of mumps virus attenuated JL2 strain as claimed in claim 1.

6. An immunogenic composition containing the full-length genome of mumps virus attenuated JL2 strain as claimed in claim 1 with suitable pharmaceutical excipients, and optionally, wherein the pharmaceutical excipients are selected from buffer(s), stabilizer(s), tonicity agent(s), surfactant(s) adjuvant(s), cryoprotectant(s) or combination thereof.

7. The immunogenic composition as claimed in claim 6 wherein

(a) Buffer is selected from phosphate-buffer histidine-buffer, citrate-buffer, succinate-buffer, acetate-buffer, arginine buffer, phosphate buffered saline, tromethamine buffer and mixtures thereof;
(b) Stabilizer is selected from amino acid(s), sugar(s), polyol(s), or combination thereof;
(c) Tonicity agent is selected from sugar(s), salt(s) or combination thereof;
(d) Surfactant is selected from polyoxyethylensorbitan fatty acid esters (Tween), polyoxyethylene alkyl ethers, alkylphenylpolyoxyethylene ethers, polyoxyethylene-polyoxypropylene copolymer and sodium dodecyl sulphate (SDS) or combination thereof;
(e) Adjuvant is selected from salts of aluminium, Mono hosphoryl Lipid analogues such as glucopyranosyl lipid adjuvant, monatide, cytokine inducers, squalene based adjuvants, lipophilic adjuvants or combination thereof; and
(f) Cryoprotectant is sugar(s).

8. The immunogenic composition as claimed in claim 7 comprises stabilizers selected from amino acid(s), sugar(s), polyol(s), or combination thereof, buffer(s) or salt(s).

9. The immunogenic composition as claimed in claim 8 wherein

- Buffer is selected from phosphate-buffer histidine-buffer, citrate-buffer, succinate-buffer, acetate-buffer, arginine buffer, phosphate buffered saline, tromethamine buffer and mixtures thereof;
- Amino acid is selected from arginine, glycine, lysine, histidine, glutamic acid, aspartic acid, isoleucine, leucine, alanine, phenylalanine, tyrosine, tryptophane, methionine, serine, proline, cysteine / cystine and suitable combination thereof;
- Sugar is selected from glucose, fructose, galactose, mannose, sorbose, ribose, deoxyribose, sucrose, trehalose, lactose, maltose, raffinose and suitable mixtures thereof preferably trehalose or raffinose;
- Polyol is selected from mannitol, sorbitol, dextran, glycerol, arabitol, propylene glycol, polyethylene glycol and suitable combinations thereof;
- Salt is selected from NaCl or KCl.

10. A rescue composition comprising the following elements:

a. Transcription vector comprising the polynucleotide sequence as claimed in claim 1;
b. An expression vector comprises isolated gene(s) encoding trans-acting protein(s) wherein the trans-acting protein is selected from NP protein, P protein, L protein and combination thereof;
c. RNA polymerase for appropriate transcription of the polynucleotide sequence as claimed in claim 1.

11. The composition of claim 10, wherein the gene(s) encoding the trans-acting protein is selected from SEQ ID NO. 9 to 11.

12. The composition of claim 10 or 11, wherein the amino acid sequence of the trans-acting proteins are selected from SEQ ID NO. 12 to 14.

## Patentansprüche

1. Isoliertes Genom vollständiger Länge vom Mumpsvirus-attenuierten JL2-Stamm, das die Polynukleotidsequenz umfasst, die SEQ.-ID Nr. 8 aufweist.

2. Vektor, der die Polynukleotidsequenz nach Anspruch 1 umfasst und optional wobei der Vektor aus pUC19, pBluescript, pBluescript II, pBluescript II SK (+), pCA oder pSC6 ausgewählt ist.

3. Wirtszelle, die den Vektor nach Anspruch 2 umfasst und optional wobei die Wirtszelle aus einer Säugerzelle oder einer Nicht-Säugerzelle ausgewählt ist.

4. Wirtszelle nach Anspruch 3, wobei die Säugerzelle aus HEK-293T-Zelle, Vero-Zelle oder MRC-5-Zelle ausgewählt ist und die Nicht-Säugerzelle aus Bakterienzelle oder Hefezelle ausgewählt ist.

5. Mumpsvirus-Impfstoff, der das Genom vollständiger Länge vom Mumpsvirus-attenuierten JL2-Stamm nach Anspruch 1 umfasst.

6. Immunogene Zusammensetzung, die das Genom vollständiger Länge vom Mumpsvirus-attenuierten JL2-Stamm nach Anspruch 1 mit geeigneten pharmazeutischen Hilfsstoffen enthält und optional wobei die pharmazeutischen Hilfsstoffe aus Puffer(n), Stabilisator(en), Tonizitätsmittel(n), Tensid(en) Adjuvans/Adjuvantien, Kryoprotektivum/Kryoprotektiva oder einer Kombination davon ausgewählt sind.

7. Immunogene Zusammensetzung nach Anspruch 6, wobei

(a) Puffer aus Folgenden ausgewählt ist: Phosphatpuffer Histidinpuffer, Citratpuffer, Succinatpuffer, Acetatpuffer, Argininpuffer, phosphatgepufferter Kochsalzlösung, Tromethaminpuffer und Mischungen davon;
(b) Stabilisator aus Aminosäure(n), Zucker(n), Polyol(en) oder einer Kombination davon ausgewählt ist;
(c) Tonizitätsmittel aus Zucker(n), Salz(en) oder einer Kombination davon ausgewählt ist;
(d) Tensid aus Folgenden ausgewählt ist: Polyoxyethylensorbitan-Fettsäureestern (Tween), Polyoxyethylenalkylethern, Alkylphenylpolyoxyethylenethern, Polyoxyethylen-Polyoxypropylen-Copolymer und Natriumdodecylsulfat (SDS) oder einer Kombination davon;
(e) Adjuvans aus Folgenden ausgewählt ist: Salzen von Aluminium, Monophosphoryl-Lipid-Analoga, wie zum Beispiel Glucopyranosyl-Lipid-Adjuvans, Monatid, Zytokin-Induktoren, Squalen-basierten Adjuvantien, lipophilen Adjuvantien oder einer Kombination davon; und
(f) Kryoprotektivum Zucker ist.

8. Immunogene Zusammensetzung nach Anspruch 7, die Stabilisatoren umfasst, die aus Aminosäure(n), Zucker(n), Polyol(en) oder einer Kombination davon, Puffer(n) oder Salz(en) ausgewählt sind.

9. Immunogene Zusammensetzung nach Anspruch 8, wobei

- Puffer aus Folgenden ausgewählt ist: Phosphatpuffer Histidinpuffer, Citratpuffer, Succinatpuffer, Acetatpuffer, Argininpuffer, phosphatgepufferter Kochsalzlösung, Tromethaminpuffer und Mischungen davon;
- Aminosäure aus Folgenden ausgewählt ist: Arginin, Glycin, Lysin, Histidin, Glutaminsäure, Asparaginsäure, Isoleucin, Leucin, Alanin, Phenylalanin, Tyrosin, Tryptophan, Methionin, Serin, Prolin, Cystein/Cystin und einer geeigneten Kombination davon;
- Zucker aus Folgenden ausgewählt ist: Glucose, Fructose, Galactose, Mannose, Sorbose, Ribose, Desoxyribose, Saccharose, Trehalose, Lactose, Maltose, Raffinose und geeigneten Mischungen davon, bevorzugt Trehalose oder Raffinose;
- Polyol aus Folgenden ausgewählt ist: Mannitol, Sorbitol, Dextran, Glycerol, Arabitol, Propylenglycol, Polyethylenglycol und geeigneten Kombinationen davon;
- Salz aus NaCl oder KCl ausgewählt ist.

10. "Rescue"-Zusammensetzung, die die folgenden Elemente umfasst:

a. Transkriptionsvektor, der die Polynukleotidsequenz nach Anspruch 1 umfasst;
b. einen Expressionsvektor, der isoliertes/isolierte Gen(e) umfasst, das/die trans-wirkende(s) Protein(e) kodiert/kodieren,
wobei das trans-wirkende Protein aus NP-Protein, P-Protein, L-Protein und einer Kombination davon ausgewählt ist;
c. RNA-Polymerase für die angemessene Transkription der Polynukleotidsequenz nach Anspruch 1.

11. Zusammensetzung nach Anspruch 10, wobei das/die Gen(e), das/die das trans-wirkende Protein kodiert/kodieren, aus SEQ.-ID Nr. 9 bis 11 ausgewählt ist/sind.

12. Zusammensetzung nach Anspruch 10 oder 11, wobei die Aminosäuresequenz der trans-wirkenden Proteine aus SEQ.-ID Nr. 12 bis 14 ausgewählt ist.

## Revendications

1. Génome complet isolé de la souche JL2 atténuée du virus ourlien qui comprend la séquence polynucléotidique ayant la SEQ ID NO : 8.

**2.** Vecteur comprenant la séquence polynucléotidique selon la revendication 1, et, optionnellement, où le vecteur est sélectionné parmi pUC19, pBluescript, pBluescript II, pBluescript II SK (+), pCA ou pSC6.

**3.** Cellule hôte comprenant le vecteur selon la revendication 2, et, optionnellement, où la cellule hôte est sélectionnée parmi une cellule mammalienne ou une cellule non mammalienne.

**4.** Cellule hôte selon la revendication 3, où la cellule mammalienne est sélectionnée parmi une cellule HEK 293T, une cellule Vero ou une cellule MRC-5, et la cellule non mammalienne est sélectionnée parmi une cellule bactérienne ou une cellule de levure.

**5.** Vaccin à virus ourlien comprenant le génome complet de la souche JL2 atténuée du virus ourlien selon la revendication 1.

**6.** Composition immunogène contenant le génome complet de la souche JL2 atténuée du virus ourlien selon la revendication 1 avec des excipients pharmaceutiquement appropriés et optionnellement, où les excipients pharmaceutiques sont sélectionnés parmi un/des tampon(s), stabilisant(s), agent(s) de tonicité, tensioactif(s), adjuvant(s), cryoprotecteur(s) ou une combinaison de ceux-ci.

**7.** Composition immunogène selon la revendication 6, dans laquelle

(a) Le tampon est sélectionné parmi un tampon phosphate, tampon histidine, tampon citrate, tampon succinate, tampon acétate, tampon arginine, solution saline à tampon phosphate, tampon trométhamine et des mélanges de ceux-ci ;
(b) Le stabilisant est sélectionné par un/des acide(s) aminé(s), sucre(s), polyol(s) ou une combinaison de ceux-ci ;
(c) L'agent de tonicité est sélectionné parmi un/des sucre(s), sel(s) ou une combinaison de ceux-ci ;
(d) Le tensioactif est sélectionné parmi des esters d'acides gras de polyoxyéthylène sorbitan (Tween), polyoxyéthylène alkyl éthers, alkylphénylpolyoxyéthylène éthers, copolymère de polyoxyéthylène-polyoxypropylène et dodécyl sulfate de sodium (SDS) ou une combinaison de ceux-ci ;
(e) L'adjuvant est sélectionné parmi des sels d'aluminium, analogues lipidiques de monophosphoryle tels qu'un adjuvant lipidique de glucopyranosyle, monatide, inducteurs de cytokines, adjuvants à base de squalène, adjuvants lipophiles ou une combinaison de ceux-ci ; et
(f) Le cryoprotecteur est un/des sucre(s).

**8.** Composition immunogène selon la revendication 7 comprenant des stabilisants sélectionnés parmi un/des acide(s) aminé(s), sucre(s), polyol(s) ou une combinaison de ceux-ci, un/des tampon(s) ou sel(s).

**9.** Composition immunogène selon la revendication 8, dans laquelle

- Le tampon est sélectionné parmi un tampon phosphate, tampon histidine, tampon citrate, tampon succinate, tampon acétate, tampon arginine, solution saline à tampon phosphate, tampon trométhamine et des mélanges de ceux-ci ;
- L'acide aminé est sélectionné parmi arginine, glycine, lysine, histidine, acide glutamique, acide aspartique, isoleucine, leucine, alanine, phénylalanine, tyrosine, tryptophane, méthionine, sérine, proline, cystéine/cystine et une combinaison appropriée de ceux-ci ;
- Le sucre est sélectionné parmi glucose, fructose, galactose, mannose, sorbose, ribose, désoxyribose, saccharose, tréhalose, lactose, maltose, raffinose et des mélanges appropriés de ceux-ci, de préférence tréhalose ou raffinose ;
- Le polyol est sélectionné parmi le mannitol, sorbitol, dextrane, glycérol, arabitol, propylène glycol, polyéthylène glycol et des combinaisons appropriées de ceux-ci ;
- Le sel est sélectionné parmi le NaCl ou le KCl.

**10.** Combinaison de sauvetage comprenant les éléments suivants :

a. Un vecteur de transcription comprenant la séquence polynucléotique selon la revendication 1 ;
b. Un vecteur d'expression constitué d'un/de gène(s) isolé(s) codant une/des protéine(s) agissant en trans, où la protéine agissant en trans est sélectionnée parmi une protéine NP, une protéine P, une protéine L et une combinaison de celles-ci ;

c. De l’ARN polymérase pour la transcription appropriée de la séquence polynucléotidique selon la revendication 1.

**11.** Composition selon la revendication 10, dans laquelle le/les gène(s) codant la protéine agissant en trans est/sont sélectionné(s) parmi les SEQ ID NO : 9 à 11.

**12.** Composition selon la revendication 10 ou 11, dans laquelle la séquence d’acides aminés des protéines agissant en trans est sélectionnée parmi les SEQ ID NO : 12 à 14.

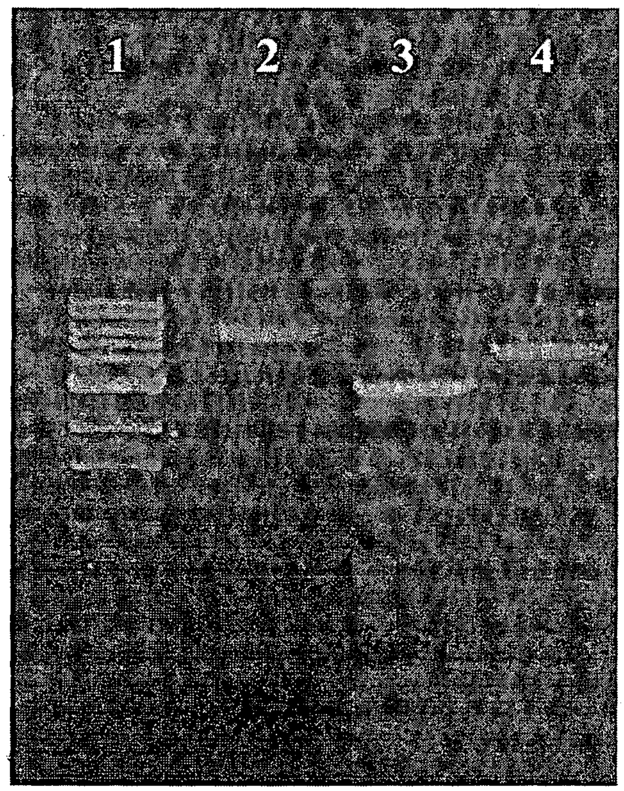


1. 1 kb ladder (λ/**HindIII**)
2. Fragment 1+2
3. Fragment 3+4
4. Fragment 5+6

**Figure 1**

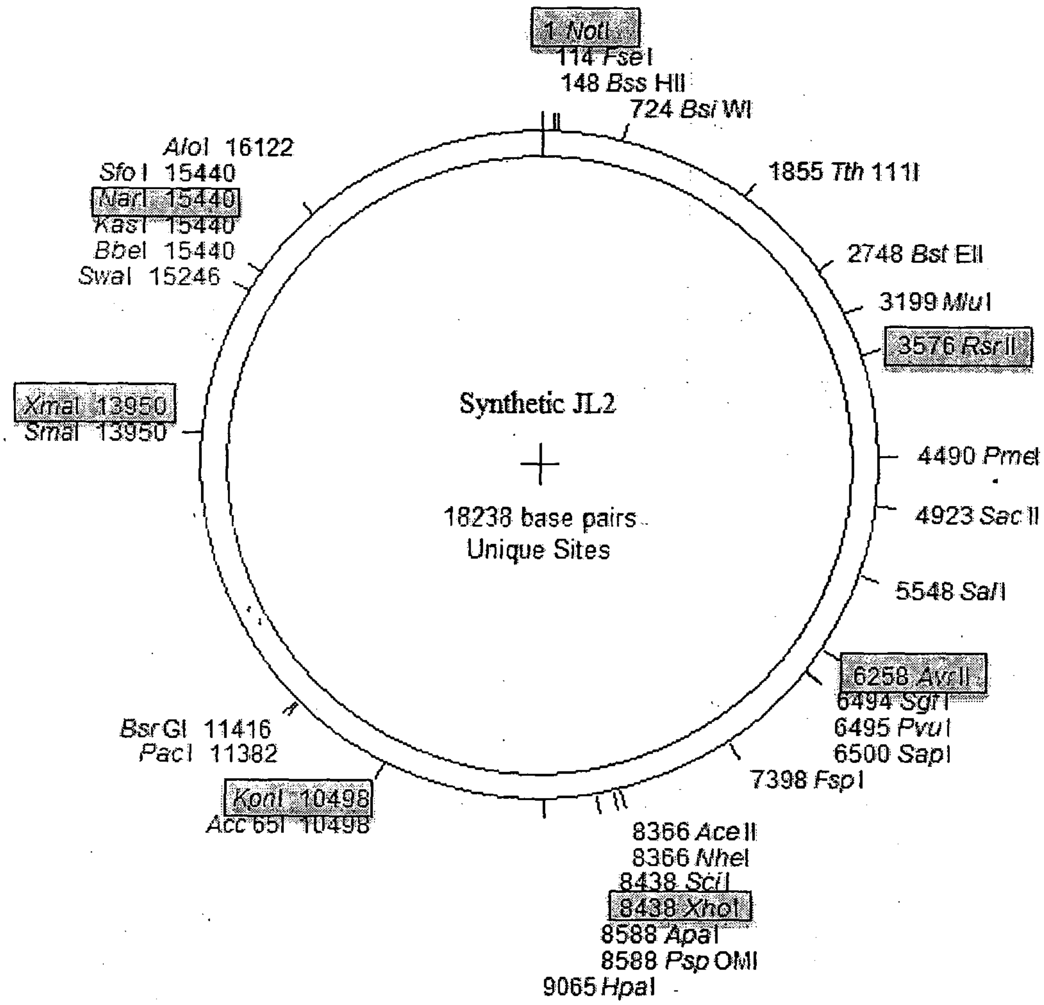
1 NotI
114 FseI
148 BssHII
724 BsiWI
1855 Tth111I
2748 BstEII
3199 MluI
3576 RsrII
4490 PmeI
4923 SacII
5548 SalI
6258 AvrII
6494 SgfI
6495 PvuI
6500 SapI
7398 FspI
8366 AceII
8366 NheI
8438 SciI
8438 XhoI
8588 ApaI
8588 PspOMI
9065 HpaI
KpnI 10498
Acc65I 10498
BsrGI 11416
PacI 11382
XmaI 13950
SmaI 13950
AloI 16122
SfoI 15440
NarI 15440
KasI 15440
BbeI 15440
SwaI 15246
Synthetic JL2
18238 base pairs
Unique Sites

**Figure 2**

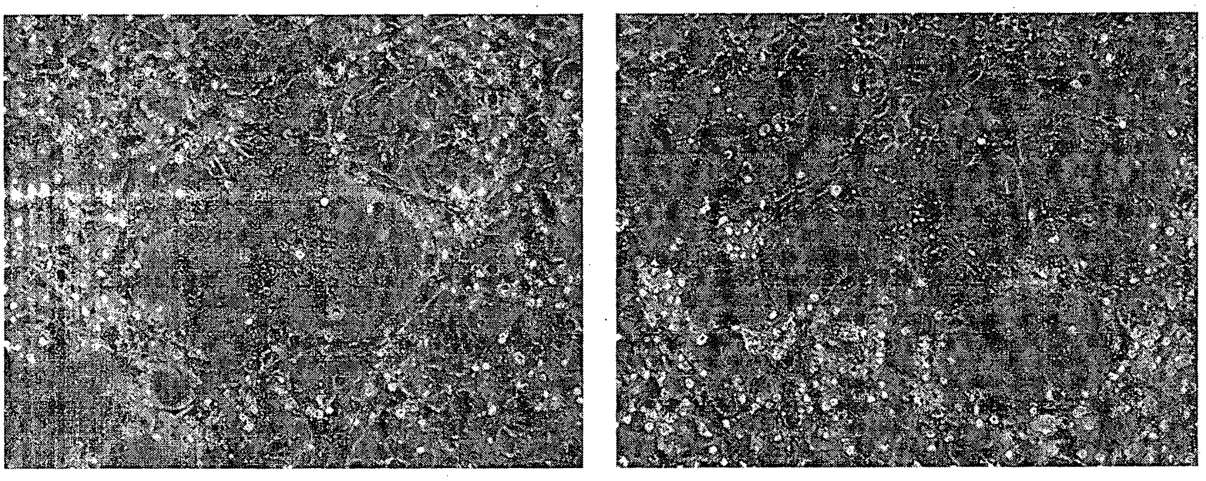

Figure 3

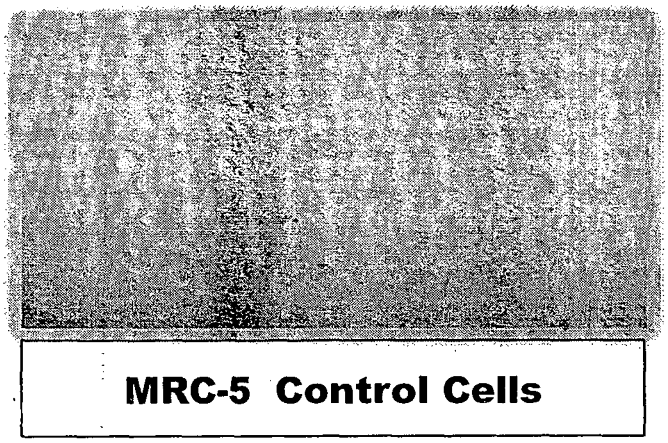

**MRC-5 Control Cells**

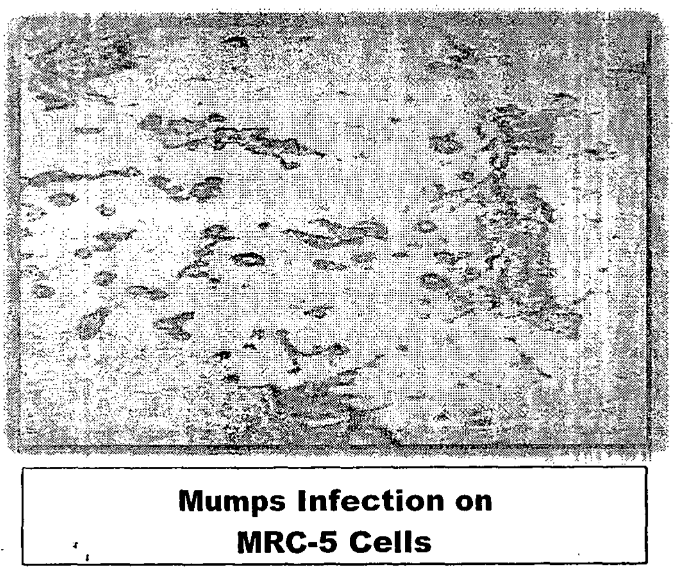

**Mumps Infection on MRC-5 Cells**

Figure 4

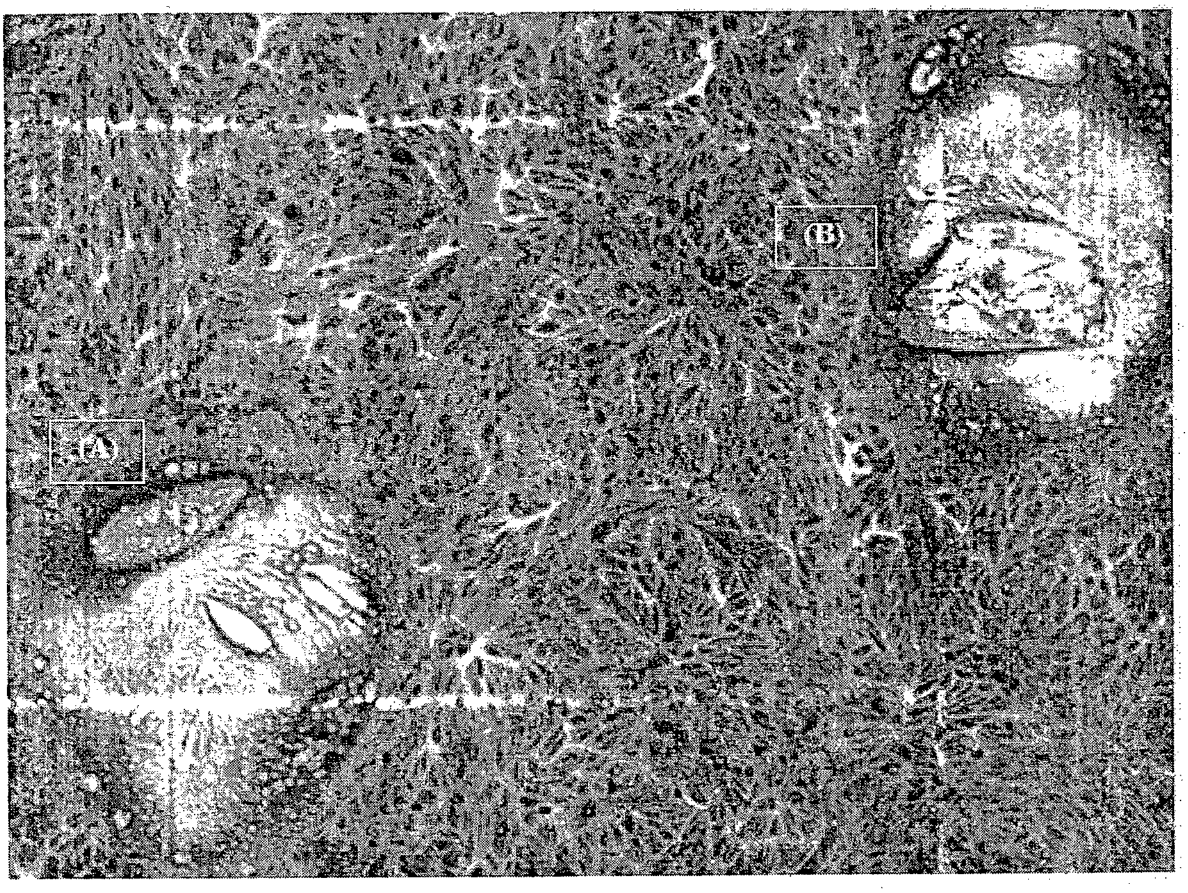


Figure 5

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- *Vaccine,* 2010, vol. 28, 1887-1892

SEQUENCE LISTING

<110> CADILA HEALTHCARE LIMITED

<120> Recombinant mumps virus Jeryl Lynn 2 based vaccine

<130> CHL-PCT0729

<140> 1055/MUM/2015
<141> 2016-03-23

<150> 1055/MUM/2015
<151> 2015-03-27

<160> 15

<170> PatentIn version 3.5

<210> 1
<211> 3595
<212> DNA
<213> Mumps Virus

<400> 1

```
atccccggcg gccgcttgta atacgactca ctataaccaa ggggagaaag aagatgggat      60
atcggtagaa caaatagtgt aagaaacagt aagcccggaa gtggtgtttc gcgatttcga     120
ggccggcctc gatcctcacc ttccattgtc actagcgcgc attttgacac tacctggaaa     180
atgtcgtctg tgctcaaagc attcgagcgg ttcacgatag aacaggaact tcaagacagg     240
ggtgaggagg gttcaattcc gccggagact ttaaagtcag cagtcaaagt cttcgttatt     300
aacacaccca atcccaccac acgctatcag atgctaaact tttgcctaag aataatctgc     360
agtcaaaatg ctagggcatc tcacagggta ggtgcattga taacattatt ctcacttccc     420
tcagcaggca tgcaaaatca tattagatta gcagatagat cacctgaagc tcagatagaa     480
cgctgcgaga ttgatggttt tgaacctggt acatataggc tgattccaaa tgcacgcgcc     540
aatcttactg ccaatgaaat tgctgcctat gctttgcttg cagatgacct ccctccaacc     600
ataaataatg gaactcctta cgtacatgca gatgttgaag gacagccatg tgatgagatt     660
gagcaattcc tggatcggtg ttacagtgta ctaatccagg cttgggtaat ggtctgtaaa     720
tgtatgacag cgtacgacca acctgctgga tctgctgatc ggcgatttgc gaaataccag     780
cagcaaggtc gccttgaagc aagatacatg ctgcagccgg aggcccaaag gttgattcaa     840
actgccatca ggaaaagtct tgttgttaga cagtacctta ccttcgaact ccagttggcg     900
agacggcagg ggttgctatc aaacagatac tatgcaatgg tgggtgacat cgggaagtac     960
attgagaatt caggacttac tgccttcttt ctcactctca aatatgcact agggaccaaa    1020
tggagtcctc tatcattggc tgcattcacc ggtgaactca ctaaactccg atccttgatg    1080
atgttatatc gagatctcgg agaacaagcc agataccttg ctctgttaga ggctccccaa    1140
ataatggact ttgcacccgg aggctaccca ttaatattca gttatgctat gggagtcggt    1200
```

acagtcctgg atgtccaaat gcgaaattac acttatgcaa gacctttcct aaacggttat 1260
tatttccaga ttggggttga gaccgcacga aggcaacaag gcactgttga caacagagta 1320
gcagatgatc tgggcctgac tcctgagcaa agaactgagg ttactcagct tgttgacagg 1380
cttgcaaggg gaagaggtgc tgggatacca ggtgggcctg tgaatccttt tgttcctcca 1440
gttcaacagc aacaacctgc tgccgtatat gaggacattc ctgcattaga ggaatcagat 1500
gacgatggtg atgaagatag aggcgcagga ttccaaaatg gagtacaagt accagctgta 1560
agacagggag gtcaaactga ctttagagca cagcctttac aagatccaat tcaagcacag 1620
cttttcatgc cattatatcc tcaagtcagc aacatcccaa ataatcagaa tcatcagatc 1680
aatcgcatcg gggggctgga aaaccaagat ttattacgat acaacgagaa tggtgattct 1740
caacaagatg caaggggcga acacggaaac actttcccaa acaatcccaa tcaaaacgca 1800
cagctgcaag tgggtgactg ggatgagtaa atcactgata tgatcaaact aaccccaatt 1860
ggactaagtc taggacaatc tagccatagc gaactgccca aattcactac attctattca 1920
taactagtct ttaagaaaaa attaggcccg gaaagaatta ggtccacgat cacaggcaca 1980
atcattctga tcgtgtttct ttccgggtaa gccatggatc aatttataaa acaggatgag 2040
actggtgatt taattgagac aggaatgaat gttgcaaacc atttcctatc cgcccccatt 2100
cagggaacca actcgctgag caaggcttca atcatccctg gcgttgcacc tgtactcatt 2160
ggcaatccag agcaaaagaa cattcagcac cctaccgcat cacatcaggg atccaagtca 2220
aagggcagag gctcaggggt caggtccatc atagtcccgc cctccgaagc aggcaatgga 2280
gggactcaga ttcctgagcc cctttttgca caaacaggac agggtggtat agtcaccaca 2340
gtctatcagg atccaactat ccaaccaaca ggttcatacc gaagtgtgga attggcgaag 2400
atcggaaaag agagaatgat taatcgattt gttgagaaac ctagaacctc aacgccggtg 2460
acagaattta agagggggc cgggagcggc tgctcaaggc cagacaatcc aagaggaggg 2520
catagacggg aatggagcct cagctgggtc caaggagagg tccgggtctt tgagtggtgc 2580
aaccctatat gctcacctat cactgccgca gcaagattcc actcctgcaa atgtgggaat 2640
tgccccgcaa agtgcgacca gtgcgaacga gattatggac ctccttaggg ggatggatgc 2700
tcgcctgcaa catcttgagc aaaaggtgga caaggtgctt gcacagggca gcatggtgac 2760
ccaaataaag aatgaattat caacagtaaa gacaacatta gcaacaattg aagggatgat 2820
ggcaacagta aaaatcatgg atcctggaaa tccgacaggg gtcccagttg atgagcttag 2880
aagaagtttt agtgatcatg tgacaattgt tagtggacca ggagatgtgt cgttcagctc 2940
ccgtgaagaa cccacactgt atttggatga gctggcgagg cccgtctcca agcctcgtcc 3000
tgcaaagcag acaaaacccc aaccagtaaa ggatttggca ggacgaaaag tgatgatcac 3060

```
caaaatgatt actgattgtg tggctaaccc tcaaatgaag caggcgttcg agcaacgatt      3120
ggcaaaggcc agcaccgagg atgctctgaa cgacatcaag aaagatatca tacggagcgc      3180
catatgaatt caccagaagc accagacgcg tggaaaaatc catgaactga gagccacaat      3240
gattccctat taaataaaaa ataagcacga acacaagtcg aatccaacca tagcagagat      3300
ggcaggatca cagatcaaaa ttcctcttcc aaagcccccc gattcagact ctcaaagact      3360
aaatgcattc cctgttatca tggctcaaga aggcaaagga cgacttctta gacaaatcag      3420
gcttaggaaa atattatcag gagatccgtc tgatcagcaa atcacatttg tgaatacata      3480
tggattcatc catgccactc cagaaacatc cgagttcatc tctgaatcat cacaacaaaa      3540
ggtaactcct gcagtgacag cgtgcatgct gtcctttggt gccggaccgg tgcta           3595
```

<210> 2
<211> 2695
<212> DNA
<213> Mumps Virus

<400> 2

```
tggtgccgga ccggtgctag aagatccaca acatatgctg aaggctcttg atcagacaga        60
cattagggtt cggaaaacag caagtgataa agagcagatc ctattcgaga tcaaccgcat       120
ccccgatcta ttcaggcatc atcaaatatc tgcggaccat ctgattcagg ccagctccga       180
taaatatgtc aagtcaccag cgaaattgat tgcaggagta aattacatct actgtgtcac       240
attcttatct gtgacagttt gttctgcctc actcaagttt cgagttgcac gcccattgct       300
tgctgcacgg tctagattag taagagcagt tcagatggaa gttttgcttc gggtaacttg       360
caaaaaagat tctcaaatgg caaagagcat gttaaatgac cctgatggag aagggtgcat       420
tgcatcagtg tggttccacc tatgtaatct gtgcaaaggc aggaataaac ttaggagtta       480
cgatgaaaat tattttgctt ctaagtgccg taagatgaat ctgacagtca gcataggaga       540
tatgtgggga ccaaccattc tagtccatgc aggcggtcac attccgacaa ctgcaaaacc       600
tttttttcaac tcaagaggct gggtctgcca cccaatccac caatcatcac catcgttggc      660
gaagacccta tggtcatctg ggtgtgaaat caaggctgcc agtgctatcc tccagggttc       720
agactatgca tcacttgcaa agactgatga cataatatat ccgaagataa aagtcgataa       780
agacgcggcc aactacaaag gagtatcctg gagtccattc aggaagtctg cctcaatgag       840
taacctatga gaatttcctc tatttcaact gatgcctata agagaatcaa caatcaagca       900
aatttgaccg gtggtaattc gtttaaactt atagaaaaaa taagcctaga aggatatcct       960
acttctcaac cttccaactt tgaaaataga atagatcagt aatcatgaag gcttatccag      1020
ttatttgctt gggctttgca atcttttcat cctctatatg tgtgaatatc aatatcttgc      1080
agcaaattgg atacatcaag caacaggtca ggcaactaag ctattactca caaagttcaa      1140
```

```
gctcctacgt agtggtcaag cttttaccga atatccaacc cactgataac agctgtgaat      1200
ttaagagtgt aattcaatac aataagacct tgagtaattt gcttcttcca attgcagaaa      1260
acataaacaa tattgcatcg ccctcacctg ggtcaagacg tcataaacgg tttgctggca      1320
ttgccattgg cattgctgcg ctcggtgttg cgaccgcggc acaagtgact gccgctgtct      1380
cattagttca agcacagaca aatgcacgtg caatagcagc gatgaaaaat tcaatacagg      1440
caactaatcg ggcagtcttc gaagtgaagg aaggcaccca gcagttagct atagcggtac      1500
aagcaataca agaccatatc aatactatta tgaacaccca attgaacaat atgtcttgtc      1560
agatccttga taaccagctt gcaacctctc taggattata cctaacagaa ttaacaacag      1620
tgttccagcc acaattgatt aatccagcat tgtcaccgat tagtatccaa gccttgaggt      1680
ctttgcttgg aagtatgaca cctgcagtgg ttcaagcaac attatctact tcaatttctg      1740
ctgctgaaat actaagtgcc ggtctaatgg agggtcagat agtttctgtt ctgctagatg      1800
agatgcagat gatagttaag ataaacattc caaccattgt cacacaatca aatgcattgg      1860
tgattgactt ctactcaatt tcaagtttca ttaataatca agaatccata attcaattgc      1920
cagacaggat cttggagatc gggaatgaac aatggcgcta tccagctaag aattgtaagt      1980
cgacaagaca tcacatattc tgccaataca atgaggcaga gaggctgagc ctagaaacaa      2040
aactatgcct tgcaggcaat attagtgcct gtgtgttctc acctatagca gggagctata      2100
tgaggcgatt tgtagcgctg gatggaacaa ttgttgcaaa ctgtcgaagt ctaacgtgtc      2160
tatgcaagag tccatcttat cctatatacc aacctgacca tcatgcagtc acgaccattg      2220
atctaacgtc atgtcaaaca ttgtccctgg acggactgga tttcagcatt gtctcactaa      2280
gcaacatcac ttacgctgag aatcttacta tttcattgtc tcagacgatc aatactcaac      2340
ccattgatat atcaactgag ctgagtaagg ttaatgcatc cctccaaaat gccgttaaat      2400
acataaaaga gagtaaccat caactccaat ccgttagtgt aagttctaaa atcggagcta      2460
taattgtagc agccttagtt ttgagcatcc tgtcgattat catttcgcta ttgttttgct      2520
gctgggctta cattgcgact aaagaaatca gaagaatcaa cttcaaaaca aatcatatca      2580
acacaatatc aagtagtgtc gatgatctca tcaggtacta attttaaatt ggtgattcat      2640
cctgcaatta aaaaaggttt agaaaaaaac taaactaaga atgaatctcc taggg           2695
```

<210> 3
<211> 2188
<212> DNA
<213> Mumps Virus

<400> 3

tcctagggtc gtaacgtcac gtcaccctgc cgttgcacta tgccggcaat ccaacctccc 60

ttatacctaa catttctatt gctaaccctt ctctatctaa tcataactct gtatgtctgg 120

```
actatattga ccattaacca taatacggcg gttcggtatg cagcactgta ccagcgatcc      180
ttctctcgct ggggttttga tcaatcactc tagaaagatc ctcagttagg gcaagtggcg      240
atcgctcttc ctagaacaag ctgcatccaa atgaagctgc actaccatga gacataaaga      300
aaaaagcaag ccagaacaaa cttaggatca caacacaaca caaaatatta gctgctatca      360
caactgtgtt ccggccacta agaaaatgga gccctcgaaa ctattcataa tgtcggacaa      420
cgccaccttt gcacctggac ctgttgttaa tgcggctggt aagaagacat tccgaacctg      480
tttccgaata ttggtcctat ccgtacaagc agttaccctt atattggtta ttgtcacttt      540
aggtgagctt attaggatga tcaatgatca aggcttgagc aatcagttgt cttcaattac      600
agacaagata agagaatcag ctgctatgat tgcatctgct gtgggagtaa tgaatcaagt      660
aattcatgga gtaacggtat ccttacccct acaaattgag ggaaaccaaa atcaattatt      720
atccacactt gccacaatct gcacaaacag aaaccaagtt tcaaactgct ctacaaacat      780
ccccttagtt aatgacctta ggtttataaa tggaatcaat aagttcatca ttgaagatta      840
tgcaacccat gatttctcca tcggccatcc actcaacatg cctagcttta tcccaactgc      900
aacctcaccc aatggttgca caagaattcc atccttttct ttaggtaaga cacattggtg      960
ttacacacat aatgtaatta atgccaactg caaggatcgt acttcatcga accaatatgt     1020
ttccatgggg attctcgttc aaaccgcgtc agggtatccc atgttcaaaa ccctaaaaat     1080
ccaatatctc agtgatggcc tgaatcggaa aagctgctca attgcaacag tccctgatgg     1140
ttgcgcaatg tactgttacg tttcaactca acttgaaacc gacgactatg cggggtccag     1200
cccacctacc cagaaactta ccctgttgtt ctataatgac accatcaaag aaaggacaat     1260
atctccgtct ggtcttgaag gaaattgggc tactttggtg ccaggagtgg ggagtggaat     1320
atatttcgaa aataagttga tctttcctgc atatgggggt gtcttgccca atagtacact     1380
aggagttaaa tcagcaagag aatttttccg gcccgttaat ccatataatc catgttcagg     1440
accaccacaa gagttagatc agcgtgcttt gagatcatat ttcccaagtt acttctctag     1500
tcgaagggta cagagtgcat ttctggtctg tgcctggaat cagatcctag ttacaaattg     1560
cgagctagtt gtcccctcaa acaatcagac acttatgggt gcagaaggaa gagttttatt     1620
gatcaataat cggctattat attatcagag gagtactagc tggtggccgt atgaactcct     1680
ctatgagata tcattcacat ttacaaactc tggtcaatca tctgtgaata tgtcctggat     1740
acctatatat tcattcaccc gtcctggttt gggcaactgc cgtggtgaaa atatatgccc     1800
aacagtctgt gtatcaggag tttatcttga tccctggcca ttaactccat acagccatca     1860
atcaggcatt aacagaaatt tctatttcac aggtgcactg ctaaattcaa gcacaaccag     1920
ggtgaatcct accctttatg tctctgccct taataatctt aaagtactag ccccatatgg     1980
tactcaagga ttgtttgcgt catacaccac aaccacctgc tttcaagata ccggtgacgc     2040
```

```
tagtgtgtat tgtgtctata ttatggaact agcatcgaat attgttggag aattccaaat     2100

tctacctgtg ctagccagat tgaccatcac ttgagttgta gtgaatgtag taggaagctt     2160

tatgggcgtg tctcatttct tctcgagt                                        2188
```

<210> 4
<211> 2069
<212> DNA
<213> Mumps Virus

<400> 4

tctcgagtat taagaaaaaa caggccagaa tggcgggcct aaatgagata ctcctacccg 60
aagtacattt aaactccccc atcgttagat ataagctttt ctactatata ttgcatggcc 120
agttaccaaa tgatttggag ccagatgact tgggcccatt agcaaatcat aattggaagg 180
caattcgagc tgaggaatcc caggttcatg cacgattaaa acagatcaga gtagaactca 240
ttgcaaggat tcctagtctc cggtggaccc gctctcaaag agagattgcc atactcattt 300
ggccaagaat acttccaata ctgcaagcat atgatcttcg gcaaagtatg caattgccca 360
cagtgtggga gaaattgact caatccacgg ttaatcttat aagtgatggt ctagaacggg 420
ttgtattaca catcagcaat caattaacag gcaagcctaa cttgtttacc aggtctagag 480
ctggacaaga cacaaaagat tactcaattc catccactag agagctatct caaatatggt 540
ttaacaatga gtggagtggg tctgtgaaga cctggcttat gattaaatat agaatgaggc 600
agctaatcac aaatcaaaag acaggtgagt taacagattt agtaaccatt gtggatacta 660
ggtccactct atgcattatt accccagaat tagtcgcttt atactccaat gagcacaaag 720
cattaacgta cctcaccttt gaaatggtat taatggtcac tgatatgtta gagggaagac 780
tgaatgtttc ttctctgtgc acagctagtc attatctgtc ccctttaaag aagcgaatcg 840
aagttctcct gacattagtt gatgaccttg ctctactcat gggggataaa gtatacggta 900
ttgtctcttc acttgagagt tttgtttacg cccaattaca gtatggtgat cctgttatag 960
acattaaagg tacattctat ggatttatat gtaatgagat tctcgaccta ctgactgaag 1020
gcaacatctt tactgaagaa gaggcaaaca aggttcttct ggacttgacg tcacagtttg 1080
acaatctatc ccctgattta acagctgagc tcctctgcat tatgagactt tggggccatc 1140
ccaccttaac tgccagccaa gcagcatcca aggtccgaga gtccatgtgc gctcctaagg 1200
tgttagattt ccaaacaata atgaaaaccc tggctttctt tcacgcaatc ctaattaacg 1260
gttataggag gagccataat ggaatctggc cgcctactac tcttcatggc aatgccccca 1320
aaagcctcat tgagatgcgg catgataatt cagagcttaa gtatgagtat gtcctcaaga 1380
attggaaaag tatatctatg ttaagaatac acaaatgctt tgatgcatca cctgatgaag 1440
atctcagcat attcatgaag gataaggcaa taagctgtcc aaagcaagac tggatgggag 1500

```
tatttaggag gagcctgatt aaacagcgct atcgtgacgt gaatcggcct ctaccacaac     1560
catttaaccg gagactgctg ttgaatttcc tagaggatga ccgattcgat cctattaaag     1620
agcttgagta tgtcaccagt ggagaatatc ttagggaccc tgaattttgt gcatcttact     1680
ctctcaaaga gaaagagata aaggctacag gtcgtatatt tgcaaaaatg acaaagagaa     1740
tgagatcgtg ccaagtaatt gcagaatcat tgttagccaa tcacgcaggt aaattaatga     1800
gagagaatgg agttgtctta gaccagttga aattaacaaa atctttatta actatgaacc     1860
aaattggcat tatatcagag cacagccgaa gatccactgc cgacaacatg accttggcac     1920
actccggttc aaataagcac aggattaaca atagtcaatt caagaagaat aaagacaaca     1980
aacatgagat gcctgatgat gggtttgaga tagcagcctg cttcctaaca accgacctca     2040
caaaatactg cttaaattgg aggtaccaa                                       2069
```

<210> 5
<211> 3462
<212> DNA
<213> Mumps Virus

<400> 5

```
gaggtaccaa gtcatcatcc cctttgcacg tacattgaat tcaatgtacg gtatacccca      60
cctgtttgaa tggatacatt taaggctgat gcgaagcact ctctatgtcg gtgatccctt     120
caatcctcca tcagatccta cccaacttga ccttgatacc gcactcaacg atgatatatt     180
tatagtttcc cctcgtggcg gaatcgaggg tttatgtcaa aaattatgga ctatgatttc     240
catctcaaca atcatattat ccgcaactga ggcaaacact agagtaatga gcatggttca     300
gggcgataac caagcaattg caatcaccac tagagtagtg cgctcgctca gtcattccga     360
gaagaaagag caagcttata aagcaagtaa attattcttt gaaagactta gagctaacaa     420
ccatggaatt ggacaccact taaaagaaca agaaacaatc cttagttctg atttcttcat     480
atacagtaag agggtgtttt acaaaggtcg aatcttgact caagcgttaa agaacgtgag     540
caagatgtgc ttaacagctg atatactggg ggattgttca caagcatcat gttccaattt     600
agctaccact gtaatgcgtc ttactgagaa tggggtcgag aaagatttgt gttatttcct     660
aaatgcattc atgacaatta gacaattatg ttatgatcta gtatttcccc aaactaaatc     720
tcttagtcag gacattacta atgcttatct taatcatcca atacttatct caagattgtg     780
tctattacca tctcaattgg ggggcttaaa ctttctttca tgtagccgcc tgtttaatag     840
aaacatagga gatccactag tgtctgcaat tgctgatgtg aaacgattaa ttaaagcggg     900
ctgtctagat atctgggtcc tgtacaacat ccttggaagg aggcctggaa agggcaagtg     960
gagcactctg gcagctgatc cctatacttt aaacatagac tatttagtcc cttcaacaac    1020
ttttttaaag aaacatgccc aatatacact gatggaacgg agtgttaatc ccatgctccg    1080
```

tggagtattt agcgaaaatg cagctgagga ggaagaggaa ctcgcacagt atctactaga 1140
tcgcgaagta gtcatgccca gggttgcaca tgttatactt gcccagtcta gttgcggtag 1200
aagaaaacag atccaaggtt acttggattc tactagaact attatcaggt attcactgga 1260
ggtgagacca ctgtcagcaa agaagctgaa tacggtaata gaatacaact tattgtatct 1320
gtcctacaat ttggagatta tcgaaaaacc caatatagtc caaccttttt tgaatgcaat 1380
caatgttgat acttgtagca tcgatatagc taggtccctt agaaaactat cctgggcaac 1440
tttacttaat ggacgtccca tcgagggatt agaaacacct gatcctattg aattggtaca 1500
tgggtgttta ataatcgggt cagatgagtg tgagcattgc agtagtggtg atgacaaatt 1560
cacctggttt ttcctcccca aggggataag gttagatgat gatccggcat ctaacccacc 1620
catcagagta ccttatatcg gatctaaaac agatgaacga agggttgcat caatggctta 1680
tatcaaaggg gcatcagtat cacttaaatc agcactcagg ttagcggggg tatatatctg 1740
ggctttcgga gatacagagg aatcatggca ggatgcctat gagttagctt ccactcgtgt 1800
taatctcaca ctagagcaat tgcaatcgct tactccttta ccaacatctg ccaacctagt 1860
ccacagattg gatgatggca ctactcaatt aaaatttacc cctgcaagct cctatgcatt 1920
ctctagcttt gttcatatat ctaacgactg tcaaattctt gagatcgatg atcaggtaac 1980
ggattctaac ctgatttacc agcaagttat gattactggc cttgctttaa ttgagacatg 2040
gaataatcct ccaatcaact tctccgttta tgaaactaca ttacacttgc atacaggctc 2100
atcttgctgt ataaggcctg tcgagtcttg tgtagtaaat ccgcctttac ttcctgtccc 2160
tttcattaat gttcctcaaa tgaataaatt tgtatatgac cctgaaccac ttagtttgct 2220
agaaatggaa aaaattgagg atattgctta tcaaaccaga attggtggtt tagatcaaat 2280
cccgcttctg gaaaaaatac ccttactagc tcaccttacc gccaaacaga tggtgaatag 2340
catcactggg cttgatgaag caacatctat aatgaatgat gctgtagttc aagcagacta 2400
tactagcaat tggattggtg aatgctgcta cacttacatt gactctgtgt ttgtttactc 2460
tggctgggca ttgttattgg aactttcata ccaaatgtat tacctaagaa ttcgaggcat 2520
acaaggaatt ctagactatg tgtatatgac cttgaggagg ataccaggaa tggccataac 2580
aggcatctca tccacaatta gtcaccctcg tatactcaga agatgcatca atttggatgt 2640
catagcccca atcaattctc cacacatagc ttcactggat tacacaaaat tgagcataga 2700
tgcagtaatg tggggaacca agcaggtgtt gaccaacatt tctcaaggta tcgattatga 2760
gatagttgtt ccttctgaaa gccaacttac actcagtgat agagtcctaa atctagttgc 2820
tcgaaaacta tcactactgg caatcatctg ggccaattac aactatcctc cgaaggttaa 2880
aggtatgtca cctgaggaca aatgtcaggc tttaactaca catctactcc aaactgtcga 2940

```
atatgttgag tacattcaga ttgaaaagac aaacatcagg aggatgatta ttgaaccaaa      3000
attaactgcc taccctagta atttgtttta tctctctcga aagctgctta atgctattcg      3060
agactctgaa gaaggacaat tcctgattgc atcctattat aacagttttg gatatctgga      3120
accgatatta atggaatcta aagtattcaa tctaagttca tccgaatcag catctcttac      3180
agaattcgat ttcatcctca acttggaatt gtccgacgcc agacttgaga aatactctct      3240
cccaagtttg cttatgacgg ctgagaatat ggataaccca tttcctcaac ccccacttca      3300
tcacgttctc agaccactag gtttgtcatc cacctcatgg tataaaacaa tcagtgtttt      3360
gaattatatt agccatatga agatatctga cggtgcccat ctatacttgg cagagggaag      3420
tggagcctct atgtcactta tagagacttt cttgcccggg ga                         3462
```

<210> 6
<211> 1504
<212> **DNA**
<213> Mumps Virus

<400> 6

tgcccgggga aaccatatgg tacaacagcc tgttcaatag tggtgagaat ccccctcaac 60
gtaatttcgc ccctttgccc acccagttta ttgaaagtgt cccctataga ttaattcaag 120
caggtatagc agcaggaaat ggtatagtgc aaagtttcta tccactctgg aacggaaaca 180
gcgatataac tgacttaagc actaaaacta gtgttgaata cattatccac aaggtaggag 240
ctgatacttg tgcattagtt catgtggatt tggaaggtgt ccctggctca atgaacagca 300
tgttggagag agctcaagta cacgcactac taatcacagt cactgtactg aaaccaggcg 360
gcttactaat cttgaaagct tcatgggaac cctttaatcg attttccttt ttactcacag 420
tactctggca attcttttcc acaataagga tcttgcgatc ttcatactcc gacccgaata 480
atcacgaggt ttacataata gccacattgg cagttgatcc cactacatcc tcctttacaa 540
ctgctctgaa tagggcacgc accctgaatg aacagggctt ttcactcatc ccacctgaat 600
tagtaagtga gtactggagg aagcgtgttg aacaaggaca gattatacag gactgtatag 660
ataaagttat atcagagtgt gtcagagatc aatatctggc agacaacaac attatcctcc 720
aggcgggagg tactccaagc acaagaaaat ggttggatct gcctgactat tcttcgttca 780
atgaactaca atctgaaatg gccagactca taacaattca tcttaaagag gtaatagaaa 840
tcctaaaggg ccaagcatca gatcatgaca ccctattatt tacttcatac aatgtaggtc 900
ccctcggaaa aataaataca atacttagat tgattgttga gagaattctt atgtatactg 960
tgaggaactg gtgtatcttg cctacccaaa ctcgtctcac cttacgacaa tctatcgagc 1020
ttggagagtt tagactaaga gatgtgataa cacccatgga gattctaaaa ctatccccca 1080
acaggaaata tctaaagtct gcattaaatc aatcgacatt caaccatcta atgggagaaa 1140
catctgacat attgttaaac cgagcttatc agaagagaat ttggaaagcc attgggtgtg 1200
taatctattg ctttggtttg ctcaccccgg atgttgaaga ttctgagcgc attgatattg 1260
acaatgacat acccgattat gatattcacg gggacataat ttaaatcgaa taaagactct 1320
tctggcatta cacaccacca agaagtgcca aaccagcacc caaactcttc taaaccgccc 1380
acgacctcga acaatcacaa ccacatcagt attaaataca gaagatcctt ttaagaaaaa 1440
attgattcta ctttctcccc ttggtggccg gcatggtccc agcctcctcg ctggcgccgg 1500
ctgg 1504

<210> 7
<211> 18238
<212> DNA
<213> Mumps Virus

<400> 7

```
gcggccgctt gtaatacgac tcactataac caaggggaga aagaagatgg gatatcggta      60
gaacaaatag tgtaagaaac agtaagcccg gaagtggtgt ttcgcgattt cgaggccggc     120
ctcgatcctc accttccatt gtcactagcg cgcattttga cactacctgg aaaatgtcgt     180
ctgtgctcaa agcattcgag cggttcacga tagaacagga acttcaagac aggggtgagg     240
agggttcaat tccgccggag actttaaagt cagcagtcaa agtcttcgtt attaacacac     300
ccaatcccac cacacgctat cagatgctaa acttttgcct aagaataatc tgcagtcaaa     360
atgctagggc atctcacagg gtaggtgcat tgataacatt attctcactt ccctcagcag     420
gcatgcaaaa tcatattaga ttagcagata gatcacctga agctcagata gaacgctgcg     480
agattgatgg ttttgaacct ggtacatata ggctgattcc aaatgcacgc gccaatctta     540
ctgccaatga aattgctgcc tatgctttgc ttgcagatga cctccctcca accataaata     600
atggaactcc ttacgtacat gcagatgttg aaggacagcc atgtgatgag attgagcaat     660
tcctggatcg gtgttacagt gtactaatcc aggcttgggt aatggtctgt aaatgtatga     720
cagcgtacga ccaacctgct ggatctgctg atcggcgatt tgcgaaatac cagcagcaag     780
gtcgccttga agcaagatac atgctgcagc cggaggccca aaggttgatt caaactgcca     840
tcaggaaaag tcttgttgtt agacagtacc ttaccttcga actccagttg gcgagacggc     900
aggggttgct atcaaacaga tactatgcaa tggtgggtga catcgggaag tacattgaga     960
attcaggact tactgccttc tttctcactc tcaaatatgc actagggacc aaatggagtc    1020
ctctatcatt ggctgcattc accggtgaac tcactaaact ccgatccttg atgatgttat    1080
atcgagatct cggagaacaa gccagatacc ttgctctgtt agaggctccc caaataatgg    1140
actttgcacc cggaggctac ccattaatat tcagttatgc tatgggagtc ggtacagtcc    1200
tggatgtcca aatgcgaaat tacacttatg caagaccttt cctaaacggt tattatttcc    1260
```

agattggggt tgagaccgca cgaaggcaac aaggcactgt tgacaacaga gtagcagatg 1320
atctgggcct gactcctgag caaagaactg aggttactca gcttgttgac aggcttgcaa 1380
ggggaagagg tgctgggata ccaggtgggc ctgtgaatcc ttttgttcct ccagttcaac 1440
agcaacaacc tgctgccgta tatgaggaca ttcctgcatt agaggaatca gatgacgatg 1500
gtgatgaaga tagaggcgca ggattccaaa atggagtaca agtaccagct gtaagacagg 1560
gaggtcaaac tgactttaga gcacagcctt tacaagatcc aattcaagca cagcttttca 1620
tgccattata tcctcaagtc agcaacatcc caaataatca gaatcatcag atcaatcgca 1680
tcgggggct ggaaaaccaa gatttattac gatacaacga gaatggtgat tctcaacaag 1740
atgcaagggg cgaacacgga aacactttcc caaacaatcc caatcaaaac gcacagctgc 1800
aagtgggtga ctgggatgag taaatcactg atatgatcaa actaacccca attggactaa 1860
gtctaggaca atctagccat agcgaactgc ccaaattcac tacattctat tcataactag 1920
tctttaagaa aaaattaggc ccggaaagaa ttaggtccac gatcacaggc acaatcattc 1980
tgatcgtgtt tctttccggg taagccatgg atcaatttat aaaacaggat gagactggtg 2040
atttaattga gacaggaatg aatgttgcaa accatttcct atccgccccc attcagggaa 2100
ccaactcgct gagcaaggct tcaatcatcc ctggcgttgc acctgtactc attggcaatc 2160
cagagcaaaa gaacattcag caccctaccg catcacatca gggatccaag tcaaagggca 2220
gaggctcagg ggtcaggtcc atcatagtcc cgccctccga agcaggcaat ggagggactc 2280
agattcctga gcccctttt gcacaaacag gacagggtgg tatagtcacc acagtctatc 2340
aggatccaac tatccaacca acaggttcat accgaagtgt ggaattggcg aagatcggaa 2400
aagagagaat gattaatcga tttgttgaga aacctagaac ctcaacgccg gtgacagaat 2460
ttaagagggg ggccgggagc ggctgctcaa ggccagacaa tccaagagga gggcatagac 2520
gggaatggag cctcagctgg gtccaaggag aggtccgggt ctttgagtgg tgcaacccta 2580
tatgctcacc tatcactgcc gcagcaagat tccactcctg caaatgtggg aattgccccg 2640
caaagtgcga ccagtgcgaa cgagattatg gacctcctta gggggatgga tgctcgcctg 2700
caacatcttg agcaaaaggt ggacaaggtg cttgcacagg gcagcatggt gacccaaata 2760
aagaatgaat tatcaacagt aaagacaaca ttagcaacaa ttgaagggat gatggcaaca 2820
gtaaaaatca tggatcctgg aaatccgaca ggggtcccag ttgatgagct tagaagaagt 2880
tttagtgatc atgtgacaat tgttagtgga ccaggagatg tgtcgttcag ctcccgtgaa 2940
gaacccacac tgtatttgga tgagctggcg aggcccgtct ccaagcctcg tcctgcaaag 3000
cagacaaaac cccaaccagt aaaggatttg gcaggacgaa aagtgatgat caccaaaatg 3060
attactgatt gtgtggctaa ccctcaaatg aagcaggcgt tcgagcaacg attggcaaag 3120
gccagcaccg aggatgctct gaacgacatc aagaaagata tcatacggag cgccatatga 3180

attcaccaga agcaccagac gcgtggaaaa atccatgaac tgagagccac aatgattccc 3240

tattaaataa aaaataagca cgaacacaag tcgaatccaa ccatagcaga gatggcagga 3300

tcacagatca aaattcctct tccaaagccc cccgattcag actctcaaag actaaatgca 3360

ttccctgtta tcatggctca agaaggcaaa ggacgacttc ttagacaaat caggcttagg 3420

aaaatattat caggagatcc gtctgatcag caaatcacat ttgtgaatac atatggattc 3480

atccatgcca ctccagaaac atccgagttc atctctgaat catcacaaca aaaggtaact 3540

cctgcagtga cagcgtgcat gctgtccttt ggtgccggac cggtgctaga agatccacaa 3600

catatgctga aggctcttga tcagacagac attagggttc ggaaaacagc aagtgataaa 3660

gagcagatcc tattcgagat caaccgcatc cccgatctat tcaggcatca tcaaatatct 3720

gcggaccatc tgattcaggc cagctccgat aaatatgtca agtcaccagc gaaattgatt 3780

gcaggagtaa attacatcta ctgtgtcaca ttcttatctg tgacagtttg ttctgcctca 3840

ctcaagtttc gagttgcacg cccattgctt gctgcacggt ctagattagt aagagcagtt 3900

cagatggaag ttttgcttcg ggtaacttgc aaaaaagatt ctcaaatggc aaagagcatg 3960

ttaaatgacc ctgatggaga agggtgcatt gcatcagtgt ggttccacct atgtaatctg 4020

tgcaaaggca ggaataaact taggagttac gatgaaaatt attttgcttc taagtgccgt 4080

aagatgaatc tgacagtcag cataggagat atgtggggac caaccattct agtccatgca 4140

ggcggtcaca ttccgacaac tgcaaaacct tttttcaact caagaggctg ggtctgccac 4200

ccaatccacc aatcatcacc atcgttggcg aagaccctat ggtcatctgg gtgtgaaatc 4260

aaggctgcca gtgctatcct ccagggttca gactatgcat cacttgcaaa gactgatgac 4320

ataatatatc cgaagataaa agtcgataaa gacgcggcca actacaaagg agtatcctgg 4380

agtccattca ggaagtctgc ctcaatgagt aacctatgag aatttcctct atttcaactg 4440

atgcctataa gagaatcaac aatcaagcaa atttgaccgg tggtaattcg tttaaactta 4500

tagaaaaaat aagcctagaa ggatatccta cttctcaacc ttccaacttt gaaaatagaa 4560

tagatcagta atcatgaagg cttatccagt tatttgcttg ggctttgcaa tcttttcatc 4620

ctctatatgt gtgaatatca atatcttgca gcaaattgga tacatcaagc aacaggtcag 4680

gcaactaagc tattactcac aaagttcaag ctcctacgta gtggtcaagc ttttaccgaa 4740

tatccaaccc actgataaca gctgtgaatt taagagtgta attcaataca ataagacctt 4800

gagtaatttg cttcttccaa ttgcagaaaa cataaacaat attgcatcgc cctcacctgg 4860

gtcaagacgt cataaacggt ttgctggcat tgccattggc attgctgcgc tcggtgttgc 4920

gaccgcggca caagtgactg ccgctgtctc attagttcaa gcacagacaa atgcacgtgc 4980

aatagcagcg atgaaaaatt caatacaggc aactaatcgg gcagtcttcg aagtgaagga 5040

aggcacccag cagttagcta tagcggtaca agcaatacaa gaccatatca atactattat 5100

gaacacccaa ttgaacaata tgtcttgtca gatccttgat aaccagcttg caacctctct 5160

aggattatac ctaacagaat taacaacagt gttccagcca caattgatta atccagcatt 5220

gtcaccgatt agtatccaag ccttgaggtc tttgcttgga agtatgacac ctgcagtggt 5280

tcaagcaaca ttatctactt caatttctgc tgctgaaata ctaagtgccg gtctaatgga 5340

gggtcagata gtttctgttc tgctagatga gatgcagatg atagttaaga taaacattcc 5400

aaccattgtc acacaatcaa atgcattggt gattgacttc tactcaattt caagtttcat 5460

taataatcaa gaatccataa ttcaattgcc agacaggatc ttggagatcg ggaatgaaca 5520

atggcgctat ccagctaaga attgtaagtc gacaagacat cacatattct gccaatacaa 5580

tgaggcagag aggctgagcc tagaaacaaa actatgcctt gcaggcaata ttagtgcctg 5640

tgtgttctca cctatagcag ggagctatat gaggcgattt gtagcgctgg atggaacaat 5700

tgttgcaaac tgtcgaagtc taacgtgtct atgcaagagt ccatcttatc ctatatacca 5760

acctgaccat catgcagtca cgaccattga tctaacgtca tgtcaaacat tgtccctgga 5820

cggactggat ttcagcattg tctcactaag caacatcact tacgctgaga atcttactat 5880

ttcattgtct cagacgatca atactcaacc cattgatata tcaactgagc tgagtaaggt 5940

taatgcatcc ctccaaaatg ccgttaaata cataaaagag agtaaccatc aactccaatc 6000

cgttagtgta agttctaaaa tcggagctat aattgtagca gccttagttt tgagcatcct 6060

gtcgattatc atttcgctat tgttttgctg ctgggcttac attgcgacta aagaaatcag 6120

aagaatcaac ttcaaaacaa atcatatcaa cacaatatca agtagtgtcg atgatctcat 6180

caggtactaa ttttaaattg gtgattcatc ctgcaattaa aaaaggttta gaaaaaaact 6240

aaactaagaa tgaatctcct agggtcgtaa cgtcacgtca ccctgccgtt gcactatgcc 6300

ggcaatccaa cctcccttat acctaacatt tctattgcta acccttctct atctaatcat 6360

aactctgtat gtctggacta tattgaccat taaccataat acggcggttc ggtatgcagc 6420

actgtaccag cgatccttct ctcgctgggg ttttgatcaa tcactctaga aagatcctca 6480

gttagggcaa gtggcgatcg ctcttcctag aacaagctgc atccaaatga agctgcacta 6540

ccatgagaca taaagaaaaa agcaagccag aacaaactta ggatcacaac acaacacaaa 6600

atattagctg ctatcacaac tgtgttccgg ccactaagaa aatggagccc tcgaaactat 6660

tcataatgtc ggacaacgcc acctttgcac ctggacctgt tgttaatgcg gctggtaaga 6720

agacattccg aacctgtttc cgaatattgg tcctatccgt acaagcagtt acccttatat 6780

tggttattgt cactttaggt gagcttatta ggatgatcaa tgatcaaggc ttgagcaatc 6840

agttgtcttc aattacagac aagataagag aatcagctgc tatgattgca tctgctgtgg 6900

gagtaatgaa tcaagtaatt catggagtaa cggtatcctt accccctacaa attgagggaa 6960

accaaaatca attattatcc acacttgcca caatctgcac aaacagaaac caagtttcaa 7020
actgctctac aaacatcccc ttagttaatg accttaggtt tataaatgga atcaataagt 7080
tcatcattga agattatgca acccatgatt tctccatcgg ccatccactc aacatgccta 7140
gctttatccc aactgcaacc tcacccaatg gttgcacaag aattccatcc ttttctttag 7200
gtaagacaca ttggtgttac acacataatg taattaatgc caactgcaag gatcgtactt 7260
catcgaacca atatgtttcc atggggattc tcgttcaaac cgcgtcaggg tatcccatgt 7320
tcaaaaccct aaaaatccaa tatctcagtg atggcctgaa tcggaaaagc tgctcaattg 7380
caacagtccc tgatggttgc gcaatgtact gttacgtttc aactcaactt gaaaccgacg 7440
actatgcggg gtccagccca cctacccaga aacttaccct gttgttctat aatgacacca 7500
tcaaagaaag gacaatatct ccgtctggtc ttgaaggaaa ttgggctact ttggtgccag 7560
gagtggggag tggaatatat ttcgaaaata agttgatctt tcctgcatat gggggtgtct 7620
tgcccaatag tacactagga gttaaatcag caagagaatt tttccggccc gttaatccat 7680
ataatccatg ttcaggacca ccacaagagt tagatcagcg tgctttgaga tcatatttcc 7740
caagttactt ctctagtcga agggtacaga gtgcatttct ggtctgtgcc tggaatcaga 7800
tcctagttac aaattgcgag ctagttgtcc cctcaaacaa tcagacactt atgggtgcag 7860
aaggaagagt tttattgatc aataatcggc tattatatta tcagaggagt actagctggt 7920
ggccgtatga actcctctat gagatatcat tcacatttac aaactctggt caatcatctg 7980
tgaatatgtc ctggatacct atatattcat tcacccgtcc tggtttgggc aactgccgtg 8040
gtgaaaatat atgcccaaca gtctgtgtat caggagttta tcttgatccc tggccattaa 8100
ctccatacag ccatcaatca ggcattaaca gaaatttcta tttcacaggt gcactgctaa 8160
attcaagcac aaccagggtg aatcctaccc tttatgtctc tgcccttaat aatcttaaag 8220
tactagcccc atatggtact caaggattgt ttgcgtcata caccacaacc acctgctttc 8280
aagataccgg tgacgctagt gtgtattgtg tctatattat ggaactagca tcgaatattg 8340
ttggagaatt ccaaattcta cctgtgctag ccagattgac catcacttga gttgtagtga 8400
atgtagtagg aagctttatg ggcgtgtctc atttcttctc gagtattaag aaaaaacagg 8460
ccagaatggc gggcctaaat gagatactcc tacccgaagt acatttaaac tcccccatcg 8520
ttagatataa gcttttctac tatatattgc atggccagtt accaaatgat ttggagccag 8580
atgacttggg cccattagca aatcataatt ggaaggcaat tcgagctgag gaatcccagg 8640
ttcatgcacg attaaaacag atcagagtag aactcattgc aaggattcct agtctccggt 8700
ggacccgctc tcaaagagag attgccatac tcatttggcc aagaatactt ccaatactgc 8760
aagcatatga tcttcggcaa agtatgcaat tgcccacagt gtgggagaaa ttgactcaat 8820

ccacggttaa tcttataagt gatggtctag aacgggttgt attacacatc agcaatcaat 8880

taacaggcaa gcctaacttg tttaccaggt ctagagctgg acaagacaca aaagattact 8940

caattccatc cactagagag ctatctcaaa tatggtttaa caatgagtgg agtgggtctg 9000

tgaagacctg gcttatgatt aaatatagaa tgaggcagct aatcacaaat caaaagacag 9060

gtgagttaac agatttagta accattgtgg atactaggtc cactctatgc attattaccc 9120

cagaattagt cgctttatac tccaatgagc acaaagcatt aacgtacctc acctttgaaa 9180

tggtattaat ggtcactgat atgttagagg gaagactgaa tgtttcttct ctgtgcacag 9240

ctagtcatta tctgtcccct ttaaagaagc gaatcgaagt tctcctgaca ttagttgatg 9300

accttgctct actcatgggg gataaagtat acggtattgt ctcttcactt gagagttttg 9360

tttacgccca attacagtat ggtgatcctg ttatagacat taaaggtaca ttctatggat 9420

ttatatgtaa tgagattctc gacctactga ctgaaggcaa catctttact gaagaagagg 9480

caaacaaggt tcttctggac ttgacgtcac agtttgacaa tctatcccct gatttaacag 9540

ctgagctcct ctgcattatg agactttggg gccatcccac cttaactgcc agccaagcag 9600

catccaaggt ccgagagtcc atgtgcgctc ctaaggtgtt agatttccaa acaataatga 9660

aaaccctggc tttctttcac gcaatcctaa ttaacggtta taggaggagc cataatggaa 9720

tctggccgcc tactactctt catggcaatg cccccaaaag cctcattgag atgcggcatg 9780

ataattcaga gcttaagtat gagtatgtcc tcaagaattg gaaaagtata tctatgttaa 9840

gaatacacaa atgctttgat gcatcacctg atgaagatct cagcatattc atgaaggata 9900

aggcaataag ctgtccaaag caagactgga tgggagtatt taggaggagc ctgattaaac 9960

agcgctatcg tgacgtgaat cggcctctac cacaaccatt taaccggaga ctgctgttga 10020

atttcctaga ggatgaccga ttcgatccta ttaaagagct tgagtatgtc accagtggag 10080

aatatcttag ggaccctgaa ttttgtgcat cttactctct caaagagaaa gagataaagg 10140

ctacaggtcg tatatttgca aaaatgacaa agagaatgag atcgtgccaa gtaattgcag 10200

aatcattgtt agccaatcac gcaggtaaat taatgagaga gaatggagtt gtcttagacc 10260

agttgaaatt aacaaaatct ttattaacta tgaaccaaat tggcattata tcagagcaca 10320

gccgaagatc cactgccgac aacatgacct tggcacactc cggttcaaat aagcacagga 10380

ttaacaatag tcaattcaag aagaataaag acaacaaaca tgagatgcct gatgatgggt 10440

ttgagatagc agcctgcttc ctaacaaccg acctcacaaa atactgctta aattggaggt 10500

accaagtcat catccccttt gcacgtacat tgaattcaat gtacggtata ccccacctgt 10560

ttgaatggat acatttaagg ctgatgcgaa gcactctcta tgtcggtgat cccttcaatc 10620

ctccatcaga tcctacccaa cttgaccttg ataccgcact caacgatgat atatttatag 10680

tttcccctcg tggcggaatc gagggtttat gtcaaaaatt atggactatg atttccatct 10740

caacaatcat attatccgca actgaggcaa acactagagt aatgagcatg gttcagggcg 10800
ataaccaagc aattgcaatc accactagag tagtgcgctc gctcagtcat tccgagaaga 10860
aagagcaagc ttataaagca agtaaattat tctttgaaag acttagagct aacaaccatg 10920
gaattggaca ccacttaaaa gaacaagaaa caatccttag ttctgatttc ttcatataca 10980
gtaagagggt gttttacaaa ggtcgaatct tgactcaagc gttaaagaac gtgagcaaga 11040
tgtgcttaac agctgatata ctggggggatt gttcacaagc atcatgttcc aatttagcta 11100
ccactgtaat gcgtcttact gagaatgggg tcgagaaaga tttgtgttat ttcctaaatg 11160
cattcatgac aattagacaa ttatgttatg atctagtatt tccccaaact aaatctctta 11220
gtcaggacat tactaatgct tatcttaatc atccaatact tatctcaaga ttgtgtctat 11280
taccatctca attgggggggc ttaaactttc tttcatgtag ccgcctgttt aatagaaaca 11340
taggagatcc actagtgtct gcaattgctg atgtgaaacg attaattaaa gcgggctgtc 11400
tagatatctg ggtcctgtac aacatccttg gaaggaggcc tggaaagggc aagtggagca 11460
ctctggcagc tgatccctat actttaaaca tagactattt agtcccttca acaacttttt 11520
taaagaaaca tgcccaatat acactgatgg aacggagtgt taatcccatg ctccgtggag 11580
tatttagcga aaatgcagct gaggaggaag aggaactcgc acagtatcta ctagatcgcg 11640
aagtagtcat gcccagggtt gcacatgtta tacttgccca gtctagttgc ggtagaagaa 11700
aacagatcca aggttacttg gattctacta gaactattat caggtattca ctggaggtga 11760
gaccactgtc agcaaagaag ctgaatacgg taatagaata caacttattg tatctgtcct 11820
acaatttgga gattatcgaa aaacccaata tagtccaacc ttttttgaat gcaatcaatg 11880
ttgatacttg tagcatcgat atagctaggt cccttagaaa actatcctgg gcaactttac 11940
ttaatggacg tcccatcgag ggattagaaa cacctgatcc tattgaattg gtacatgggt 12000
gtttaataat cgggtcagat gagtgtgagc attgcagtag tggtgatgac aaattcacct 12060
ggtttttcct ccccaagggg ataaggttag atgatgatcc ggcatctaac ccacccatca 12120
gagtacctta tatcggatct aaaacagatg aacgaagggt tgcatcaatg gcttatatca 12180
aaggggcatc agtatcactt aaatcagcac tcaggttagc gggggtatat atctgggctt 12240
tcggagatac agaggaatca tggcaggatg cctatgagtt agcttccact cgtgttaatc 12300
tcacactaga gcaattgcaa tcgcttactc ctttaccaac atctgccaac ctagtccaca 12360
gattggatga tggcactact caattaaaat ttacccctgc aagctcctat gcattctcta 12420
gctttgttca tatatctaac gactgtcaaa ttcttgagat cgatgatcag gtaacggatt 12480
ctaacctgat ttaccagcaa gttatgatta ctggccttgc tttaattgag acatggaata 12540
atcctccaat caacttctcc gtttatgaaa ctacattaca cttgcataca ggctcatctt 12600

gctgtataag gcctgtcgag tcttgtgtag taaatccgcc tttacttcct gtccctttca 12660
ttaatgttcc tcaaatgaat aaatttgtat atgaccctga accacttagt ttgctagaaa 12720
tggaaaaaat tgaggatatt gcttatcaaa ccagaattgg tggtttagat caaatcccgc 12780
ttctggaaaa aataccctta ctagctcacc ttaccgccaa acagatggtg aatagcatca 12840
ctgggcttga tgaagcaaca tctataatga atgatgctgt agttcaagca gactatacta 12900
gcaattggat tggtgaatgc tgctacactt acattgactc tgtgtttgtt tactctggct 12960
gggcattgtt attggaactt tcataccaaa tgtattacct aagaattcga ggcatacaag 13020
gaattctaga ctatgtgtat atgaccttga ggaggatacc aggaatggcc ataacaggca 13080
tctcatccac aattagtcac cctcgtatac tcagaagatg catcaatttg gatgtcatag 13140
ccccaatcaa ttctccacac atagcttcac tggattacac aaaattgagc atagatgcag 13200
taatgtgggg aaccaagcag gtgttgacca acatttctca aggtatcgat tatgagatag 13260
ttgttccttc tgaaagccaa cttacactca gtgatagagt cctaaatcta gttgctcgaa 13320
aactatcact actggcaatc atctgggcca attacaacta tcctccgaag gttaaaggta 13380
tgtcacctga ggacaaatgt caggctttaa ctacacatct actccaaact gtcgaatatg 13440
ttgagtacat tcagattgaa aagacaaaca tcaggaggat gattattgaa ccaaaattaa 13500
ctgcctaccc tagtaatttg ttttatctct ctcgaaagct gcttaatgct attcgagact 13560
ctgaagaagg acaattcctg attgcatcct attataacag ttttggatat ctggaaccga 13620
tattaatgga atctaaagta ttcaatctaa gttcatccga atcagcatct cttacagaat 13680
tcgatttcat cctcaacttg gaattgtccg acgccagact tgagaaatac tctctcccaa 13740
gtttgcttat gacggctgag aatatggata acccatttcc tcaaccccca cttcatcacg 13800
ttctcagacc actaggtttg tcatccacct catggtataa aacaatcagt gttttgaatt 13860
atattagcca tatgaagata tctgacggtg cccatctata cttggcagag ggaagtggag 13920
cctctatgtc acttatagag actttcttgc ccggggaaac catatggtac aacagcctgt 13980
tcaatagtgg tgagaatccc cctcaacgta atttcgcccc tttgcccacc cagtttattg 14040
aaagtgtccc ctatagatta attcaagcag gtatagcagc aggaaatggt atagtgcaaa 14100
gtttctatcc actctggaac ggaaacagcg atataactga cttaagcact aaaactagtg 14160
ttgaatacat tatccacaag gtaggagctg atacttgtgc attagttcat gtggatttgg 14220
aaggtgtccc tggctcaatg aacagcatgt tggagagagc tcaagtacac gcactactaa 14280
tcacagtcac tgtactgaaa ccaggcggct tactaatctt gaaagcttca tgggaaccct 14340
ttaatcgatt ttccttttta ctcacagtac tctggcaatt cttttccaca ataaggatct 14400
tgcgatcttc atactccgac ccgaataatc acgaggttta cataatagcc acattggcag 14460
ttgatcccac tacatcctcc tttacaactg ctctgaatag ggcacgcacc ctgaatgaac 14520

agggcttttc actcatccca cctgaattag taagtgagta ctggaggaag cgtgttgaac 14580
aaggacagat tatacaggac tgtatagata aagttatatc agagtgtgtc agagatcaat 14640
atctggcaga caacaacatt atcctccagg cgggaggtac tccaagcaca agaaaatggt 14700
tggatctgcc tgactattct tcgttcaatg aactacaatc tgaaatggcc agactcataa 14760
caattcatct taaagaggta atagaaatcc taaagggcca agcatcagat catgacaccc 14820
tattatttac ttcatacaat gtaggtcccc tcggaaaaat aaatacaata cttagattga 14880
ttgttgagag aattcttatg tatactgtga ggaactggtg tatcttgcct acccaaactc 14940
gtctcacctt acgacaatct atcgagcttg gagagtttag actaagagat gtgataacac 15000
ccatggagat tctaaaacta tcccccaaca ggaaatatct aaagtctgca ttaaatcaat 15060
cgacattcaa ccatctaatg ggagaaacat ctgacatatt gttaaaccga gcttatcaga 15120
agagaatttg gaaagccatt gggtgtgtaa tctattgctt tggtttgctc accccggatg 15180
ttgaagattc tgagcgcatt gatattgaca atgacatacc cgattatgat attcacgggg 15240
acataattta aatcgaataa agactcttct ggcattacac accaccaaga agtgccaaac 15300
cagcacccaa actcttctaa accgcccacg acctcgaaca atcacaacca catcagtatt 15360
aaatacagaa gatcctttta agaaaaaatt gattctactt tctccccttg gtggccggca 15420
tggtcccagc ctcctcgctg gcgccggctg ggcaacattc cgaggggacc gtcccctcgg 15480
taatggcgaa tgggacgcgg ccgatccggc tgctaacaaa gcccgaaagg aagctgagtt 15540
ggctgctgcc accgctgagc aataactagc ataacccctt ggggcctcta aacgggtctt 15600
gaggggtttt ttgctgaaag gaggaactat atccggatgc ggccgatccg gctgctaaca 15660
aagcccgaaa ggaagctgag ttggctgctg ccaccgctga gcaataacta gcataacccc 15720
ttggggcctc taaacgggtc ttgaggggtt ttttgctgaa aggaggaact atatccggat 15780
ggccgccacc ggccggtggg ccttgcagca catccccctt tcgccagcag cctgaatggc 15840
gaatgggacg cgccctgtag cggcgcatta agcgcggcgg gtgtggtggt tacgcgcagc 15900
gtgaccgcta cacttgccag cgccctagcg cccgctcctt tcgctttctt cccttccttt 15960
ctcgccacgt tcgccggctt tccccgtcaa gctctaaatc gggggctccc tttagggttc 16020
cgatttagtg ctttacggca cctcgacccc aaaaaacttg attagggtga tggttcacgt 16080
gggccatcgc cctgatagac ggtttttcgc cctttgacgt tggagtccac gttctttaat 16140
agtggactct tgttccaaac tggaacaaca ctcaacccta tctcggtcta ttcttttgat 16200
ttataaggga ttttgccgat ttcggcctat tggttaaaaa atgagctgat ttaacaaaaa 16260
tttaacgcga attttaacaa aatattaacg cttacaattt aggtggcact tttcggggaa 16320
atgtgcgcgg aacccctatt tgtttatttt tctaaataca ttcaaatatg tatccgctca 16380

tgagacaata accctgataa atgcttcaat aatattgaaa aaggaagagt atgagtattc 16440

aacatttccg tgtcgccctt attccctttt ttgcggcatt ttgccttcct gtttttgctc 16500

acccagaaac gctggtgaaa gtaaaagatg ctgaagatca gttgggtgca cgagtgggtt 16560

acatcgaact ggatctcaac agcggtaaga tccttgagag ttttcgcccc gaagaacgtt 16620

ttccaatgat gagcactttt aaagttctgc tatgtggcgc ggtattatcc cgtattgacg 16680

ccgggcaaga gcaactcggt cgccgcatac actattctca gaatgacttg gttgagtact 16740

caccagtcac agaaaagcat cttacggatg gcatgacagt aagagaatta tgcagtgctg 16800

ccataaccat gagtgataac actgcggcca acttacttct gacaacgatt ggaggaccga 16860

aggagctaac cgcttttttg cacaacatgg gggatcatgt aactcgcctt gatcgttggg 16920

aaccggagct gaatgaagcc ataccaaacg acgagcgtga caccacgatg cctgtagcaa 16980

tggcaacaac gttgcgtaaa ctattaactg gcgaactact tactctagct tcccggcaac 17040

aattaataga ctggatggag gcggataaag ttgcaggacc acttctgcgc tcggcccttc 17100

cggctggctg gtttattgct gataaatctg gagccggtga gcgtgggtct cgcggtatca 17160

ttgcagcact ggggccagat ggtaagccct cccgtatcgt agttatctac acgacgggga 17220

gtcaggcaac tatggatgaa cgaaatagac agatcgctga gataggtgcc tcactgatta 17280

agcattggta actgtcagac caagtttact catatatact ttagattgat ttaaaacttc 17340

atttttaatt taaaaggatc taggtgaaga tcctttttga taatctcatg accaaaatcc 17400

cttaacgtga gttttcgttc cactgagcgt cagaccccgt agaaaagatc aaaggatctt 17460

cttgagatcc tttttttctg cgcgtaatct gctgcttgca aacaaaaaaa ccaccgctac 17520

cagcggtggt ttgtttgccg gatcaagagc taccaactct ttttccgaag gtaactggct 17580

tcagcagagc gcagatacca aatactgttc ttctagtgta gccgtagtta ggccaccact 17640

tcaagaactc tgtagcaccg cctacatacc tcgctctgct aatcctgtta ccagtggctg 17700

ctgccagtgg cgataagtcg tgtcttaccg ggttggactc aagacgatag ttaccggata 17760

aggcgcagcg gtcgggctga acggggggtt cgtgcacaca gcccagcttg gagcgaacga 17820

cctacaccga actgagatac ctacagcgtg agctatgaga aagcgccacg cttcccgaag 17880

ggagaaaggc ggacaggtat ccggtaagcg gcagggtcgg aacaggagag cgcacgaggg 17940

agcttccagg gggaaacgcc tggtatcttt atagtcctgt cgggtttcgc cacctctgac 18000

ttgagcgtcg atttttgtga tgctcgtcag gggggcggag cctatggaaa aacgccagca 18060

acgcggcctt tttacggttc ctggcctttt gctggccttt tgctcacatg ttctttcctg 18120

cgttatcccc tgattctgtg gataaccgta ttaccgcctt tgagtgagct gataccgctc 18180

gccgcagccg aacgaccgag cgcagcgagt cagtgagcga ggatctagag gatccccg 18238

<210> 8
<211> 15384
<212> DNA
<213> Mumps Virus

<400> 8

accaagggga gaaagaagat gggatatcgg tagaacaaat agtgtaagaa acagtaagcc 60
cggaagtggt gtttcgcgat ttcgaggccg gcctcgatcc tcaccttcca ttgtcactag 120
cgcgcatttt gacactacct ggaaaatgtc gtctgtgctc aaagcattcg agcggttcac 180
gatagaacag gaacttcaag acaggggtga ggagggttca attccgccgg agactttaaa 240
gtcagcagtc aaagtcttcg ttattaacac acccaatccc accacacgct atcagatgct 300
aaacttttgc ctaagaataa tctgcagtca aaatgctagg gcatctcaca gggtaggtgc 360
attgataaca ttattctcac ttccctcagc aggcatgcaa aatcatatta gattagcaga 420
tagatcacct gaagctcaga tagaacgctg cgagattgat ggttttgaac ctggtacata 480
taggctgatt ccaaatgcac gcgccaatct tactgccaat gaaattgctg cctatgcttt 540
gcttgcagat gacctccctc caaccataaa taatggaact ccttacgtac atgcagatgt 600
tgaaggacag ccatgtgatg agattgagca attcctggat cggtgttaca gtgtactaat 660
ccaggcttgg gtaatggtct gtaaatgtat gacagcgtac gaccaacctg ctggatctgc 720
tgatcggcga tttgcgaaat accagcagca aggtcgcctt gaagcaagat acatgctgca 780
gccggaggcc caaaggttga ttcaaactgc catcaggaaa agtcttgttg ttagacagta 840
ccttaccttc gaactccagt tggcgagacg gcaggggttg ctatcaaaca gatactatgc 900
aatggtgggt gacatcggga agtacattga gaattcagga cttactgcct tctttctcac 960
tctcaaatat gcactaggga ccaaatggag tcctctatca ttggctgcat tcaccggtga 1020
actcactaaa ctccgatcct tgatgatgtt atatcgagat ctcggagaac aagccagata 1080
ccttgctctg ttagaggctc cccaaataat ggactttgca cccggaggct acccattaat 1140
attcagttat gctatgggag tcggtacagt cctggatgtc caaatgcgaa attacactta 1200
tgcaagacct ttcctaaacg gttattattt ccagattggg gttgagaccg cacgaaggca 1260
acaaggcact gttgacaaca gagtagcaga tgatctgggc ctgactcctg agcaaagaac 1320
tgaggttact cagcttgttg acaggcttgc aaggggaaga ggtgctggga taccaggtgg 1380
gcctgtgaat ccttttgttc ctccagttca acagcaacaa cctgctgccg tatatgagga 1440
cattcctgca ttagaggaat cagatgacga tggtgatgaa gatagaggcg caggattcca 1500
aaatggagta caagtaccag ctgtaagaca gggaggtcaa actgacttta gagcacagcc 1560
tttacaagat ccaattcaag cacagctttt catgccatta tatcctcaag tcagcaacat 1620
cccaaataat cagaatcatc agatcaatcg catcgggggg ctggaaaacc aagatttatt 1680
acgatacaac gagaatggtg attctcaaca agatgcaagg ggcgaacacg gaaacacttt 1740

cccaaacaat cccaatcaaa acgcacagct gcaagtgggt gactgggatg agtaaatcac 1800
tgatatgatc aaactaaccc caattggact aagtctagga caatctagcc atagcgaact 1860
gcccaaattc actacattct attcataact agtctttaag aaaaaattag gcccggaaag 1920
aattaggtcc acgatcacag gcacaatcat tctgatcgtg tttctttccg ggtaagccat 1980
ggatcaattt ataaaacagg atgagactgg tgatttaatt gagacaggaa tgaatgttgc 2040
aaaccatttc ctatccgccc ccattcaggg aaccaactcg ctgagcaagg cttcaatcat 2100
ccctggcgtt gcacctgtac tcattggcaa tccagagcaa aagaacattc agcaccctac 2160
cgcatcacat cagggatcca agtcaaaggg cagaggctca ggggtcaggt ccatcatagt 2220
cccgccctcc gaagcaggca atggagggac tcagattcct gagccccttt ttgcacaaac 2280
aggacagggt ggtatagtca ccacagtcta tcaggatcca actatccaac caacaggttc 2340
ataccgaagt gtggaattgg cgaagatcgg aaaagagaga atgattaatc gatttgttga 2400
gaaacctaga acctcaacgc cggtgacaga atttaagagg ggggccggga gcggctgctc 2460
aaggccagac aatccaagag gagggcatag acgggaatgg agcctcagct gggtccaagg 2520
agaggtccgg gtctttgagt ggtgcaaccc tatatgctca cctatcactg ccgcagcaag 2580
attccactcc tgcaaatgtg ggaattgccc cgcaaagtgc gaccagtgcg aacgagatta 2640
tggacctcct taggggggatg gatgctcgcc tgcaacatct tgagcaaaag gtggacaagg 2700
tgcttgcaca gggcagcatg gtgacccaaa taaagaatga attatcaaca gtaaagacaa 2760
cattagcaac aattgaaggg atgatggcaa cagtaaaaat catggatcct ggaaatccga 2820
caggggtccc agttgatgag cttagaagaa gttttagtga tcatgtgaca attgttagtg 2880
gaccaggaga tgtgtcgttc agctcccgtg aagaacccac actgtatttg gatgagctgg 2940
cgaggcccgt ctccaagcct cgtcctgcaa agcagacaaa accccaacca gtaaaggatt 3000
tggcaggacg aaaagtgatg atcaccaaaa tgattactga ttgtgtggct aaccctcaaa 3060
tgaagcaggc gttcgagcaa cgattggcaa aggccagcac cgaggatgct ctgaacgaca 3120
tcaagaaaga tatcatacgg agcgccatat gaattcacca gaagcaccag acgcgtggaa 3180
aaatccatga actgagagcc acaatgattc cctattaaat aaaaaataag cacgaacaca 3240
agtcgaatcc aaccatagca gagatggcag gatcacagat caaaattcct cttccaaagc 3300
cccccgattc agactctcaa agactaaatg cattccctgt tatcatggct caagaaggca 3360
aaggacgact tcttagacaa atcaggctta ggaaaatatt atcaggagat ccgtctgatc 3420
agcaaatcac atttgtgaat acatatggat tcatccatgc cactccagaa acatccgagt 3480
tcatctctga atcatcacaa caaaaggtaa ctcctgcagt gacagcgtgc atgctgtcct 3540
ttggtgccgg accggtgcta gaagatccac aacatatgct gaaggctctt gatcagacag 3600

acattagggt tcggaaaaca gcaagtgata aagagcagat cctattcgag atcaaccgca 3660
tccccgatct attcaggcat catcaaatat ctgcggacca tctgattcag gccagctccg 3720
ataaatatgt caagtcacca gcgaaattga ttgcaggagt aaattacatc tactgtgtca 3780
cattcttatc tgtgacagtt tgttctgcct cactcaagtt tcgagttgca cgcccattgc 3840
ttgctgcacg gtctagatta gtaagagcag ttcagatgga agttttgctt cgggtaactt 3900
gcaaaaaaga ttctcaaatg gcaaagagca tgttaaatga ccctgatgga gaagggtgca 3960
ttgcatcagt gtggttccac ctatgtaatc tgtgcaaagg caggaataaa cttaggagtt 4020
acgatgaaaa ttattttgct tctaagtgcc gtaagatgaa tctgacagtc agcataggag 4080
atatgtgggg accaaccatt ctagtccatg caggcggtca cattccgaca actgcaaaac 4140
ctttttttcaa ctcaagaggc tgggtctgcc acccaatcca ccaatcatca ccatcgttgg 4200
cgaagaccct atggtcatct gggtgtgaaa tcaaggctgc cagtgctatc ctccagggtt 4260
cagactatgc atcacttgca aagactgatg acataatata tccgaagata aaagtcgata 4320
aagacgcggc caactacaaa ggagtatcct ggagtccatt caggaagtct gcctcaatga 4380
gtaacctatg agaatttcct ctatttcaac tgatgcctat aagagaatca acaatcaagc 4440
aaatttgacc ggtggtaatt cgtttaaact tatagaaaaa ataagcctag aaggatatcc 4500
tacttctcaa ccttccaact ttgaaaatag aatagatcag taatcatgaa ggcttatcca 4560
gttatttgct tgggctttgc aatcttttca tcctctatat gtgtgaatat caatatcttg 4620
cagcaaattg gatacatcaa gcaacaggtc aggcaactaa gctattactc acaaagttca 4680
agctcctacg tagtggtcaa gcttttaccg aatatccaac ccactgataa cagctgtgaa 4740
tttaagagtg taattcaata caataagacc ttgagtaatt tgcttcttcc aattgcagaa 4800
aacataaaca atattgcatc gccctcacct gggtcaagac gtcataaacg gtttgctggc 4860
attgccattg gcattgctgc gctcggtgtt gcgaccgcgg cacaagtgac tgccgctgtc 4920
tcattagttc aagcacagac aaatgcacgt gcaatagcag cgatgaaaaa ttcaatacag 4980
gcaactaatc gggcagtctt cgaagtgaag gaaggcaccc agcagttagc tatagcggta 5040
caagcaatac aagaccatat caatactatt atgaacaccc aattgaacaa tatgtcttgt 5100
cagatccttg ataaccagct tgcaacctct ctaggattat acctaacaga attaacaaca 5160
gtgttccagc cacaattgat taatccagca ttgtcaccga ttagtatcca agccttgagg 5220
tctttgcttg gaagtatgac acctgcagtg gttcaagcaa cattatctac ttcaatttct 5280
gctgctgaaa tactaagtgc cggtctaatg gagggtcaga tagtttctgt tctgctagat 5340
gagatgcaga tgatagttaa gataaacatt ccaaccattg tcacacaatc aaatgcattg 5400
gtgattgact tctactcaat ttcaagtttc attaataatc aagaatccat aattcaattg 5460
ccagacagga tcttggagat cgggaatgaa caatggcgct atccagctaa gaattgtaag 5520

tcgacaagac atcacatatt ctgccaatac aatgaggcag agaggctgag cctagaaaca 5580
aaactatgcc ttgcaggcaa tattagtgcc tgtgtgttct cacctatagc agggagctat 5640
atgaggcgat ttgtagcgct ggatggaaca attgttgcaa actgtcgaag tctaacgtgt 5700
ctatgcaaga gtccatctta tcctatatac caacctgacc atcatgcagt cacgaccatt 5760
gatctaacgt catgtcaaac attgtccctg gacggactgg atttcagcat tgtctcacta 5820
agcaacatca cttacgctga gaatcttact atttcattgt ctcagacgat caatactcaa 5880
cccattgata tatcaactga gctgagtaag gttaatgcat ccctccaaaa tgccgttaaa 5940
tacataaaag agagtaacca tcaactccaa tccgttagtg taagttctaa aatcggagct 6000
ataattgtag cagccttagt tttgagcatc ctgtcgatta tcatttcgct attgttttgc 6060
tgctgggctt acattgcgac taaagaaatc agaagaatca acttcaaaac aaatcatatc 6120
aacacaatat caagtagtgt cgatgatctc atcaggtact aattttaaat tggtgattca 6180
tcctgcaatt aaaaaaggtt tagaaaaaaa ctaaactaag aatgaatctc ctagggtcgt 6240
aacgtcacgt caccctgccg ttgcactatg ccggcaatcc aacctccctt atacctaaca 6300
tttctattgc taacccttct ctatctaatc ataactctgt atgtctggac tatattgacc 6360
attaaccata atacggcggt tcggtatgca gcactgtacc agcgatcctt ctctcgctgg 6420
ggttttgatc aatcactcta gaaagatcct cagttagggc aagtggcgat cgctcttcct 6480
agaacaagct gcatccaaat gaagctgcac taccatgaga cataaagaaa aaagcaagcc 6540
agaacaaact taggatcaca acacaacaca aaatattagc tgctatcaca actgtgttcc 6600
ggccactaag aaaatggagc cctcgaaact attcataatg tcggacaacg ccacctttgc 6660
acctggacct gttgttaatg cggctggtaa gaagacattc cgaacctgtt tccgaatatt 6720
ggtcctatcc gtacaagcag ttacccttat attggttatt gtcactttag gtgagcttat 6780
taggatgatc aatgatcaag gcttgagcaa tcagttgtct tcaattacag acaagataag 6840
agaatcagct gctatgattg catctgctgt gggagtaatg aatcaagtaa ttcatggagt 6900
aacggtatcc ttacccctac aaattgaggg aaaccaaaat caattattat ccacacttgc 6960
cacaatctgc acaaacagaa accaagtttc aaactgctct acaaacatcc ccttagttaa 7020
tgaccttagg tttataaatg gaatcaataa gttcatcatt gaagattatg caacccatga 7080
tttctccatc ggccatccac tcaacatgcc tagctttatc ccaactgcaa cctcacccaa 7140
tggttgcaca agaattccat ccttttcttt aggtaagaca cattggtgtt acacacataa 7200
tgtaattaat gccaactgca aggatcgtac ttcatcgaac caatatgttt ccatggggat 7260
tctcgttcaa accgcgtcag ggtatcccat gttcaaaacc ctaaaaatcc aatatctcag 7320
tgatggcctg aatcggaaaa gctgctcaat tgcaacagtc cctgatggtt gcgcaatgta 7380

ctgttacgtt tcaactcaac ttgaaaccga cgactatgcg gggtccagcc cacctaccca 7440
gaaacttacc ctgttgttct ataatgacac catcaaagaa aggacaatat ctccgtctgg 7500
tcttgaagga aattgggcta ctttggtgcc aggagtgggg agtggaatat atttcgaaaa 7560
taagttgatc tttcctgcat atgggggtgt cttgcccaat agtacactag gagttaaatc 7620
agcaagagaa tttttccggc ccgttaatcc atataatcca tgttcaggac caccacaaga 7680
gttagatcag cgtgctttga gatcatattt cccaagttac ttctctagtc gaagggtaca 7740
gagtgcattt ctggtctgtg cctggaatca gatcctagtt acaaattgcg agctagttgt 7800
cccctcaaac aatcagacac ttatgggtgc agaaggaaga gttttattga tcaataatcg 7860
gctattatat tatcagagga gtactagctg gtggccgtat gaactcctct atgagatatc 7920
attcacattt acaaactctg gtcaatcatc tgtgaatatg tcctggatac ctatatattc 7980
attcacccgt cctggtttgg gcaactgccg tggtgaaaat atatgcccaa cagtctgtgt 8040
atcaggagtt tatcttgatc cctggccatt aactccatac agccatcaat caggcattaa 8100
cagaaatttc tatttcacag gtgcactgct aaattcaagc acaaccaggg tgaatcctac 8160
cctttatgtc tctgccctta ataatcttaa agtactagcc ccatatggta ctcaaggatt 8220
gtttgcgtca tacaccacaa ccacctgctt tcaagatacc ggtgacgcta gtgtgtattg 8280
tgtctatatt atggaactag catcgaatat tgttggagaa ttccaaattc tacctgtgct 8340
agccagattg accatcactt gagttgtagt gaatgtagta ggaagcttta tgggcgtgtc 8400
tcatttcttc tcgagtatta agaaaaaaca ggccagaatg gcgggcctaa atgagatact 8460
cctacccgaa gtacatttaa actcccccat cgttagatat aagcttttct actatatatt 8520
gcatggccag ttaccaaatg atttggagcc agatgacttg ggcccattag caaatcataa 8580
ttggaaggca attcgagctg aggaatccca ggttcatgca cgattaaaac agatcagagt 8640
agaactcatt gcaaggattc ctagtctccg gtggacccgc tctcaaagag agattgccat 8700
actcatttgg ccaagaatac ttccaatact gcaagcatat gatcttcggc aaagtatgca 8760
attgcccaca gtgtgggaga aattgactca atccacggtt aatcttataa gtgatggtct 8820
agaacgggtt gtattacaca tcagcaatca attaacaggc aagcctaact tgtttaccag 8880
gtctagagct ggacaagaca caaaagatta ctcaattcca tccactagag agctatctca 8940
aatatggttt aacaatgagt ggagtgggtc tgtgaagacc tggcttatga ttaaatatag 9000
aatgaggcag ctaatcacaa atcaaaagac aggtgagtta acagatttag taaccattgt 9060
ggatactagg tccactctat gcattattac cccagaatta gtcgctttat actccaatga 9120
gcacaaagca ttaacgtacc tcacctttga aatggtatta atggtcactg atatgttaga 9180
gggaagactg aatgtttctt ctctgtgcac agctagtcat tatctgtccc ctttaaagaa 9240
gcgaatcgaa gttctcctga cattagttga tgaccttgct ctactcatgg gggataaagt 9300

atacggtatt gtctcttcac ttgagagttt tgtttacgcc caattacagt atggtgatcc 9360

tgttatagac attaaaggta cattctatgg atttatatgt aatgagattc tcgacctact 9420

gactgaaggc aacatcttta ctgaagaaga ggcaaacaag gttcttctgg acttgacgtc 9480

acagtttgac aatctatccc ctgatttaac agctgagctc ctctgcatta tgagactttg 9540

gggccatccc accttaactg ccagccaagc agcatccaag gtccgagagt ccatgtgcgc 9600

tcctaaggtg ttagatttcc aaacaataat gaaaaccctg gctttctttc acgcaatcct 9660

aattaacggt tataggagga gccataatgg aatctggccg cctactactc ttcatggcaa 9720

tgcccccaaa agcctcattg agatgcggca tgataattca gagcttaagt atgagtatgt 9780

cctcaagaat tggaaaagta tatctatgtt aagaatacac aaatgctttg atgcatcacc 9840

tgatgaagat ctcagcatat tcatgaagga taaggcaata agctgtccaa agcaagactg 9900

gatgggagta tttaggagga gcctgattaa acagcgctat cgtgacgtga atcggcctct 9960

accacaacca tttaaccgga gactgctgtt gaatttccta gaggatgacc gattcgatcc 10020

tattaaagag cttgagtatg tcaccagtgg agaatatctt agggaccctg aattttgtgc 10080

atcttactct ctcaaagaga aagagataaa ggctacaggt cgtatatttg caaaaatgac 10140

aaagagaatg agatcgtgcc aagtaattgc agaatcattg ttagccaatc acgcaggtaa 10200

attaatgaga gagaatggag ttgtcttaga ccagttgaaa ttaacaaaat ctttattaac 10260

tatgaaccaa attggcatta tatcagagca cagccgaaga tccactgccg acaacatgac 10320

cttggcacac tccggttcaa ataagcacag gattaacaat agtcaattca agaagaataa 10380

agacaacaaa catgagatgc ctgatgatgg gtttgagata gcagcctgct tcctaacaac 10440

cgacctcaca aaatactgct taaattggag gtaccaagtc atcatcccct ttgcacgtac 10500

attgaattca atgtacggta taccccacct gtttgaatgg atacatttaa ggctgatgcg 10560

aagcactctc tatgtcggtg atcccttcaa tcctccatca gatcctaccc aacttgacct 10620

tgataccgca ctcaacgatg atatatttat agtttcccct cgtggcggaa tcgagggttt 10680

atgtcaaaaa ttatggacta tgatttccat ctcaacaatc atattatccg caactgaggc 10740

aaacactaga gtaatgagca tggttcaggg cgataaccaa gcaattgcaa tcaccactag 10800

agtagtgcgc tcgctcagtc attccgagaa gaaagagcaa gcttataaag caagtaaatt 10860

attctttgaa agacttagag ctaacaacca tggaattgga caccacttaa aagaacaaga 10920

aacaatcctt agttctgatt tcttcatata cagtaagagg gtgttttaca aaggtcgaat 10980

cttgactcaa gcgttaaaga acgtgagcaa gatgtgctta acagctgata tactggggga 11040

ttgttcacaa gcatcatgtt ccaatttagc taccactgta atgcgtctta ctgagaatgg 11100

ggtcgagaaa gatttgtgtt atttcctaaa tgcattcatg acaattagac aattatgtta 11160

tgatctagta tttccccaaa ctaaatctct tagtcaggac attactaatg cttatcttaa 11220

tcatccaata cttatctcaa gattgtgtct attaccatct caattggggg gcttaaactt 11280

tctttcatgt agccgcctgt ttaatagaaa cataggagat ccactagtgt ctgcaattgc 11340

tgatgtgaaa cgattaatta aagcgggctg tctagatatc tgggtcctgt acaacatcct 11400

tggaaggagg cctggaaagg gcaagtggag cactctggca gctgatccct atactttaaa 11460

catagactat ttagtccctt caacaacttt tttaaagaaa catgcccaat atacactgat 11520

ggaacggagt gttaatccca tgctccgtgg agtatttagc gaaaatgcag ctgaggagga 11580

agaggaactc gcacagtatc tactagatcg cgaagtagtc atgcccaggg ttgcacatgt 11640

tatacttgcc cagtctagtt gcggtagaag aaaacagatc caaggttact tggattctac 11700

tagaactatt atcaggtatt cactggaggt gagaccactg tcagcaaaga agctgaatac 11760

ggtaatagaa tacaacttat tgtatctgtc ctacaatttg gagattatcg aaaaacccaa 11820

tatagtccaa ccttttttga atgcaatcaa tgttgatact tgtagcatcg atatagctag 11880

gtcccttaga aaactatcct gggcaacttt acttaatgga cgtcccatcg agggattaga 11940

aacacctgat cctattgaat tggtacatgg gtgtttaata atcgggtcag atgagtgtga 12000

gcattgcagt agtggtgatg acaaattcac ctggtttttc ctccccaagg ggataaggtt 12060

agatgatgat ccggcatcta acccacccat cagagtacct tatatcggat ctaaaacaga 12120

tgaacgaagg gttgcatcaa tggcttatat caaaggggca tcagtatcac ttaaatcagc 12180

actcaggtta gcgggggtat atatctgggc tttcggagat acagaggaat catggcagga 12240

tgcctatgag ttagcttcca ctcgtgttaa tctcacacta gagcaattgc aatcgcttac 12300

tcctttacca acatctgcca acctagtcca cagattggat gatggcacta ctcaattaaa 12360

atttacccct gcaagctcct atgcattctc tagctttgtt catatatcta acgactgtca 12420

aattcttgag atcgatgatc aggtaacgga ttctaacctg atttaccagc aagttatgat 12480

tactggcctt gctttaattg agacatggaa taatcctcca atcaacttct ccgtttatga 12540

aactacatta cacttgcata caggctcatc ttgctgtata aggcctgtcg agtcttgtgt 12600

agtaaatccg cctttacttc ctgtcccttt cattaatgtt cctcaaatga ataaatttgt 12660

atatgaccct gaaccactta gtttgctaga aatggaaaaa attgaggata ttgcttatca 12720

aaccagaatt ggtggtttag atcaaatccc gcttctggaa aaaatacccc tactagctca 12780

ccttaccgcc aaacagatgg tgaatagcat cactgggctt gatgaagcaa catctataat 12840

gaatgatgct gtagttcaag cagactatac tagcaattgg attggtgaat gctgctacac 12900

ttacattgac tctgtgtttg tttactctgg ctgggcattg ttattggaac tttcatacca 12960

aatgtattac ctaagaattc gaggcataca aggaattcta gactatgtgt atatgacctt 13020

gaggaggata ccaggaatgg ccataacagg catctcatcc acaattagtc accctcgtat 13080

actcagaaga tgcatcaatt tggatgtcat agccccaatc aattctccac acatagcttc 13140
actggattac acaaaattga gcatagatgc agtaatgtgg ggaaccaagc aggtgttgac 13200
caacatttct caaggtatcg attatgagat agttgttcct tctgaaagcc aacttacact 13260
cagtgataga gtcctaaatc tagttgctcg aaaactatca ctactggcaa tcatctgggc 13320
caattacaac tatcctccga aggttaaagg tatgtcacct gaggacaaat gtcaggcttt 13380
aactacacat ctactccaaa ctgtcgaata tgttgagtac attcagattg aaaagacaaa 13440
catcaggagg atgattattg aaccaaaatt aactgcctac cctagtaatt tgttttatct 13500
ctctcgaaag ctgcttaatg ctattcgaga ctctgaagaa ggacaattcc tgattgcatc 13560
ctattataac agttttggat atctggaacc gatattaatg gaatctaaag tattcaatct 13620
aagttcatcc gaatcagcat ctcttacaga attcgatttc atcctcaact tggaattgtc 13680
cgacgccaga cttgagaaat actctctccc aagtttgctt atgacggctg agaatatgga 13740
taacccattt cctcaacccc cacttcatca cgttctcaga ccactaggtt tgtcatccac 13800
ctcatggtat aaaacaatca gtgttttgaa ttatattagc catatgaaga tatctgacgg 13860
tgcccatcta tacttggcag agggaagtgg agcctctatg tcacttatag agactttctt 13920
gcccggggaa accatatggt acaacagcct gttcaatagt ggtgagaatc cccctcaacg 13980
taatttcgcc cctttgccca cccagtttat tgaaagtgtc ccctatagat taattcaagc 14040
aggtatagca gcaggaaatg gtatagtgca aagtttctat ccactctgga acggaaacag 14100
cgatataact gacttaagca ctaaaactag tgttgaatac attatccaca aggtaggagc 14160
tgatacttgt gcattagttc atgtggattt ggaaggtgtc cctggctcaa tgaacagcat 14220
gttggagaga gctcaagtac acgcactact aatcacagtc actgtactga aaccaggcgg 14280
cttactaatc ttgaaagctt catgggaacc ctttaatcga ttttcctttt tactcacagt 14340
actctggcaa ttcttttcca caataaggat cttgcgatct tcatactccg acccgaataa 14400
tcacgaggtt tacataatag ccacattggc agttgatccc actacatcct cctttacaac 14460
tgctctgaat agggcacgca ccctgaatga acagggcttt tcactcatcc cacctgaatt 14520
agtaagtgag tactggagga agcgtgttga acaaggacag attatacagg actgtataga 14580
taaagttata tcagagtgtg tcagagatca atatctggca gacaacaaca ttatcctcca 14640
ggcgggaggt actccaagca caagaaaatg gttggatctg cctgactatt cttcgttcaa 14700
tgaactacaa tctgaaatgg ccagactcat aacaattcat cttaaagagg taatagaaat 14760
cctaaagggc caagcatcag atcatgacac cctattattt acttcataca atgtaggtcc 14820
cctcggaaaa ataaatacaa tacttagatt gattgttgag agaattctta tgtatactgt 14880
gaggaactgg tgtatcttgc ctacccaaac tcgtctcacc ttacgacaat ctatcgagct 14940

tggagagttt agactaagag atgtgataac acccatggag attctaaaac tatcccccaa 15000

caggaaatat ctaaagtctg cattaaatca atcgacattc aaccatctaa tgggagaaac 15060

atctgacata ttgttaaacc gagcttatca gaagagaatt tggaaagcca ttgggtgtgt 15120

aatctattgc tttggtttgc tcaccccgga tgttgaagat tctgagcgca ttgatattga 15180

caatgacata cccgattatg atattcacgg ggacataatt taaatcgaat aaagactctt 15240

ctggcattac acaccaccaa gaagtgccaa accagcaccc aaactcttct aaaccgccca 15300

cgacctcgaa caatcacaac cacatcagta ttaaatacag aagatccttt taagaaaaaa 15360

ttgattctac tttctcccct tggt 15384

<210> 9
<211> 1650
<212> DNA
<213> Mumps Virus

<400> 9

```
atgtcgtctg tgctcaaagc attcgagcgg ttcacgatag aacaggaact tcaagacagg      60
ggtgaggagg gttcaattcc gccggagact ttaaagtcag cagtcaaagt cttcgttatt     120
aacacaccca atcccaccac acgctatcag atgctaaact tttgcctaag aataatctgc     180
agtcaaaatg ctagggcatc tcacagggta ggtgcattga taacattatt ctcacttccc     240
tcagcaggca tgcaaaatca tattagatta gcagatagat cacctgaagc tcagatagaa     300
cgctgcgaga ttgatggttt tgaacctggt acatataggc tgattccaaa tgcacgcgcc     360
aatcttactg ccaatgaaat tgctgcctat gctttgcttg cagatgacct ccctccaacc     420
ataaataatg gaactcctta cgtacatgca gatgttgaag gacagccatg tgatgagatt     480
gagcaattcc tggatcggtg ttacagtgta ctaatccagg cttgggtaat ggtctgtaaa     540
tgtatgacag cgtacgacca acctgctgga tctgctgatc ggcgatttgc gaaataccag     600
cagcaaggtc gccttgaagc aagatacatg ctgcagccgg aggcccaaag gttgattcaa     660
actgccatca ggaaaagtct tgttgttaga cagtacctta ccttcgaact ccagttggcg     720
agacggcagg ggttgctatc aaacagatac tatgcaatgg tgggtgacat cgggaagtac     780
attgagaatt caggacttac tgccttcttt ctcactctca aatatgcact agggaccaaa     840
tggagtcctc tatcattggc tgcattcacc ggtgaactca ctaaactccg atccttgatg     900
atgttatatc gagatctcgg agaacaagcc agataccttg ctctgttaga ggctccccaa     960
ataatggact ttgcacccgg aggctaccca ttaatattca gttatgctat gggagtcggt    1020
acagtcctgg atgtccaaat gcgaaattac acttatgcaa gacctttcct aaacggttat    1080
tatttccaga ttggggttga gaccgcacga aggcaacaag gcactgttga caacagagta    1140
gcagatgatc tgggcctgac tcctgagcaa agaactgagg ttactcagct tgttgacagg    1200
cttgcaaggg gaagaggtgc tgggatacca ggtgggcctg tgaatccttt tgttcctcca    1260
gttcaacagc aacaacctgc tgccgtatat gaggacattc ctgcattaga ggaatcagat    1320
gacgatggtg atgaagatag aggcgcagga ttccaaaatg gagtacaagt accagctgta    1380
agacagggag gtcaaactga ctttagagca cagcctttac aagatccaat tcaagcacag    1440
cttttcatgc cattatatcc tcaagtcagc aacatcccaa ataatcagaa tcatcagatc    1500
aatcgcatcg gggggctgga aaaccaagat ttattacgat acaacgagaa tggtgattct    1560
caacaagatg caaggggcga acacggaaac actttcccaa acaatcccaa tcaaaacgca    1620
cagctgcaag tgggtgactg ggatgagtaa                                     1650
```

<210> 10
<211> 1176
<212> DNA
<213> Mumps Virus

<400> 10

```
atggatcaat ttataaaaca ggatgagact ggtgatttaa ttgagacagg aatgaatgtt      60
gcaaaccatt tcctatccgc ccccattcag ggaaccaact cgctgagcaa ggcttcaatc     120
atccctggcg ttgcacctgt actcattggc aatccagagc aaaagaacat tcagcaccct     180
accgcatcac atcagggatc caagtcaaag ggcagaggct caggggtcag gtccatcata     240
gtcccgccct ccgaagcagg caatggaggg actcagattc ctgagcccct ttttgcacaa     300
acaggacagg gtggtatagt caccacagtc tatcaggatc caactatcca accaacaggt     360
tcataccgaa gtgtggaatt ggcgaagatc ggaaaagaga gaatgattaa tcgatttgtt     420
gagaaaccta gaacctcaac gccggtgaca gaatttaaga ggggggggcc gggagcggct     480
gctcaaggcc agacaatcca agaggagggc atagacggga atggagcctc agctgggtcc     540
aaggagaggt ccgggtcttt gagtggtgca accctatatg ctcacctatc actgccgcag     600
caagattcca ctcctgcaaa tgtgggaatt gccccgcaaa gtgcgaccag tgcgaacgag     660
attatggacc tccttagggg gatggatgct cgcctgcaac atcttgagca aaaggtggac     720
aaggtgcttg cacagggcag catggtgacc caaataaaga atgaattatc aacagtaaag     780
acaacattag caacaattga agggatgatg gcaacagtaa aaatcatgga tcctggaaat     840
ccgacagggg tcccagttga tgagcttaga agaagtttta gtgatcatgt gacaattgtt     900
agtggaccag gagatgtgtc gttcagctcc cgtgaagaac ccacactgta tttggatgag     960
ctggcgaggc ccgtctccaa gcctcgtcct gcaaagcaga caaaacccca accagtaaag    1020
gatttggcag gacgaaaagt gatgatcacc aaaatgatta ctgattgtgt ggctaaccct    1080
caaatgaagc aggcgttcga gcaacgattg gcaaaggcca gcaccgagga tgctctgaac    1140
gacatcaaga aagatatcat acggagcgcc atatga                              1176
```

<210> 11
<211> 6786
<212> DNA
<213> Mumps Virus

<400> 11

atggcgggcc taaatgagat actcctaccc gaagtacatt taaactcccc catcgttaga 60

tataagcttt tctactatat attgcatggc cagttaccaa atgatttgga gccagatgac 120

ttgggcccat tagcaaatca taattggaag gcaattcgag ctgaggaatc ccaggttcat 180

gcacgattaa aacagatcag agtagaactc attgcaagga ttcctagtct ccggtggacc 240

cgctctcaaa gagagattgc catactcatt tggccaagaa tacttccaat actgcaagca 300

tatgatcttc ggcaaagtat gcaattgccc acagtgtggg agaaattgac tcaatccacg 360

gttaatctta taagtgatgg tctagaacgg gttgtattac acatcagcaa tcaattaaca 420

ggcaagccta acttgtttac caggtctaga gctggacaag acacaaaaga ttactcaatt 480

ccatccacta gagagctatc tcaaatatgg tttaacaatg agtggagtgg gtctgtgaag 540

acctggctta tgattaaata tagaatgagg cagctaatca caaatcaaaa gacaggtgag 600

ttaacagatt tagtaaccat tgtggatact aggtccactc tatgcattat taccccagaa 660

ttagtcgctt tatactccaa tgagcacaaa gcattaacgt acctcacctt tgaaatggta 720

ttaatggtca ctgatatgtt agagggaaga ctgaatgttt cttctctgtg cacagctagt 780

cattatctgt cccctttaaa gaagcgaatc gaagttctcc tgacattagt tgatgacctt 840

gctctactca tgggggataa agtatacggt attgtctctt cacttgagag ttttgtttac 900

gcccaattac agtatggtga tcctgttata gacattaaag gtacattcta tggatttata 960

tgtaatgaga ttctcgacct actgactgaa ggcaacatct ttactgaaga agaggcaaac 1020

aaggttcttc tggacttgac gtcacagttt gacaatctat cccctgattt aacagctgag 1080

ctcctctgca ttatgagact ttggggccat cccaccttaa ctgccagcca agcagcatcc 1140

aaggtccgag agtccatgtg cgctcctaag gtgttagatt tccaaacaat aatgaaaacc 1200

ctggctttct ttcacgcaat cctaattaac ggttatagga ggagccataa tggaatctgg 1260

ccgcctacta ctcttcatgg caatgccccc aaaagcctca ttgagatgcg gcatgataat 1320

tcagagctta agtatgagta tgtcctcaag aattggaaaa gtatatctat gttaagaata 1380

cacaaatgct ttgatgcatc acctgatgaa gatctcagca tattcatgaa ggataaggca 1440

ataagctgtc caaagcaaga ctggatggga gtatttagga ggagcctgat taaacagcgc 1500

tatcgtgacg tgaatcggcc tctaccacaa ccatttaacc ggagactgct gttgaatttc 1560

ctagaggatg accgattcga tcctattaaa gagcttgagt atgtcaccag tggagaatat 1620

cttagggacc ctgaattttg tgcatcttac tctctcaaag agaaagagat aaaggctaca 1680

ggtcgtatat ttgcaaaaat gacaaagaga atgagatcgt gccaagtaat tgcagaatca 1740

ttgttagcca atcacgcagg taaattaatg agagagaatg gagttgtctt agaccagttg 1800

aaattaacaa aatctttatt aactatgaac caaattggca ttatatcaga gcacagccga 1860

agatccactg ccgacaacat gaccttggca cactccggtt caaataagca caggattaac 1920

aatagtcaat tcaagaagaa taaagacaac aaacatgaga tgcctgatga tgggtttgag 1980

atagcagcct gcttcctaac aaccgacctc acaaaatact gcttaaattg gaggtaccaa 2040

gtcatcatcc cctttgcacg tacattgaat tcaatgtacg gtatacccca cctgtttgaa 2100

tggatacatt taaggctgat gcgaagcact ctctatgtcg gtgatccctt caatcctcca 2160

tcagatccta cccaacttga ccttgatacc gcactcaacg atgatatatt tatagtttcc 2220

cctcgtggcg gaatcgaggg tttatgtcaa aaattatgga ctatgatttc catctcaaca 2280

atcatattat ccgcaactga ggcaaacact agagtaatga gcatggttca gggcgataac 2340

caagcaattg caatcaccac tagagtagtg cgctcgctca gtcattccga gaagaaagag 2400

caagcttata aagcaagtaa attattcttt gaaagactta gagctaacaa ccatggaatt 2460

ggacaccact taaaagaaca agaaacaatc cttagttctg atttcttcat atacagtaag 2520

agggtgtttt acaaaggtcg aatcttgact caagcgttaa agaacgtgag caagatgtgc 2580

ttaacagctg atatactggg ggattgttca caagcatcat gttccaattt agctaccact 2640

gtaatgcgtc ttactgagaa tggggtcgag aaagatttgt gttatttcct aaatgcattc 2700

atgacaatta gacaattatg ttatgatcta gtatttcccc aaactaaatc tcttagtcag 2760

gacattacta atgcttatct taatcatcca atacttatct caagattgtg tctattacca 2820

tctcaattgg ggggcttaaa ctttctttca tgtagccgcc tgtttaatag aaacatagga 2880

gatccactag tgtctgcaat tgctgatgtg aaacgattaa ttaaagcggg ctgtctagat 2940

atctgggtcc tgtacaacat ccttggaagg aggcctggaa agggcaagtg gagcactctg 3000

gcagctgatc cctatacttt aaacatagac tatttagtcc cttcaacaac ttttttaaag 3060

aaacatgccc aatatacact gatggaacgg agtgttaatc ccatgctccg tggagtattt 3120

agcgaaaatg cagctgagga ggaagaggaa ctcgcacagt atctactaga tcgcgaagta 3180

gtcatgccca gggttgcaca tgttatactt gcccagtcta gttgcggtag aagaaaacag 3240

atccaaggtt acttggattc tactagaact attatcaggt attcactgga ggtgagacca 3300

ctgtcagcaa agaagctgaa tacggtaata gaatacaact tattgtatct gtcctacaat 3360

ttggagatta tcgaaaaacc caatatagtc caaccttttt tgaatgcaat caatgttgat 3420

acttgtagca tcgatatagc taggtccctt agaaaactat cctgggcaac tttacttaat 3480

ggacgtccca tcgagggatt agaaacacct gatcctattg aattggtaca tgggtgttta 3540

ataatcgggt cagatgagtg tgagcattgc agtagtggtg atgacaaatt cacctggttt 3600

ttcctcccca aggggataag gttagatgat gatccggcat ctaacccacc catcagagta 3660

ccttatatcg gatctaaaac agatgaacga agggttgcat caatggctta tatcaaaggg 3720

gcatcagtat cacttaaatc agcactcagg ttagcggggg tatatatctg ggctttcgga 3780

gatacagagg aatcatggca ggatgcctat gagttagctt ccactcgtgt taatctcaca 3840

ctagagcaat tgcaatcgct tactccttta ccaacatctg ccaacctagt ccacagattg 3900

gatgatggca ctactcaatt aaaatttacc cctgcaagct cctatgcatt ctctagcttt 3960

gttcatatat ctaacgactg tcaaattctt gagatcgatg atcaggtaac ggattctaac 4020

ctgatttacc agcaagttat gattactggc cttgctttaa ttgagacatg gaataatcct 4080

ccaatcaact tctccgttta tgaaactaca ttacacttgc atacaggctc atcttgctgt 4140

ataaggcctg tcgagtcttg tgtagtaaat ccgcctttac ttcctgtccc tttcattaat 4200

gttcctcaaa tgaataaatt tgtatatgac cctgaaccac ttagtttgct agaaatggaa 4260

aaaattgagg atattgctta tcaaaccaga attggtggtt tagatcaaat cccgcttctg 4320

gaaaaaatac ccttactagc tcaccttacc gccaaacaga tggtgaatag catcactggg 4380

cttgatgaag caacatctat aatgaatgat gctgtagttc aagcagacta tactagcaat 4440

tggattggtg aatgctgcta cacttacatt gactctgtgt ttgtttactc tggctgggca 4500

ttgttattgg aactttcata ccaaatgtat tacctaagaa ttcgaggcat acaaggaatt 4560

ctagactatg tgtatatgac cttgaggagg ataccaggaa tggccataac aggcatctca 4620

tccacaatta gtcaccctcg tatactcaga agatgcatca atttggatgt catagcccca 4680

atcaattctc cacacatagc ttcactggat tacacaaaat tgagcataga tgcagtaatg 4740

tggggaacca agcaggtgtt gaccaacatt tctcaaggta tcgattatga gatagttgtt 4800

ccttctgaaa gccaacttac actcagtgat agagtcctaa atctagttgc tcgaaaacta 4860

tcactactgg caatcatctg ggccaattac aactatcctc cgaaggttaa aggtatgtca 4920

cctgaggaca aatgtcaggc tttaactaca catctactcc aaactgtcga atatgttgag 4980

tacattcaga ttgaaaagac aaacatcagg aggatgatta ttgaaccaaa attaactgcc 5040

taccctagta atttgtttta tctctctcga aagctgctta atgctattcg agactctgaa 5100

gaaggacaat tcctgattgc atcctattat aacagttttg gatatctgga accgatatta 5160

atggaatcta aagtattcaa tctaagttca tccgaatcag catctcttac agaattcgat 5220

ttcatcctca acttggaatt gtccgacgcc agacttgaga aatactctct cccaagtttg 5280

cttatgacgg ctgagaatat ggataaccca tttcctcaac ccccacttca tcacgttctc 5340

agaccactag gtttgtcatc cacctcatgg tataaaacaa tcagtgtttt gaattatatt 5400

agccatatga agatatctga cggtgcccat ctatacttgg cagagggaag tggagcctct 5460

atgtcactta tagagacttt cttgcccggg gaaaccatat ggtacaacag cctgttcaat 5520
agtggtgaga atccccctca acgtaatttc gcccctttgc ccacccagtt tattgaaagt 5580
gtcccctata gattaattca agcaggtata gcagcaggaa atggtatagt gcaaagtttc 5640
tatccactct ggaacggaaa cagcgatata actgacttaa gcactaaaac tagtgttgaa 5700
tacattatcc acaaggtagg agctgatact tgtgcattag ttcatgtgga tttggaaggt 5760
gtccctggct caatgaacag catgttggag agagctcaag tacacgcact actaatcaca 5820
gtcactgtac tgaaaccagg cggcttacta atcttgaaag cttcatggga accctttaat 5880
cgattttcct ttttactcac agtactctgg caattctttt ccacaataag gatcttgcga 5940
tcttcatact ccgacccgaa taatcacgag gtttacataa tagccacatt ggcagttgat 6000
cccactacat cctcctttac aactgctctg aatagggcac gcaccctgaa tgaacagggc 6060
ttttcactca tcccacctga attagtaagt gagtactgga ggaagcgtgt tgaacaagga 6120
cagattatac aggactgtat agataaagtt atatcagagt gtgtcagaga tcaatatctg 6180
gcagacaaca acattatcct ccaggcggga ggtactccaa gcacaagaaa atggttggat 6240
ctgcctgact attcttcgtt caatgaacta caatctgaaa tggccagact cataacaatt 6300
catcttaaag aggtaataga aatcctaaag ggccaagcat cagatcatga caccctatta 6360
tttacttcat acaatgtagg tcccctcgga aaaataaata caatacttag attgattgtt 6420
gagagaattc ttatgtatac tgtgaggaac tggtgtatct tgcctaccca aactcgtctc 6480
accttacgac aatctatcga gcttggagag tttagactaa gagatgtgat aacacccatg 6540
gagattctaa aactatcccc caacaggaaa tatctaaagt ctgcattaaa tcaatcgaca 6600
ttcaaccatc taatgggaga aacatctgac atattgttaa accgagctta tcagaagaga 6660
atttggaaag ccattgggtg tgtaatctat tgctttggtt tgctcacccc ggatgttgaa 6720
gattctgagc gcattgatat tgacaatgac atacccgatt atgatattca cggggacata 6780
atttaa 6786

<210> 12
<211> 549
<212> PRT
<213> Mumps Virus

<400> 12

```
Met Ser Ser Val Leu Lys Ala Phe Glu Arg Phe Thr Ile Glu Gln Glu
1               5                   10                  15

Leu Gln Asp Arg Gly Glu Glu Gly Ser Ile Pro Pro Glu Thr Leu Lys
            20                  25                  30

Ser Ala Val Lys Val Phe Val Ile Asn Thr Pro Asn Pro Thr Thr Arg
```

```
            35                  40                  45

Tyr Gln Met Leu Asn Phe Cys Leu Arg Ile Ile Cys Ser Gln Asn Ala
    50                  55                  60

Arg Ala Ser His Arg Val Gly Ala Leu Ile Thr Leu Phe Ser Leu Pro
65                  70                  75                  80

Ser Ala Gly Met Gln Asn His Ile Arg Leu Ala Asp Arg Ser Pro Glu
                85                  90                  95

Ala Gln Ile Glu Arg Cys Glu Ile Asp Gly Phe Glu Pro Gly Thr Tyr
            100                 105                 110

Arg Leu Ile Pro Asn Ala Arg Ala Asn Leu Thr Ala Asn Glu Ile Ala
        115                 120                 125

Ala Tyr Ala Leu Leu Ala Asp Asp Leu Pro Pro Thr Ile Asn Asn Gly
    130                 135                 140

Thr Pro Tyr Val His Ala Asp Val Glu Gly Gln Pro Cys Asp Glu Ile
145                 150                 155                 160

Glu Gln Phe Leu Asp Arg Cys Tyr Ser Val Leu Ile Gln Ala Trp Val
                165                 170                 175

Met Val Cys Lys Cys Met Thr Ala Tyr Asp Gln Pro Ala Gly Ser Ala
            180                 185                 190

Asp Arg Arg Phe Ala Lys Tyr Gln Gln Gln Gly Arg Leu Glu Ala Arg
        195                 200                 205

Tyr Met Leu Gln Pro Glu Ala Gln Arg Leu Ile Gln Thr Ala Ile Arg
    210                 215                 220

Lys Ser Leu Val Val Arg Gln Tyr Leu Thr Phe Glu Leu Gln Leu Ala
225                 230                 235                 240

Arg Arg Gln Gly Leu Leu Ser Asn Arg Tyr Tyr Ala Met Val Gly Asp
                245                 250                 255

Ile Gly Lys Tyr Ile Glu Asn Ser Gly Leu Thr Ala Phe Phe Leu Thr
            260                 265                 270

Leu Lys Tyr Ala Leu Gly Thr Lys Trp Ser Pro Leu Ser Leu Ala Ala
        275                 280                 285
```

```
Phe Thr Gly Glu Leu Thr Lys Leu Arg Ser Leu Met Met Leu Tyr Arg
    290                 295                 300

Asp Leu Gly Glu Gln Ala Arg Tyr Leu Ala Leu Leu Glu Ala Pro Gln
305                 310                 315                 320

Ile Met Asp Phe Ala Pro Gly Gly Tyr Pro Leu Ile Phe Ser Tyr Ala
                325                 330                 335

Met Gly Val Gly Thr Val Leu Asp Val Gln Met Arg Asn Tyr Thr Tyr
            340                 345                 350

Ala Arg Pro Phe Leu Asn Gly Tyr Tyr Phe Gln Ile Gly Val Glu Thr
        355                 360                 365

Ala Arg Arg Gln Gln Gly Thr Val Asp Asn Arg Val Ala Asp Asp Leu
    370                 375                 380

Gly Leu Thr Pro Glu Gln Arg Thr Glu Val Thr Gln Leu Val Asp Arg
385                 390                 395                 400

Leu Ala Arg Gly Arg Gly Ala Gly Ile Pro Gly Gly Pro Val Asn Pro
                405                 410                 415

Phe Val Pro Pro Val Gln Gln Gln Gln Pro Ala Ala Val Tyr Glu Asp
            420                 425                 430

Ile Pro Ala Leu Glu Glu Ser Asp Asp Asp Gly Asp Glu Asp Arg Gly
        435                 440                 445

Ala Gly Phe Gln Asn Gly Val Gln Val Pro Ala Val Arg Gln Gly Gly
    450                 455                 460

Gln Thr Asp Phe Arg Ala Gln Pro Leu Gln Asp Pro Ile Gln Ala Gln
465                 470                 475                 480

Leu Phe Met Pro Leu Tyr Pro Gln Val Ser Asn Ile Pro Asn Asn Gln
                485                 490                 495

Asn His Gln Ile Asn Arg Ile Gly Gly Leu Glu Asn Gln Asp Leu Leu
            500                 505                 510

Arg Tyr Asn Glu Asn Gly Asp Ser Gln Gln Asp Ala Arg Gly Glu His
        515                 520                 525

Gly Asn Thr Phe Pro Asn Asn Pro Asn Gln Asn Ala Gln Leu Gln Val
    530                 535                 540
```

Gly Asp Trp Asp Glu
545

<210> 13
<211> 391
<212> PRT
<213> Mumps Virus

<400> 13

```
Met Asp Gln Phe Ile Lys Gln Asp Glu Thr Gly Asp Leu Ile Glu Thr
1               5                   10                  15

Gly Met Asn Val Ala Asn His Phe Leu Ser Ala Pro Ile Gln Gly Thr
            20                  25                  30

Asn Ser Leu Ser Lys Ala Ser Ile Ile Pro Gly Val Ala Pro Val Leu
        35                  40                  45

Ile Gly Asn Pro Glu Gln Lys Asn Ile Gln His Pro Thr Ala Ser His
    50                  55                  60

Gln Gly Ser Lys Ser Lys Gly Arg Gly Ser Gly Val Arg Ser Ile Ile
65                  70                  75                  80

Val Pro Pro Ser Glu Ala Gly Asn Gly Gly Thr Gln Ile Pro Glu Pro
                85                  90                  95

Leu Phe Ala Gln Thr Gly Gln Gly Gly Ile Val Thr Thr Val Tyr Gln
            100                 105                 110

Asp Pro Thr Ile Gln Pro Thr Gly Ser Tyr Arg Ser Val Glu Leu Ala
        115                 120                 125

Lys Ile Gly Lys Glu Arg Met Ile Asn Arg Phe Val Glu Lys Pro Arg
    130                 135                 140

Thr Ser Thr Pro Val Thr Glu Phe Lys Arg Gly Gly Pro Gly Ala Ala
145                 150                 155                 160

Ala Gln Gly Gln Thr Ile Gln Glu Glu Gly Ile Asp Gly Asn Gly Ala
                165                 170                 175

Ser Ala Gly Ser Lys Glu Arg Ser Gly Ser Leu Ser Gly Ala Thr Leu
            180                 185                 190

Tyr Ala His Leu Ser Leu Pro Gln Gln Asp Ser Thr Pro Ala Asn Val
        195                 200                 205
```

Gly Ile Ala Pro Gln Ser Ala Thr Ser Ala Asn Glu Ile Met Asp Leu
210 215 220

Leu Arg Gly Met Asp Ala Arg Leu Gln His Leu Glu Gln Lys Val Asp
225 230 235 240

Lys Val Leu Ala Gln Gly Ser Met Val Thr Gln Ile Lys Asn Glu Leu
245 250 255

Ser Thr Val Lys Thr Thr Leu Ala Thr Ile Glu Gly Met Met Ala Thr
260 265 270

Val Lys Ile Met Asp Pro Gly Asn Pro Thr Gly Val Pro Val Asp Glu
275 280 285

Leu Arg Arg Ser Phe Ser Asp His Val Thr Ile Val Ser Gly Pro Gly
290 295 300

Asp Val Ser Phe Ser Ser Arg Glu Glu Pro Thr Leu Tyr Leu Asp Glu
305 310 315 320

Leu Ala Arg Pro Val Ser Lys Pro Arg Pro Ala Lys Gln Thr Lys Pro
325 330 335

Gln Pro Val Lys Asp Leu Ala Gly Arg Lys Val Met Ile Thr Lys Met
340 345 350

Ile Thr Asp Cys Val Ala Asn Pro Gln Met Lys Gln Ala Phe Glu Gln
355 360 365

Arg Leu Ala Lys Ala Ser Thr Glu Asp Ala Leu Asn Asp Ile Lys Lys
370 375 380

Asp Ile Ile Arg Ser Ala Ile
385 390

<210> 14
<211> 2261
<212> PRT
<213> Mumps Virus

<400> 14

Met Ala Gly Leu Asn Glu Ile Leu Leu Pro Glu Val His Leu Asn Ser
1 5 10 15

Pro Ile Val Arg Tyr Lys Leu Phe Tyr Tyr Ile Leu His Gly Gln Leu
20 25 30

```
Pro Asn Asp Leu Glu Pro Asp Asp Leu Gly Pro Leu Ala Asn His Asn
        35                  40                  45

Trp Lys Ala Ile Arg Ala Glu Glu Ser Gln Val His Ala Arg Leu Lys
    50                  55                  60

Gln Ile Arg Val Glu Leu Ile Ala Arg Ile Pro Ser Leu Arg Trp Thr
65                  70                  75                  80

Arg Ser Gln Arg Glu Ile Ala Ile Leu Ile Trp Pro Arg Ile Leu Pro
                85                  90                  95

Ile Leu Gln Ala Tyr Asp Leu Arg Gln Ser Met Gln Leu Pro Thr Val
            100                 105                 110

Trp Glu Lys Leu Thr Gln Ser Thr Val Asn Leu Ile Ser Asp Gly Leu
        115                 120                 125

Glu Arg Val Val Leu His Ile Ser Asn Gln Leu Thr Gly Lys Pro Asn
    130                 135                 140

Leu Phe Thr Arg Ser Arg Ala Gly Gln Asp Thr Lys Asp Tyr Ser Ile
145                 150                 155                 160

Pro Ser Thr Arg Glu Leu Ser Gln Ile Trp Phe Asn Asn Glu Trp Ser
                165                 170                 175

Gly Ser Val Lys Thr Trp Leu Met Ile Lys Tyr Arg Met Arg Gln Leu
            180                 185                 190

Ile Thr Asn Gln Lys Thr Gly Glu Leu Thr Asp Leu Val Thr Ile Val
        195                 200                 205

Asp Thr Arg Ser Thr Leu Cys Ile Ile Thr Pro Glu Leu Val Ala Leu
    210                 215                 220

Tyr Ser Asn Glu His Lys Ala Leu Thr Tyr Leu Thr Phe Glu Met Val
225                 230                 235                 240

Leu Met Val Thr Asp Met Leu Glu Gly Arg Leu Asn Val Ser Ser Leu
                245                 250                 255

Cys Thr Ala Ser His Tyr Leu Ser Pro Leu Lys Lys Arg Ile Glu Val
            260                 265                 270

Leu Leu Thr Leu Val Asp Asp Leu Ala Leu Leu Met Gly Asp Lys Val
        275                 280                 285
```

Tyr Gly Ile Val Ser Ser Leu Glu Ser Phe Val Tyr Ala Gln Leu Gln
    290                 295                 300

Tyr Gly Asp Pro Val Ile Asp Ile Lys Gly Thr Phe Tyr Gly Phe Ile
305                 310                 315                 320

Cys Asn Glu Ile Leu Asp Leu Leu Thr Glu Gly Asn Ile Phe Thr Glu
                325                 330                 335

Glu Glu Ala Asn Lys Val Leu Leu Asp Leu Thr Ser Gln Phe Asp Asn
            340                 345                 350

Leu Ser Pro Asp Leu Thr Ala Glu Leu Leu Cys Ile Met Arg Leu Trp
        355                 360                 365

Gly His Pro Thr Leu Thr Ala Ser Gln Ala Ala Ser Lys Val Arg Glu
    370                 375                 380

Ser Met Cys Ala Pro Lys Val Leu Asp Phe Gln Thr Ile Met Lys Thr
385                 390                 395                 400

Leu Ala Phe Phe His Ala Ile Leu Ile Asn Gly Tyr Arg Arg Ser His
                405                 410                 415

Asn Gly Ile Trp Pro Pro Thr Thr Leu His Gly Asn Ala Pro Lys Ser
            420                 425                 430

Leu Ile Glu Met Arg His Asp Asn Ser Glu Leu Lys Tyr Glu Tyr Val
        435                 440                 445

Leu Lys Asn Trp Lys Ser Ile Ser Met Leu Arg Ile His Lys Cys Phe
    450                 455                 460

Asp Ala Ser Pro Asp Glu Asp Leu Ser Ile Phe Met Lys Asp Lys Ala
465                 470                 475                 480

Ile Ser Cys Pro Lys Gln Asp Trp Met Gly Val Phe Arg Arg Ser Leu
                485                 490                 495

Ile Lys Gln Arg Tyr Arg Asp Val Asn Arg Pro Leu Pro Gln Pro Phe
            500                 505                 510

Asn Arg Arg Leu Leu Leu Asn Phe Leu Glu Asp Asp Arg Phe Asp Pro
        515                 520                 525

Ile Lys Glu Leu Glu Tyr Val Thr Ser Gly Glu Tyr Leu Arg Asp Pro

```
        530                 535                 540

Glu Phe Cys Ala Ser Tyr Ser Leu Lys Glu Lys Glu Ile Lys Ala Thr
545                 550                 555                 560

Gly Arg Ile Phe Ala Lys Met Thr Lys Arg Met Arg Ser Cys Gln Val
                565                 570                 575

Ile Ala Glu Ser Leu Leu Ala Asn His Ala Gly Lys Leu Met Arg Glu
            580                 585                 590

Asn Gly Val Val Leu Asp Gln Leu Lys Leu Thr Lys Ser Leu Leu Thr
        595                 600                 605

Met Asn Gln Ile Gly Ile Ile Ser Glu His Ser Arg Arg Ser Thr Ala
    610                 615                 620

Asp Asn Met Thr Leu Ala His Ser Gly Ser Asn Lys His Arg Ile Asn
625                 630                 635                 640

Asn Ser Gln Phe Lys Lys Asn Lys Asp Asn Lys His Glu Met Pro Asp
                645                 650                 655

Asp Gly Phe Glu Ile Ala Ala Cys Phe Leu Thr Thr Asp Leu Thr Lys
            660                 665                 670

Tyr Cys Leu Asn Trp Arg Tyr Gln Val Ile Ile Pro Phe Ala Arg Thr
        675                 680                 685

Leu Asn Ser Met Tyr Gly Ile Pro His Leu Phe Glu Trp Ile His Leu
    690                 695                 700

Arg Leu Met Arg Ser Thr Leu Tyr Val Gly Asp Pro Phe Asn Pro Pro
705                 710                 715                 720

Ser Asp Pro Thr Gln Leu Asp Leu Asp Thr Ala Leu Asn Asp Asp Ile
                725                 730                 735

Phe Ile Val Ser Pro Arg Gly Gly Ile Glu Gly Leu Cys Gln Lys Leu
            740                 745                 750

Trp Thr Met Ile Ser Ile Ser Thr Ile Ile Leu Ser Ala Thr Glu Ala
        755                 760                 765

Asn Thr Arg Val Met Ser Met Val Gln Gly Asp Asn Gln Ala Ile Ala
    770                 775                 780
```

```
Ile Thr Thr Arg Val Val Arg Ser Leu Ser His Ser Glu Lys Lys Glu
785                 790                 795                 800

Gln Ala Tyr Lys Ala Ser Lys Leu Phe Phe Glu Arg Leu Arg Ala Asn
                805                 810                 815

Asn His Gly Ile Gly His His Leu Lys Glu Gln Glu Thr Ile Leu Ser
            820                 825                 830

Ser Asp Phe Phe Ile Tyr Ser Lys Arg Val Phe Tyr Lys Gly Arg Ile
        835                 840                 845

Leu Thr Gln Ala Leu Lys Asn Val Ser Lys Met Cys Leu Thr Ala Asp
    850                 855                 860

Ile Leu Gly Asp Cys Ser Gln Ala Ser Cys Ser Asn Leu Ala Thr Thr
865                 870                 875                 880

Val Met Arg Leu Thr Glu Asn Gly Val Glu Lys Asp Leu Cys Tyr Phe
                885                 890                 895

Leu Asn Ala Phe Met Thr Ile Arg Gln Leu Cys Tyr Asp Leu Val Phe
            900                 905                 910

Pro Gln Thr Lys Ser Leu Ser Gln Asp Ile Thr Asn Ala Tyr Leu Asn
        915                 920                 925

His Pro Ile Leu Ile Ser Arg Leu Cys Leu Leu Pro Ser Gln Leu Gly
    930                 935                 940

Gly Leu Asn Phe Leu Ser Cys Ser Arg Leu Phe Asn Arg Asn Ile Gly
945                 950                 955                 960

Asp Pro Leu Val Ser Ala Ile Ala Asp Val Lys Arg Leu Ile Lys Ala
                965                 970                 975

Gly Cys Leu Asp Ile Trp Val Leu Tyr Asn Ile Leu Gly Arg Arg Pro
            980                 985                 990

Gly Lys Gly Lys Trp Ser Thr Leu  Ala Ala Asp Pro Tyr  Thr Leu Asn
        995                 1000                 1005

Ile Asp  Tyr Leu Val Pro Ser  Thr Thr Phe Leu Lys  Lys His Ala
    1010                 1015                 1020

Gln Tyr  Thr Leu Met Glu Arg  Ser Val Asn Pro Met  Leu Arg Gly
    1025                 1030                 1035
```

Val Phe Ser Glu Asn Ala Ala Glu Glu Glu Glu Glu Leu Ala Gln
1040 1045 1050

Tyr Leu Leu Asp Arg Glu Val Val Met Pro Arg Val Ala His Val
1055 1060 1065

Ile Leu Ala Gln Ser Ser Cys Gly Arg Arg Lys Gln Ile Gln Gly
1070 1075 1080

Tyr Leu Asp Ser Thr Arg Thr Ile Ile Arg Tyr Ser Leu Glu Val
1085 1090 1095

Arg Pro Leu Ser Ala Lys Lys Leu Asn Thr Val Ile Glu Tyr Asn
1100 1105 1110

Leu Leu Tyr Leu Ser Tyr Asn Leu Glu Ile Ile Glu Lys Pro Asn
1115 1120 1125

Ile Val Gln Pro Phe Leu Asn Ala Ile Asn Val Asp Thr Cys Ser
1130 1135 1140

Ile Asp Ile Ala Arg Ser Leu Arg Lys Leu Ser Trp Ala Thr Leu
1145 1150 1155

Leu Asn Gly Arg Pro Ile Glu Gly Leu Glu Thr Pro Asp Pro Ile
1160 1165 1170

Glu Leu Val His Gly Cys Leu Ile Ile Gly Ser Asp Glu Cys Glu
1175 1180 1185

His Cys Ser Ser Gly Asp Asp Lys Phe Thr Trp Phe Phe Leu Pro
1190 1195 1200

Lys Gly Ile Arg Leu Asp Asp Asp Pro Ala Ser Asn Pro Pro Ile
1205 1210 1215

Arg Val Pro Tyr Ile Gly Ser Lys Thr Asp Glu Arg Arg Val Ala
1220 1225 1230

Ser Met Ala Tyr Ile Lys Gly Ala Ser Val Ser Leu Lys Ser Ala
1235 1240 1245

Leu Arg Leu Ala Gly Val Tyr Ile Trp Ala Phe Gly Asp Thr Glu
1250 1255 1260

Glu Ser Trp Gln Asp Ala Tyr Glu Leu Ala Ser Thr Arg Val Asn
1265 1270 1275

```
Leu Thr  Leu Glu Gln Leu Gln  Ser Leu Thr Pro Leu  Pro Thr Ser
    1280                 1285                 1290

Ala Asn  Leu Val His Arg Leu  Asp Asp Gly Thr Thr  Gln Leu Lys
    1295                 1300                 1305

Phe Thr  Pro Ala Ser Ser Tyr  Ala Phe Ser Ser Phe  Val His Ile
    1310                 1315                 1320

Ser Asn  Asp Cys Gln Ile Leu  Glu Ile Asp Asp Gln  Val Thr Asp
    1325                 1330                 1335

Ser Asn  Leu Ile Tyr Gln Gln  Val Met Ile Thr Gly  Leu Ala Leu
    1340                 1345                 1350

Ile Glu  Thr Trp Asn Asn Pro  Pro Ile Asn Phe Ser  Val Tyr Glu
    1355                 1360                 1365

Thr Thr  Leu His Leu His Thr  Gly Ser Ser Cys Cys  Ile Arg Pro
    1370                 1375                 1380

Val Glu  Ser Cys Val Val Asn  Pro Pro Leu Leu Pro  Val Pro Phe
    1385                 1390                 1395

Ile Asn  Val Pro Gln Met Asn  Lys Phe Val Tyr Asp  Pro Glu Pro
    1400                 1405                 1410

Leu Ser  Leu Leu Glu Met Glu  Lys Ile Glu Asp Ile  Ala Tyr Gln
    1415                 1420                 1425

Thr Arg  Ile Gly Gly Leu Asp  Gln Ile Pro Leu Leu  Glu Lys Ile
    1430                 1435                 1440

Pro Leu  Leu Ala His Leu Thr  Ala Lys Gln Met Val  Asn Ser Ile
    1445                 1450                 1455

Thr Gly  Leu Asp Glu Ala Thr  Ser Ile Met Asn Asp  Ala Val Val
    1460                 1465                 1470

Gln Ala  Asp Tyr Thr Ser Asn  Trp Ile Gly Glu Cys  Cys Tyr Thr
    1475                 1480                 1485

Tyr Ile  Asp Ser Val Phe Val  Tyr Ser Gly Trp Ala  Leu Leu Leu
    1490                 1495                 1500

Glu Leu  Ser Tyr Gln Met Tyr  Tyr Leu Arg Ile Arg  Gly Ile Gln
```

1505 1510 1515

Gly Ile Leu Asp Tyr Val Tyr Met Thr Leu Arg Arg Ile Pro Gly
1520 1525 1530

Met Ala Ile Thr Gly Ile Ser Ser Thr Ile Ser His Pro Arg Ile
1535 1540 1545

Leu Arg Arg Cys Ile Asn Leu Asp Val Ile Ala Pro Ile Asn Ser
1550 1555 1560

Pro His Ile Ala Ser Leu Asp Tyr Thr Lys Leu Ser Ile Asp Ala
1565 1570 1575

Val Met Trp Gly Thr Lys Gln Val Leu Thr Asn Ile Ser Gln Gly
1580 1585 1590

Ile Asp Tyr Glu Ile Val Val Pro Ser Glu Ser Gln Leu Thr Leu
1595 1600 1605

Ser Asp Arg Val Leu Asn Leu Val Ala Arg Lys Leu Ser Leu Leu
1610 1615 1620

Ala Ile Ile Trp Ala Asn Tyr Asn Tyr Pro Pro Lys Val Lys Gly
1625 1630 1635

Met Ser Pro Glu Asp Lys Cys Gln Ala Leu Thr Thr His Leu Leu
1640 1645 1650

Gln Thr Val Glu Tyr Val Glu Tyr Ile Gln Ile Glu Lys Thr Asn
1655 1660 1665

Ile Arg Arg Met Ile Ile Glu Pro Lys Leu Thr Ala Tyr Pro Ser
1670 1675 1680

Asn Leu Phe Tyr Leu Ser Arg Lys Leu Leu Asn Ala Ile Arg Asp
1685 1690 1695

Ser Glu Glu Gly Gln Phe Leu Ile Ala Ser Tyr Tyr Asn Ser Phe
1700 1705 1710

Gly Tyr Leu Glu Pro Ile Leu Met Glu Ser Lys Val Phe Asn Leu
1715 1720 1725

Ser Ser Ser Glu Ser Ala Ser Leu Thr Glu Phe Asp Phe Ile Leu
1730 1735 1740

```
Asn Leu  Glu Leu Ser Asp Ala  Arg Leu Glu Lys Tyr  Ser Leu Pro
    1745                 1750                 1755

Ser Leu  Leu Met Thr Ala Glu  Asn Met Asp Asn Pro  Phe Pro Gln
    1760                 1765                 1770

Pro Pro  Leu His His Val Leu  Arg Pro Leu Gly Leu  Ser Ser Thr
    1775                 1780                 1785

Ser Trp  Tyr Lys Thr Ile Ser  Val Leu Asn Tyr Ile  Ser His Met
    1790                 1795                 1800

Lys Ile  Ser Asp Gly Ala His  Leu Tyr Leu Ala Glu  Gly Ser Gly
    1805                 1810                 1815

Ala Ser  Met Ser Leu Ile Glu  Thr Phe Leu Pro Gly  Glu Thr Ile
    1820                 1825                 1830

Trp Tyr  Asn Ser Leu Phe Asn  Ser Gly Glu Asn Pro  Pro Gln Arg
    1835                 1840                 1845

Asn Phe  Ala Pro Leu Pro Thr  Gln Phe Ile Glu Ser  Val Pro Tyr
    1850                 1855                 1860

Arg Leu  Ile Gln Ala Gly Ile  Ala Ala Gly Asn Gly  Ile Val Gln
    1865                 1870                 1875

Ser Phe  Tyr Pro Leu Trp Asn  Gly Asn Ser Asp Ile  Thr Asp Leu
    1880                 1885                 1890

Ser Thr  Lys Thr Ser Val Glu  Tyr Ile Ile His Lys  Val Gly Ala
    1895                 1900                 1905

Asp Thr  Cys Ala Leu Val His  Val Asp Leu Glu Gly  Val Pro Gly
    1910                 1915                 1920

Ser Met  Asn Ser Met Leu Glu  Arg Ala Gln Val His  Ala Leu Leu
    1925                 1930                 1935

Ile Thr  Val Thr Val Leu Lys  Pro Gly Gly Leu Leu  Ile Leu Lys
    1940                 1945                 1950

Ala Ser  Trp Glu Pro Phe Asn  Arg Phe Ser Phe Leu  Leu Thr Val
    1955                 1960                 1965

Leu Trp  Gln Phe Phe Ser Thr  Ile Arg Ile Leu Arg  Ser Ser Tyr
    1970                 1975                 1980
```

```
Ser Asp  Pro Asn Asn His Glu  Val Tyr Ile Ile Ala  Thr Leu Ala
    1985                 1990                 1995

Val Asp  Pro Thr Thr Ser Ser  Phe Thr Thr Ala Leu  Asn Arg Ala
    2000                 2005                 2010

Arg Thr  Leu Asn Glu Gln Gly  Phe Ser Leu Ile Pro  Pro Glu Leu
    2015                 2020                 2025

Val Ser  Glu Tyr Trp Arg Lys  Arg Val Glu Gln Gly  Gln Ile Ile
    2030                 2035                 2040

Gln Asp  Cys Ile Asp Lys Val  Ile Ser Glu Cys Val  Arg Asp Gln
    2045                 2050                 2055

Tyr Leu  Ala Asp Asn Asn Ile  Ile Leu Gln Ala Gly  Gly Thr Pro
    2060                 2065                 2070

Ser Thr  Arg Lys Trp Leu Asp  Leu Pro Asp Tyr Ser  Ser Phe Asn
    2075                 2080                 2085

Glu Leu  Gln Ser Glu Met Ala  Arg Leu Ile Thr Ile  His Leu Lys
    2090                 2095                 2100

Glu Val  Ile Glu Ile Leu Lys  Gly Gln Ala Ser Asp  His Asp Thr
    2105                 2110                 2115

Leu Leu  Phe Thr Ser Tyr Asn  Val Gly Pro Leu Gly  Lys Ile Asn
    2120                 2125                 2130

Thr Ile  Leu Arg Leu Ile Val  Glu Arg Ile Leu Met  Tyr Thr Val
    2135                 2140                 2145

Arg Asn  Trp Cys Ile Leu Pro  Thr Gln Thr Arg Leu  Thr Leu Arg
    2150                 2155                 2160

Gln Ser  Ile Glu Leu Gly Glu  Phe Arg Leu Arg Asp  Val Ile Thr
    2165                 2170                 2175

Pro Met  Glu Ile Leu Lys Leu  Ser Pro Asn Arg Lys  Tyr Leu Lys
    2180                 2185                 2190

Ser Ala  Leu Asn Gln Ser Thr  Phe Asn His Leu Met  Gly Glu Thr
    2195                 2200                 2205

Ser Asp  Ile Leu Leu Asn Arg  Ala Tyr Gln Lys Arg  Ile Trp Lys
    2210                 2215                 2220
```

```
Ala Ile  Gly Cys Val Ile Tyr  Cys Phe Gly Leu Leu  Thr Pro Asp
    2225                 2230                 2235

Val Glu  Asp Ser Glu Arg Ile  Asp Ile Asp Asn Asp  Ile Pro Asp
    2240                 2245                 2250

Tyr Asp  Ile His Gly Asp Ile  Ile
    2255                 2260
```

<210> 15
<211> 8127
<212> DNA
<213> Mumps Virus

<400> 15

```
gattcgtgtc ttaccgggtt ggactcaaga cgatagttac cggataaggc gcagcggtcg      60
ggctgaacgg ggggttcgtg cacacagccc agcttggagc gaacgaccta caccgaactg     120
agatacctac agcgtgagct atgagaaagc gccacgcttc ccgaagggag aaaggcggac     180
aggtatccgg taagcggcag ggtcggaaca ggagagcgca cgagggagct tccaggggga     240
aacgcctggt atctttatag tcctgtcggg tttcgccacc tctgacttga gcgtcgattt     300
ttgtgatgct cgtcaggggg gcggagccta tggaaaaacg ccagcaacgc ggccttttta     360
cggttcctgg ccttttgctg gccttttgct cacatgttct ttcctgcgtt atcccctgat     420
tctgtggata accgtattac cgcctttgag tgagctgata ccgctcgccg cagccgaacg     480
accgagcgca gcgagtcagt gagcgaggaa gcggaagagc gcccaatacg caaaccgcct     540
ctccccgcgc gttggccgat tcattaatgc agcaattagt cagcaaccat agtcccgccc     600
ctaactccgc ccatcccgcc cctaactccg cccagttccg cccattctcc gccccatggc     660
tgactaattt tttttattta tgcagaggcc gaggccgcct cggcctctga gctattccag     720
aagtagtgaa gaggcttttt tggaggccta gcatcgataa gcttgtcgag ccccagctgg     780
ttctttccgc ctcagaagcc atagagccca ccgcatcccc agcatgcctg ctattgtctt     840
cccaatcctc cccttgctg tcctgcccca ccccaccccc cagaatagaa tgacacctac     900
tcagacaatg cgatgcaatt tcctcatttt attaggaaag gacagtggga gtggcacctt     960
ccagggtcaa ggaaggcacg gggagggggc aaacaacaga tggctggcaa ctagaaggca    1020
cagtcgaggc tgatcagcga gctctagaga attgatcccc tcagaagaac tcgtcaagaa    1080
ggcgatagaa ggcgatgcgc tgcgaatcgg gagcggcgat accgtaaagc acgaggaagc    1140
ggtcagccca ttcgccgcca agctcttcag caatatcacg ggtagccaac gctatgtcct    1200
gatagcggtc cgccacaccc agccggccac agtcgatgaa tccagaaaag cggccatttt    1260
ccaccatgat attcggcaag caggcatcgc catgggtcac gacgagatca tcgccgtcgg    1320
```

gcatgcgcgc cttgagcctg gcgaacagtt cggctggcgc gagcccctga tgctcttcgt 1380
ccagatcatc ctgatcgaca agaccggctt ccatccgagt acgtgctcgc tcgatgcgat 1440
gtttcgcttg gtggtcgaat gggcaggtag ccggatcaag cgtatgcagc cgccgcattg 1500
catcagccat gatggatact ttctcggcag gagcaaggtg agatgacagg agatcctgcc 1560
ccggcacttc gcccaatagc agccagtccc ttcccgcttc agtgacaacg tcgagcacag 1620
ctgcgcaagg aacgcccgtc gtggccagcc acgatagccg cgctgcctcg tcctgcagtt 1680
cattcagggc accggacagg tcggtcttga caaaaagaac cgggcgcccc tgcgctgaca 1740
gccggaacac ggcggcatca gagcagccga ttgtctgttg tgcccagtca tagccgaata 1800
gcctctccac ccaagcggcc ggagaacctg cgtgcaatcc atcttgttca atggccgatc 1860
ccatattggc tgcaggtcga aaggcccgga gatgaggaag aggagaacag cgcggcagac 1920
gtgcgctttt gaagcgtgca gaatgccggg cctccggagg accttcgggc gcccgccccg 1980
cccctgagcc cgcccctgag cccgcccccg gacccacccc ttcccagcct ctgagcccag 2040
aaagcgaagg agcaaagctg ctattggccg ctgccccaaa ggcctacccg cttccattgc 2100
tcagcggtgc tgtccatctg cacgagacta gtgagacgtg ctacttccat ttgtcacgtc 2160
ctgcacgacg cgagctgcgg ggcggggggg aacttcctga ctaggggagg agtagaaggt 2220
ggcgcgaagg ggccaccaaa gaacggagcc ggttggcgcc taccggtgga tgtggaatgt 2280
gtgcgaggcc agaggccact tgtgtagcgc caagtgccca gcggggctgc taaagcgcat 2340
gctccagact gccttgggaa aagcgcctcc cctacccggt agaattcgat atcaagctta 2400
tcgataccgt cgacctcgac cctaggcttt tgcaaaaagc tcctcgaggg atctccataa 2460
gagaagaggg acagctatga ctgggagtag tcaggagagg aggaaaaatc tggctagtaa 2520
aacatgtaag gaaaatttta gggatgttaa agaaaaaaat aacacaaaac aaaatataaa 2580
aaaaatctaa cctcaagtca aggcttttct atggaataag gaatggacag caggggggctg 2640
tttcatatac tgatgacctc tttatagcca acctttgttc atggcagcca gcatatgggc 2700
atatgttgcc aaactctaaa ccaaatactc attctgatgt tttaaatgat ttgccctccc 2760
atatgtcctt ccgagtgaga gacacaaaaa attccaacac actattgcaa tgaaaataaa 2820
tttcctttat tagccagaag tcagatgctc aaggggcttc atgatgtccc cataattttt 2880
ggcagaggga aaaagatctc agtggtattt gtgagccagg gcattggcca caccagccac 2940
caccttctga taggcagcct gcacctgagg agtgaattct ttgccaaaat gatgagacag 3000
cacaacaacc agcacgttgc ccaggagctg taggaaagag aagaaggcat gaacatggtt 3060
agcagagggg cccggtttgg actcagagta ttttatcctc atctcaaaca gtgtatatca 3120
ttgtaaccat aaagagaaag gcaggatgat gaccagggtg tagttgtttc taccaataag 3180

aatatttcca cgccagccag aatttatatg cagaaatatt ctaccttatc atttaattat 3240

aacaattgtt ctctaaaact gtgctgaagt acaatataat ataccctgat tgccttgaaa 3300

aaaaagtgat tagagaaagt acttacaatc tgacaaataa acaaaagtga atttaaaaat 3360

tcgttacaaa tgcaagctaa agtttaacga aaaagttaca gaaaatgaaa agaaaataag 3420

aggagacaat ggttgtcaac agagtagaaa gtgaaagaaa caaaattatc atgagggtcc 3480

atggtgatac aagggacatc ttcccattct aaacaacacc ctgaaaactt tgccccctcc 3540

atataacatg aattttacaa tagcgaaaaa gaaagaacaa tcaagggtcc ccaaactcac 3600

cctgaagttc tcaggatccg gagtcgtatt gatttggcgt tacgcgaacg cgaagtccga 3660

ctctaagatg tcacggaggt tcaagttacc tttagccgga agtgctggca ttttgtccaa 3720

ttgagactcg tgcaactggt cagcgaactg gtcgtagaaa tcagccagta catcacaaga 3780

ctcatatgtg tcaaccatag tttcgcgcac tgctttgaac aggttcgcag cgtcagccgg 3840

aatggtaccg aaggagtcgt gaatcagtgc aaaagattcg attccgtact tctcgtgtgc 3900

ccacactaca gtcttacgaa ggtggctacc gtcttggctg tgtacaaagt taggagcgat 3960

accagactcc tgtttgtgtg catcaatctc gctatctttg ttggtgttaa tggtaggctg 4020

taagcggaac tgaccgagga acatcaggtt caagcgcgtc tgaataggct tcttgtattc 4080

ctgccacaca gggaaaccat caggagttac ccaatgcaca gcgcaacgct tgcgaagaat 4140

ctctccagtc ttcttatctt tgacctcagc agccagcagc ttagcagcag acttaagcca 4200

gttcattgct tcaaccgcag ctaccaccgt cacgctcaca gattcccaaa tcagcttagc 4260

catgtatcca gcagcctgat tcggctgagt gaacatcaga cccttgccgg aatcaatagc 4320

tggctgaatg gtatcttcca gcacttgttg acggaagccg aactctttgg acccgtaagc 4380

cagcgtcatg actgaacgct tagtcacact gcgagtaaca ccgtaagcca gccattgacc 4440

agccagtgcc ttagtgccca gcttgacttt ctcagagatt tcaccagtgt tctcatcggt 4500

cacggtaact acttcgttat cggtcccatt gattgcgtct gcttgtagaa tctcgttgac 4560

tttcttagca acaatcccgt agatgtcctg aacggtttca ctaggaagca agttaaccgc 4620

gcgaccacct acctcatctc ggagcatcgc ggagaagtgc tggatgccag agcaagaccc 4680

gtcaaacgcc agcggaaggg agcagttata gctcaggccg tggtgctgta ccccagcgta 4740

ctcaaagcag aacgcaagga agcagaacgg agaatcttgc tcagcccacc aagtgttctc 4800

cagtggagac ttagcgcaag ccatgatgtt ctcgtggttt tcctcaatga acttgatgcg 4860

ctcagggaac ggaaccttat cgacacccgc acagtttgca ccgtggattt tcagccagta 4920

gtaaccttcc ttaccgattg gtttaccttt cgccagcgta agcagtcctt tggtcatatc 4980

gttaccttgc gggttgaaca ttgacacagc gtaaacacga ccgcgccagt ccatgttgta 5040

agggaaccag atggccttat ggttagcaaa cttattggct tgctcaagca tgaactcaag 5100

gctgatacgg cgagacttgc gagccttgtc cttgcggtac acagcagcgg cagcacgttt 5160

ccacgcggtg agagcctcag gattcatgtc gatgtcttcc ggtttcatcg ggagttcttc 5220

acgctcaatc gcagggatgt cctcgaccgg acaatgcttc cacttggtga ttacgttggc 5280

gaccgctagg actttcttgt tgattttcca tgcggtgttt tgcgcaatgt taatcgcttt 5340

gtacacctca ggcatgtaaa cgtcttcgta gcgcatcagt gctttcttac tgtgagtacg 5400

caccagcgcc agaggacgac gaccgttagc ccaatagcca ccaccagtaa tgccagtcca 5460

cggcttagga ggaactacgc aaggttggaa catcggagag atgccagcca gcgcacctgc 5520

acgggttgcg atagcctcag cgtattcagg tgcgagttcg atagtctcag agtcttgacc 5580

tactacgcca gcattttggc ggtgtaagct aaccattccg gttgactcaa tgagcatctc 5640

gatgcagcgt actcctacat gaatagagtc ttccttatgc cacgaagacc acgcctcgcc 5700

accgagtaga cccttagaga gcatgtcagc ctcgacaact tgcataaatg ctttcttgta 5760

gacgtgccct acgcgcttgt tgagttgttc ctcaacgttt ttcttgaagt gcttagcttc 5820

aaggtcacgg atacgaccga agcgagcctc gtcctcaatg gcccgaccga ttgcgcttgc 5880

tacagcctga acggttgtat tgtcagcact ggttaggcaa gccagagtgg tcttaatggt 5940

gatgtacgct acggcttccg gcttgatttc ttgcaggaac tggaaggctg tcgggcgctt 6000

gccgcgctta gctttcactt cctcaaacca gtcgttgatg cgtgcaatca tcttagggag 6060

tagggtagtg atgagaggct tggcggcagc gttatccgca acctcaccag ctttaagttg 6120

acgctcaaac atcttgcgga agcgtgcttc acccatctcg taagactcat gctcaagggc 6180

caactgttcg cgagctaaac gctcaccgta atggtcagcc agagtgttga acgggatagc 6240

agccagttcg atgtcagaga agtcgttctt agcgatgtta atcgtgttca tttagtgcct 6300

cttccagtat ccctaatcct gctcttgtcc ctgataatag gatcttgaat cctaacgacc 6360

tcgagatccc ccggaggctg gatcggtccc ggtgtcttct atggaggtca aaacagcgtg 6420

gatggcgtct ccaggcgatc tgacggttca ctaaacgagc tctgcttata tagacctccc 6480

accgtacacg cctaccgccc atttgcgtca atggggcgga gttgttacga cattttggaa 6540

agtcccgttg attttggtgc caaaacaaac tcccattgac gtcaatgggg tggagacttg 6600

gaaatccccg tgagtcaaac cgctatccac gcccattgat gtactgccaa aaccgcatca 6660

ccatggtaat agcgatgact aatacgtaga tgtactgcca agtaggaaag tcccataagg 6720

tcatgtactg ggcataatgc caggcgggcc atttaccgtc attgacgtca atagggggcg 6780

tacttggcat atgatacact tgatgtactg ccaagtgggc agtttaccgt aaatactcca 6840

cccattgacg tcaatggaaa gtccctattg gcgttactat gggaacatac gtcattattg 6900

acgtcaatgg gcgggggtcg ttgggcggtc agccaggcgg gccatttacc gtaagttatg 6960

```
taacggggcg agctcgaatt ccgtgtattc tatagtgtca cctaaatcgt atgtgtatga     7020
tacataaggt tatgtattaa ttgtagccgc gttctaacga caatatgtac aagcctaatt     7080
gtgtagcatc tggcttactg aagcagaccc tatcatctct ctcgtaaact gccgtcagag     7140
tcggtttggt tggacgaacc ttctgagttt ctggtaacgc cgtcccgcac ccggaaatgg     7200
tcagcgaacc aatcagcagg gtcatcgcta gccagatcct ctacgccgga cgcatcgtgg     7260
ccggcatcac cggcgccaca ggtgcggttg ctggcgccta tatcgccgac atcaccgatg     7320
gggaagatcg ggctcgccac ttcgggctca tgagcgcttg tttcggcgtg ggtatggtgg     7380
caggccccgt ggccggggga ctgttgggcg ccatctcctt gcatgcacca ttccttgcgg     7440
cggcggtgct caacggcctc aacctactac tgggctgctt cctaatgcag gagtcgcata     7500
agggagagcg tcgaatggtg cactctcagt acaatctgct ctgatgccgc atagttaagc     7560
cagccccgac acccgccaac acccgctgac gcgccctgac gggcttgtct gctcccggca     7620
tccgcttaca gacaagctgt gaccgtctcc gggagctgca tgtgtcagag gttttcaccg     7680
tcatcaccga aacgcgcgag acgaaagggc ctcgtgatac gcctattttt ataggttaat     7740
gtcatgataa taatggtttc ttagacgtca ggtggcactt ttcggggaaa tgtgcgcgga     7800
acccctattt gtttattttt ctaaatacat tcaaatatgt atccgctcat gagacaataa     7860
ccctgataaa tgcttcaata atattgaaaa aggaagagta tgagtattca acatttccgt     7920
gtcgccctta ttcccttttt tgcggcattt tgccttcctg tttttgctca cccagaaacg     7980
ctggtgaaag taaaagatgc tgaagatcag ttgggtgcac gagtgggtta catcgaactg     8040
gatctcaaca gcggtaagat ccttgagagt tttcgccccg aagaacgttt tccaatgatg     8100
agcactttta aagttctgct atgtggc                                        8127
```